# EUROPEAN PATENT APPLICATION

(11) **EP 2 554 162 A1**
(43) Date of publication of application: **06.02.2013**
(21) Application number: 12190206.8
(22) Date of filing: 02.02.2007
(51) Int. Cl.: A61K 31/136, A61K 31/167, A61K 31/44, A61K 31/505, A61K 31/506, A61P 25/18

(54) **Use of KCNQ-Openers for Treating or Reducing the Symptoms of Schizophrenia**

(30) Priority: 07.02.2006 DK 200600175; 07.02.2006 US 771304 P
(62) Divisional of application: 07702527.8
(71) Applicant: H. Lundbeck A/S, 2500 Valby (DK)
(72) Inventor: Bak-Jensen, Henriette Husum, 2300 Copenhagen S. (DK); Tornøe, Christian Wenzel, 2800 Lyngby (DK); Rottländer, Mario, 2670 Greve (DK); Greve, Daniel Rodriquez, 3660 Stenløse (DK); Khanzhin, Nikolay, 3050 Humlebæk (DK); Ritzén, Andreas, 1749 Copenhagen V. (DK); Watson, William Patrick, Coventry, CV3 6HH (GB)
(74) Representative: Kalum, Bo

(57) **Abstract**

The invention relates to a novel method for treating or reducing the symptoms of schizophrenia, said method comprising administering to a host in need thereof an effective amount of a compound able to selectively increase the ion flow through KCNQ potassium channels. Furthermore the invention relates to the use of selective KCNQ potassium channel openers for the preparation of a pharmaceutical composition for treating or reducing the symptoms of schizophrenia and related symptoms, disorders and diseases. Furthermore the invention relates to a method of screening for a compound, which is a selective KCNQ potassium channel opener and which is capable of having an anti-psychotic potential.

## Description

### Field of the invention

The present invention relates to a novel method for treating or reducing the symptoms of schizophrenia, said method comprising administering to a host in need thereof an effective amount of a compound able to selectively increase the ion flow through KCNQ potassium channels. Furthermore the present invention relates to the use of selective KCNQ potassium channel openers for the preparation of a pharmaceutical composition for treating or reducing the symtoms of schizophrenia and related symptoms, disorders and diseases. Furthermore the present invention relates to a method of screening for a compound, which is a selective KCNQ potassium channel opener and which is capable of having an anti-psychotic potential.

### Background of the invention

The dopaminergic system is known to be disrupted in schizophrenia and related disorders (Meltzer and Stahl Schizophrenia Bulletin, 1976, 2, 19-76) and the compounds currently available for the treatment of schizophrenia all modulate the dopaminergic system. These compound do so by inhibiting the signalling properties of a number of brain-expressed receptors, most notably the dopamine D2 receptor. However, a number of other receptors are also involved in the activity of many antipsychotic drugs, including serotonergic, noradrenergic, histaminergic and muscarinic receptors (Scolnick, Schizophrenia Bulletin, 2004, 72, 75-77).

The current antipsychotic compounds all produce a range of side effects in addition to their effect of reducing the symptoms of schizophrenia and related disorders. The nature of the side effects depends upon the exact pharmacology of the compound in question. All clinically used antipsychotics inhibit the dopamine D2 receptor to some degree or other (Seeman et al., Nature 261, 717-719). Those compounds that require a high degree of dopamine D2 receptor block, for example haloperidol, cause extra pyramidal side effects and elevations in prolactin levels. Extra pyramidal side effects include parkinsonism, rigidity, akinesia and after prolonged treatment tardive dyskinesia may develop (Pierre, Drug Safety, 2005, 28, 191-208). Prolactin elevation can cause a number of endocrine disturbances, such as gynaecomastia, galactorrhoea, sexual disfunction, infertility, oligomenorrhoea and amenorrhoea (Haddad and Wieck Drugs, 2004, 64, 2291-2314).

The current antipsychotic compounds, particularly the newer class of atypical antipsychotics, such as olanzapine, quetiapine and risperidone, are also associated with insulin resistance, disturbances in glucose and lipid metabolism, diabetes and excessive weight gain (Melkersson and Dahl, Drugs 2004, 64, 701-723).

In addition, the current antipsychotics may cause 'slowness of thinking', which contributes to the cognitive symptoms of schizophrenia. Furthermore, anhedonia, the decrease in mood, may also occur with some antipsychotics and may appear to worsen the negative symptoms of schizophrenia (Heinz et al, Schizophrenia Research, 1998, 31,19-26).

The current antipsychotics also inadequately treat the symptoms of schizophrenia. The symptoms of schizophrenia fall into three broad categories: positive, negative and cognitive. The positive symptoms are those which represent an 'excess' of normal experience, such as hallucination and delusions. The negative symptoms are those where the patients shows a lack of normal experience, such as anhedonia and lack of social interaction. The cognitive symptoms, relate to the cognitive deficits in schizophrenia, such as lack of sustained attention and deficits in decision making. The current antipsychotics largely treat the positive symptoms of schizophrenia and have limited impact on the negative or cognitive symptoms (Mishara and Goldberg, Biological Psychiatry, 2004, 55, 1013-1022). In addition, the clinical benefit derived from antipsychotics takes several weeks of treatment to develop. In a recent large comparative study (the CATIE study) approximately 30-40% of patients discontinued treatment (switched to another drug) because of lack of efficacy (Lieberman et al New England Journal Of Medicine, 2005, 353, 1209-1223).

Major depressive disorder is a chronic recurring disease with considerable morbidity in the general population. The hallmark of the disease is a depressed mood. The clinical picture may be further characterised by anhedonic symptoms, sleep disturbances, psychomotor agitation or retardation, sexual dysfunction, weight loss, concentration difficulties and delusional ideas. However, the most serious complication of a depressive episode is that of suicidal ideation leading to suicide attempts (DSM IV, American Psychiatric Association, Washington D.C. 1994). Consequently, it is the goal of treatment of the depression that the symptoms are effectively alleviated, the treatment is safe and highly tolerable and the treatment has an early on set of effect.

Bipolar disorder, previously referred to as manic-depressive illness, is characterised by episodes of depression and mania (type I) or episodes of depression and hypomania (type II). The symptoms of a bipolar depressive episode are not different from those characterising a major depressive episode. This is also the reason why many bipolar patients are initially diagnosed as suffering from major depression. However, it is the occurrence of manic or hypomanic episodes that give rise to a bipolar diagnosis, which is distinct from a major depression diagnosis. Bipolar disorders are life-threatening conditions since patients diagnosed with a bipolar disorder have an estimated suicide risk 15 times higher than in the general population (Harris and Barraclough, 1997, British Journal of Psychiatry, 170:205-228). At present bipolar disorder is treated by maintaining the bipolar patients on moodstabilisers (mainly lithium or antiepileptics) and adding antimanic agents (lithium or antipsychotics) or antidepressants (tricyclic antidepressants or selective serotonin re-uptake inhibitors) when the patients relapse into a manic or depressive episode, respectively (Liebermann and Goodwin, Curr Psychiatry Rep. 2004, 6:459-65). Thus, there is a desire to develop novel therapeutic treatments for bipolar disorder in order to meet the need of effectively treating *all* three crucial elements in these disorders with only one therapeutic agent: such novel agents should alleviate manic symptoms with a fast onset of action (antimanic activity), alleviate depression symptoms with a fast onset of action (antidepressant activity), prevent the recurrence of mania as well as depression symptoms (mood stabilising activity).

Ion channels are cellular proteins that regulate the flow of ions, including potassium, calcium, chloride and sodium into and out of cells. Such channels are present in all animal and human cells and affect a variety of processes including neuronal transmission, muscle contraction, and cellular secretion.

Humans have over 70 genes encoding potassium channel subtypes (Jentsch Nature Reviews Neuroscience 2000, 1, 21-30) with a great diversity with regard to both structure and function. Neuronal potassium channels, which are found in the brain, are primarily responsible for maintaining a negative resting membrane potential, as well as controlling membrane repolarisation following an action potential.

One subset of potassium channel genes is the KCNQ family. Mutations in four out of five KCNQ genes have been shown to underlie diseases including cardiac arrhythmias, deafness and epilepsy (Jentsch Nature Reviews Neuroscience 2000, 1, 21-30).

KCNQ1 (KvLQT1) is co-assembled with the product of the KCNE1 (minimal K(+)-channel protein) gene in the heart to form a cardiac-delayed rectifier-like K(+) current. Mutations in this channel can cause one form of inherited long QT syndrome type 1 (LQT1), as well as being associated with a form of deafness (Robbins Pharmacol Ther 2001, 90, 1-19).

The genes KCNQ2 and KCNQ3 were discovered in 1998 and appear to be mutated in an inherited form of epilepsy known as benign familial neonatal convulsions (Rogawski Trends in Neurosciences 2000, 23, 393-398). The proteins encoded by the KCNQ2 and KCNQ3 genes are localised in the pyramidal neurons of the human cortex and hippocampus, regions of the brain associated with seizure generation and propagation (Cooper et al. Proceedings National Academy of Science U S A 2000, 97, 4914-4919).

KCNQ2 and KCNQ3 are two potassium channel subunits that form "M-currents" when expressed in vitro. KCNQ5 has also been shown to contribute to the M-current in cultured hippocampal neurons (Shah et al., Journal of Physiology 2002, 544, 29-37). KCNQ4 potassium channels have been shown to possess M-current-like properties when expressed in cell lines (Søgaard et al., American Journal of Physiology and Cellular Physiology, 2001, 280, C859-C866). The M-current is a non-inactivating potassium current found in many neuronal cell types. In each cell type, it is dominant in controlling membrane excitability by being the only sustained current in the range of action potential initiation (Marrion Annual Review Physiology 1997, 59, 483-504). Modulation of the M-current has dramatic effects on neuronal excitability, for example activation of the current will reduce neuronal excitability. Openers of these KCNQ channels or activators of the M-current, will reduce neuronal activity and may thus be of use in the treatment of seizures and other diseases and disorders characterised by excessive neuronal activity, such as neuronal hyperexcitability including convulsive disorders, epilepsy, neuropathic pain, anxiety and schizophrenia.

The KCNQ4 gene is thought to encode the molecular correlate of potassium channels found in outer hair cells of the cochlea and in Type I hair cells of the vestibular apparatus, in which mutations can lead to a form of inherited deafness.

KCNQ2 and KCNQ4 are also expressed in the substantia nigra and ventral tegmental area (Kharkovets et al, 2000 Proceedings National Academy of Science USA, 97, 4333-4338), which contain the cell bodies of two of the major dopaminergic systems in the brain the nigrostriatal and mesolimbic systems respectively. There is functional coupling between dopamine D2 receptors and KCNQ4 channels when expressed in oocytes or SH-SY5Y cells (Ljungstrom et al., European Journal of Physiology, 2003, 446, 684-694), which suggests similar coupling in vivo when the D2 receptor and KCNQ4 channels are expressed in the same cells.

Retigabine (D-23129; N-(2-amino-4-(4-fluorobenzylamino)-phenyl) carbamic acid ethyl ester) and analogues thereof are disclosed in EP554543. Retigabine is an anti-convulsive compound with a broad spectrum and potent anticonvulsant properties, both in vitro and in vivo. It is active after oral and intraperitoneal administration in rats and mice in a range of anticonvulsant tests (Rostock et al. Epilepsy Research 1996, 23, 211-223). In clinical trials, retigabine has recently shown effectiveness in reducing the incidence of seizures in epileptic patients (Bialer et al. Epilepsy Research 2002, 51, 31-71).

Retigabine has been shown to activate a K(+) current in neuronal cells and the pharmacology of this induced current displays concordance with the published pharmacology of the M-channel. Retigabine has also been shown to bind to KCNQ channels (Wuttke et al, Molecular Pharmacology, 2005, 67,1009-1017). These data suggest that activation of KCNQ channels is responsible for at least some of the anticonvulsant activity of this agent (Wickenden et al. Molecular Pharmacology 2000, 58, 591-600) - and that other agents working by the same mechanism may have similar uses.

Retigabine has been shown to suppress the firing of dopaminergic neurons in the ventral tegmental area *ex vivo* (Hansen et al., *Society for Neuroscience Abstracts,* 2005, 153.11). However, it is not known whether this effect of retigabine translates into an *in vivo* inhibition of dopaminergic neurons in the ventral tegmental area, or whether this effect is associated with anti-psychotic-like behaviour in animals.

Thus there is a great desire for compounds that are effective in the treatment of the symptoms of schizophrenia and related diseases and disorders, but have reduced propensity to cause or are devoid of the common side effects of antipsychotics, which are extra pyramidal side effects, prolactin elevation, weight gain, disturbances in glucose and lipid metabolism and cardiovascular problems. Additionally a great desire exists for compounds with a fast onset of action that are effective in the treatment of schizophrenia and related diseases and disorders. Moreover a great desire exists for compounds that exhibit a significantly greater efficacy in treating the positive, the negative and the cognitive symptoms of schizophrenia and may treat a greater percentage of patients than currently benefit from existing antipsychotic drugs. Additionally a great desire exists for a treatment where compliance can be improved.

It has now for the first time surprisingly been found that compounds that activate KCNQ channels are able to modulate the dopaminergic system in vivo and display efficacy in the commonly used animal models of schizophrenia.

### Summary of the invention

In a first aspect the present invention relates to a method for treating or reducing the symptoms of schizophrenia, said method comprising administering to a host in need thereof an effective amount of a compound able to selectively increase the ion flow through KCNQ potassium channels.

In a second aspect the present invention, relates to a method for treating or reducing the symptoms of schizophrenia, said method comprising administering to a host in need thereof an effective amount of a compound able to selectively increase the ion flow through KCNQ potassium channels and wherein said compound able to increase the ion flow through KCNQ potassium channels is effective in a model predictive for an anti-psychotic potential of said compound.

In a third aspect the present invention, relates to a method for treating or reducing the symptoms of schizophrenia, said method comprising administering to a host in need thereof an effective amount of a compound able to selectively increase the ion flow through KCNQ potassium channels and wherein said compound does not to any reasonably extent manifest any side-effects associated with compounds known to treat schizophrenia.

In a fourth aspect the present invention, relates to a method for treating or reducing the symptoms of schizophrenia, said method comprising administering to a host in need thereof an effective amount of a compound able to selectively increase the ion flow through KCNQ potassium channels and wherein said compound is administered in an amount of more than 1 mg/day

In a fifth aspect the present invention, relates to a method for treating or reducing the symptoms of schizophrenia, said method comprising administering to a host in need thereof an effective amount of a compound able to selectively increase the ion flow through KCNQ potassium channels and wherein said compound has a fast-onset of action

In a sixth aspect the present invention, relates to a method for treating or reducing the symptoms of schizophrenia, said method comprising administering to a host in need thereof an effective amount of a compound able to selectively increase the ion flow through KCNQ potassium channels wherein said compound is a compound according to formula 1, 2, 3, 4, 5, 6, 7, 8 or 9,
where formula 1 is: 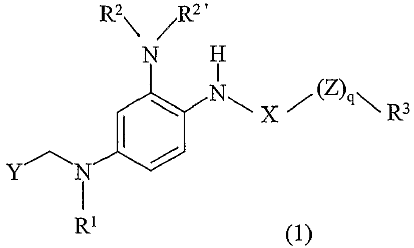 wherein
**R¹** is selected from the group consisting of hydrogen, C₁₋₆-alk(en/yn)yl, C₃₋₈-cycloalk(en)yl, C₃-₈-cycloalk(en)yl-C₁-₆-alk(en/yn)yl, acyl, hydroxy-C₁₋₆-alk(en/yn)yl and hydroxy-C₃₋₈-cycloalk(en)yl;
**R²** and **R^{2'}** are independently selected from the group consisting of hydrogen, C₁₋₆-alk(en/yn)yl, C₃₋₈-cycloalk(en)yl, aryl, C₃₋₈-cycloalk(en)yl-C₁₋₆-alk(en/yn)yl, aryl-C₁₋₆-alk(en/yn)yl, acyl, hydroxy-C₁₋₆-alk(en/yn)yl and hydroxy-C₃₋₈-cycloalk(en)yl;
**R³** is selected from the group consisting of hydrogen, C₁₋₆-alk(en/yn)yl, C₃₋₈-cycloalk(en)yl, aryl, C₃₋₈-cycloalk(en)yl-C₁₋₆-alk(en/yn)yl, aryl-C₁₋₆-alk(en/yn)yl, hydroxy-C₁₋₆-alk(en/yn)yl, aryl-C₃₋₈-cycloalk(en)yl, NR¹⁰R^{10'}-C₁₋₆-alk(en/yn)yl, NR¹⁰R^{10'}-C₃₋₈-cycloalk(en)yl and hydroxy-C₃₋₈-cycloalk(en)yl; wherein
**R¹⁰** and **R^{10'}** are independently selected from the group consisting of hydrogen, C₁₋₆-alk(en/yn)yl, C₃₋₈-cycloalk(en)yl, C₃-₈-cycloalk(en)yl-C₁₋₆-alk(en/yn)yl, hydroxy-C₁₋₆-alk(en/yn)yl, hydroxy-C₃-₈-cycloalk(en)yl, hydroxy-C₃₋₈-cycloalk(en)yl-C₁₋₆-alk(en/yn)yl, halo-C₁₋₆-alk(en/yn)yl, halo-C₃-₈-cycloalk(en)yl, halo-C₃₋₈-cycloalk(en)yl-C₁₋₆-alk(en/yn)yl, cyano-C₁₋₆-alk(en/yn)yl, cyano-C₃₋₈-cycloalk(en)yl and cyano-C₃₋₈-cycloalk(en)yl-C₁₋₆-alk(en/yn)yl, or **R¹⁰** and **R^{10'}** together with the nitrogen atom to which they are attached form a 4-8 membered saturated or unsaturated ring which optionally contains 1, 2 or 3 further heteroatoms;
X is CO or SO₂;
**Z** is O or NR⁴, wherein
**R⁴** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, hydroxy-C₁₋₆-alk(en/yn)yl and hydroxy-C₃₋₈-cycloalk(en)yl; or
**R³** and **R⁴** together with the nitrogen atom to which they are attached form a 4-8 membered saturated or unsaturated ring which optionally contains 1, 2 or 3 further heteroatoms, the ring formed by **R³** and **R⁴** and the nitrogen atom is optionally substituted with one or more substituents independently selected from C₁₋₆-alk(en/yn)yl, aryl and aryl-C₁₋₆-alk(en/yn)yl;
**q** is 0 or 1;
and
**Y** represents a heteroaryl of formula II or III 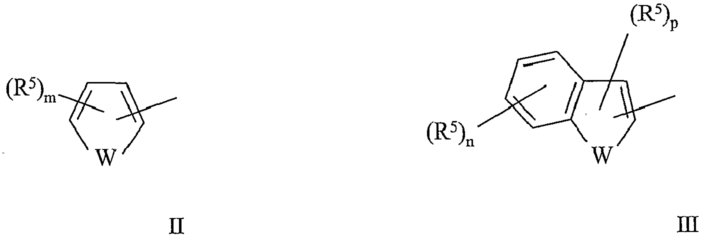 wherein
**W** is 0 or S;
**m** is 0,1,2 or 3;
**n** is 0, 1, 2, 3 or 4;
**p** is 0 or 1; and
each **R⁵** is independently selected from the group consisting of C₁₋₆-alk(en/yn)yl, C₃₋₈-cycloalk(en)yl, aryl, C₃₋₈-cycloalk(en)yl-C₁₋₆-alk(en/yn)yl, aryl-C₁₋₆-alk(en/yn)yl, acyl, halogen, halo-C₁₋₆-alk(en/yn)yl, C₁₋₆-alk(en/yn)yloxy, -CO-NR⁶R^{6'}, cyano, nitro, -NR⁷R^{7'}, -S-R⁸, -SO₂R⁸, SO₂OR⁸_{,}
wherein
**R⁶** and **R^{6'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl and aryl;
**R⁷** and **R^{7'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, aryl and acyl; and
**R⁸** is selected from the group consisting of C₁₋₆-alk(en/yn)yl, C₃₋₈-cycloalk(en)yl, C₃-₈-cycloalk(en)yl-C₁₋₆-alk(en/yn)yl, aryl and -NR⁹R^{9'}; wherein
**R⁹** and **R^{9'}** are independently selected from the group consisting of hydrogen, C₁₋₆-alk(en/yn)yl, C₃₋₈-cycloalk(en)yl and C₃₋₈-cycloalk(en)yl-C₁₋₆-alk(en/yn)yl;
or pharmaceutically acceptable salts thereof; and
where formula 2 is: 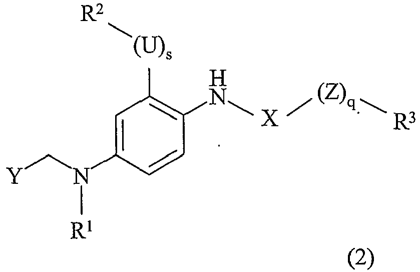 wherein
**S** is 0 or 1;
**U** is O, S, SO₂ SO₂N**R¹¹**, CO-O or CON**R¹¹**; wherein
**R¹¹** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl; or **R²** and **R¹¹** together with the nitrogen atom form a 5-8 membered saturated or unsaturated ring which optionally contains 1, 2 or 3 further heteroatoms;
**q** is 0 or 1;
**X** is CO or SO₂; with the proviso that **q** is 0 when **X** is SO₂;
**Z** is O or S;
**R¹** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, acyl, hydroxy-C₁₋₆-alk(en/yn)yl, hydroxy-C₃₋₈-cycloalk(en)yl, hydroxy-C₃-₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, cyano-C₁₋₆-alk(en/yn)yl, cyano-C₃₋₈-cycloalk(en)yl and cyano-C₃₋₈-cycloalk(en)yl-C₁₋₆-alk(en/yn)yl;
**R²** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar, Ar-C₁₋₆-alk(en/yn)yl, Ar-C₃₋₈-cycloalk(en)yl, Ar-C₃₋₈-cycloalk(en)yl-C₁₋₆-alk(en/yn)yl, acyl, hydroxy-C₁₋₆-alk(en/yn)yl, hydroxy-C₃₋₈-cycloalk(en)yl, hydroxy-C₃₋₈-cycloalk(en)yl-C₁₋₆-alk(en/yn)yl, halogen, halo-C₁₋₆-alk(en/yn)yl, halo-C₃₋₈-cycloalk(en)yl, halo-C₃₋₈-cycloalk(en)yl-C₁₋₆-alk(en/yn)yl, cyano, cyano-C₁₋₆-alk(en/yn)yl, cyano-C₃₋₈-cycloalk(en)yl, cyano-C₃₋₈-cycloalk(en)yl-C₁₋₆-alk(en/yn)yl, N**R¹⁰R^{10'}**-C₁₋₆-alk(en/yn)yl, NR**¹⁰R^{10'}**-C₃₋₈-cycloalk(en)yl and N**R¹⁰R^{10'}**-C₃₋₈-cycloalk(en)yl-C₁₋₆-alk(en/yn)yl; wherein
**R¹⁰** and **R^{10'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, hydroxy-C₁₋₆-alk(en/yn)yl, hydroxy-C₃₋₈-cycloalk(en)yl, hydroxy-C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, cyano-C₁₋₆-alk(en/yn)yl, cyano-C₃₋₈-cycloalk(en)yl and cyano-C₃₋₈-cycloalk(en)yl-C₁₋₆-alk(en/yn)yl, or **R¹⁰** and **R^{10'}** together with the nitrogen atom form a 5-8 membered saturated or unsaturated ring which optionally contains 1, 2 or 3 further heteroatoms; provided that when **R²** is halogen or cyano then s is 0; and provided that **U** is O or S when **s** is 1 and **R²** is a hydrogen atom or acyl;
**R³** is selected from the group consisting of C₁₋₆-alk(en/yn)yl, C₃₋₈-cycloalk(en)yl, heterocycloalk(en)yl, C₃₋₈-cycloalk(en)yl-C₁₋₆-alk(en/yn)yl, C₁₋₆-alk(en/yn)yl-C₃₋₈-cycloalk(en)yl, C₁₋₆-alk(en/yn)yl-heterocycloalk(en)yl, heterocycloalk(en)yl-C₁-₆-alk(en/yn)yl, Ar, Ar-C₁₋₆-alk(en/yn)yl, Ar-C₃₋₈-cycloalk(en)yl, Ar-heterocycloalk(en)yl, Ar-C₃₋₈-cycloalk(en)yl-C₁₋₆-alk(en/yn)yl, Ar-C₁₋₆-alk(en/yn)yl-C₃₋₈-cycloalk(en)yl, Ar-C₁₋₆-allc(en/yn)yl-heterocycloalk(en)yl, C₁-₆-alk(en/yn)yloxy-C₁₋₆-alk(en)/yn)yl, C₃₋₈-cycloalk(en)yloxy-C₁₋₆-alk(en/yn)yl, C₁₋₆-alk(en/yn)yloxy-C₃₋₈-cycloalk(en)yl, C₁₋₆-alk(en/yn)yloxy-heterocycloalk(en)yl, Ar-oxy-C₁₋₆-alk(en/yn)yl, Ar-C₁₋₆-alk(en/yn)yloxy-C₁₋₆-alk(en/yn)yl, C₁₋₆-alk(en/yn)yloxy-carbonyl-C₁₋₆-alk(en/yn)yl, C₃₋₈-cycloalk(en)yloxy-carbonyl-C₁-₆-alk(en/yn)yl, C₃₋₈-cycloalk(en)yl-C₁₋₆-alk(en/yn)yloxy-carbonyl-C₁₋₆-alk(en/yn)yl, hydroxy-C₁₋₆-alk(en/yn)yl, hydroxy-C₃₋₈-cycloalk(en)yl, hydroxy-heterocycloalk(en)yl, hydroxy-C₃₋₈-cycloalk(en)yl-C₁₋₆-alk(en/yn)yl, hydroxy-C₁₋₆-alk(en/yn)yl-C₃₋₈-cycloalk(en)yl, hydroxy-C₁₋₆-alk(en/yn)yl-heterocycloalk(en)yl, halo-C₁₋₆-alk(en/yn)yl, halo-C₃₋₈-cycloalk(en)yl, halo-heterocycloalk(en)yl, halo-C₃₋₈-cycloalk(en)yl-C₁₋₆-alk(en/yn)yl, halo-C₁₋₆-alk(en/yn)yl-C₃₋₈-cycloalk(en)yl, halo-C₁₋₆-alk(en/yn)yl-heterocycloalk(en)yl, halo-C₁₋₆-alk(en/yn)yl-Ar, halo-C₃₋₈-cycloalk(en)yl-Ar, halo-C₃₋₈-cycloalk(en)yl-C₁₋₆-alk(en/yn)yl-Ar, halo-C₁₋₆-alk(en/yn)yl-C₃₋₈-cycloalk(en)yl-Ar, cyano-C₁₋₆-alk(en/yn)yl, cyano-C₃₋₈-cycloalk(en)yl, cyano-heterocycloalk(en)yl, cyano-C₃₋₈-cycloalk(en)yl-C₁₋₆-alk(en/yn)yl, cyano-C₁₋₆-alk(en/yn)yl-C₃₋₈-cycloalk(en)yl, cyano-C₁₋₆-alk(en/yn)yl-heterocycloalk(en)yl, acyl-C₁₋₆-alk(en/yn)yl, acyl-C₃₋₈-cycloalk(en)yl, acyl-heterocycloalk(en)yl, acyl-C₃₋₈-cycloalk(en)yl-C₁₋₆-alk(en/yn)yl, acyl-C₁₋₆-alk(en/yn)yl-C₃₋₈-cycloalk(en)yl, acyl-C₁₋₆-alk(en/yn)yl-heterocycloalk(en)yl, N**R¹²R¹²**', optionally substituted N**R¹²R¹²'**-C₁₋₆--alk(en/yn)yl, optionally substituted N**R¹²R¹²'-**C3.8-cycloalk(en)yl, optionally substituted N**R¹²R^{12'}** -C₃₋₈-cycloalk(en)yl-C₁₋₆-alk(en/yn)yl; wherein **R¹²** and **R^{12'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar, Ar-C₁₋₆-alk(en/yn)yl, Ar-C₃₋₈-cycloalk(en)yl, Ar-C₃₋₈-cycloalk(en)yl-C₁₋₆-alk(en/yn)yl, Ar-heterocycloalk(en)yl, Ar-oxy-C₁₋₆-alk(en/yn)yl, Ar-oxy-C₃₋₈-cycloalk(en)yl, Ar-oxy-C₃₋₈-cycloalk(en)yl-C₁₋₆-alk(en/yn)yl, Ar-oxy-heterocycloalk(en)yl, hydroxy-C₁₋₆-alk(en/yn)yl, hydroxy-C₃₋₈-cycloalk(en)yl, hydroxy-C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, cyano-C₁₋₆-alk(en/yn)yl, cyano-C₃₋₈-cycloalk(en)yl and cyano-C₃₋₈-cycloalk(en)yl-C₁₋₆-alk(en/yn)yl, or
**R¹²** and **R**^{**12**'} together with the nitrogen atom form a 5-8 membered saturated or unsaturated ring which optionally contains 1, 2 or 3 further heteroatoms;
with the proviso that when **R³** is N**R¹²R**^{**12**'} then q is 0;
and
**Y** represents a group of formula **XXIV, XXV**, **XXVI**, **XXVII**, **XXVIII**, **XXXXI** or **XXXXII:** 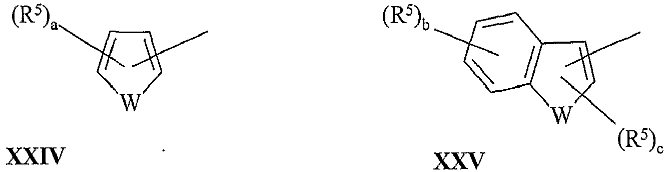 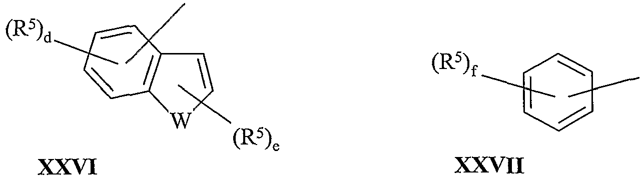 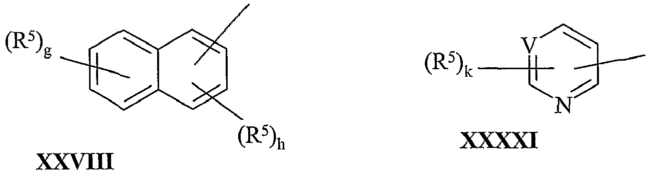 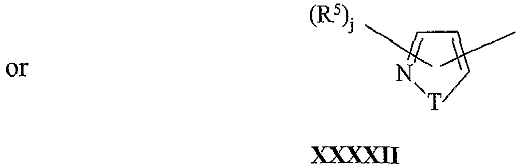 wherein
the line represents a bond attaching the group represented by **Y** to the carbon atom;
**W** is O or S;
**V** is N, C or CH;
**T** is N, NH or O;
**a** is 0, 1, 2 or 3;
**b** is 0, 1, 2, 3 or 4;
**c** is 0 or 1;
**d** is 0, 1, 2 or 3;
**e** is 0, 1 or 2;
**f** is 0, 1, 2, 3, 4 or 5;
**g** is 0, 1, 2, 3 or 4;
**h** is 0, 1, 2 or 3;
**j** is 0, 1 or 2;
**k** is 0, 1, 2 or 3; and
each **R⁵** is independently selected from the group consisting of a C₁₋₆-alk(en/yn)yl, C₃₋₈-cycloalk(en)yl, C₃₋₈-cycloalk(en)yl-C₁₋₆-alk(en/yn)yl, Ar, ArC₁₋₆-alk(en/yn)yl, Ar-C₃₋₈-cycloalk(en)yl, Ar-C₃₋₈-cycloalk(en)yl-C₁₋₆-alk(en/yn)yl, Ar-oxy, Ar-oxy-C₁₋₆-alk(en/yn)yl, Ar-oxy-C₃₋₈-cycloalk(en)yl, C₁₋₆-alk(en/yn)yl-heterocycloalk(en)yl, Ar-oxy-C₃₋₈-cycloalk(en)yl-C₁₋₆-alk(en/yn)yl, acyl, C₁₋₆-alk(en/yn)yloxy, C₃₋₈-cycloalk(en)yloxy, C₃₋₈-cycloalk(en)yl-C₁₋₆-alk(en/yn)yloxy, C₁₋₆-alk(en/yn)yloxy-carbonyl, halogen, halo-C₁₋₆-alk(en/yn)yl, halo-C₃₋₈-cycloalk(en)yl, halo-C₃₋₈-cycloalk(en)yl-C₁₋₆-alk(en/yn)yl, -CO-NR⁶R^{6'}, cyano, cyano-C₁₋₆-alk(en/yn)yl, cyano-C₃₋₈-cycloalk(en)yl, cyano-C₃₋₈-cycloalk(en)yl-C₁₋₆-alk(en/yn)yl, N**R⁷R^{7'},** S-**R⁸** and SO₂**R⁸**, or two adjacent **R⁵** together with the aromatic group form a 5-8 membered ring which optionally contains one or two heteroatoms;
**R**⁶ and **R**^{6'} are independently selected from the group consisting of hydrogen, C₁-₆-alk(en/yn)yl, C₃₋₈-cycloalk(en)yl, C₃₋₈-cycloalk(en)yl-C₁₋₆-alk(en/yn)yl and Ar;
**R⁷** and **R**^{**7**'} are independently selected from the group consisting of hydrogen, C₁-₆-alk(en/yn)yl, C₃₋₈-cycloalk(en)yl, C₃₋₈-cycloalk(en)yl-C₁₋₆-alk(en/yn)yl, Ar, heterocycloalk(en)yl-C₁₋₆-alk(en/yn)yl, heterocycloalk(en)yl-C₃₋₈-cycloalk(en)yl, heterocycloalk(en)yl-C₃₋₈-cycloalk(en)yl-C₁₋₆-alk(en/yn)yl, heterocycloalk(en)yl-Ar and acyl; or
**R⁷** and **R^{7'}** together with the nitrogen atom form a 5-8 membered saturated or unsaturated ring which optionally contains 1, 2 or 3 further heteroatoms; and
**R⁸** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar and -N**R⁹R^{9'}**; wherein **R⁹** and **R^{9'}** are independently selected from the group consisting of hydrogen, C₁₋₆-alk(en/yn)yl, C₃₋₈-cycloalk(en)yl and C₃₋₈-cycloalk(en)yl-C₁₋₆-alk(en/yn)yl;
or pharmaceutically acceptable salts thereof; and
where formula 3 is: 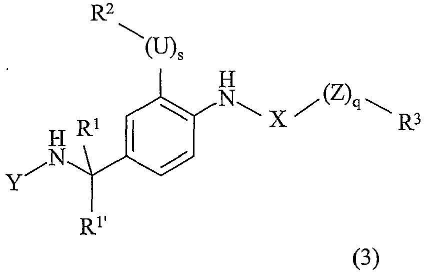 wherein
**U** is O, S or NR^{2'} ;
**s** is 0 or 1;
**X** is CO or SO₂;
**Z** is O, S or N**R**⁴, wherein **R**⁴ is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, hydroxy-C₁₋₆-alk(en/yn)yl and hydroxy-C₃₋₈-cycloalk(en)yl;
**q** is 0 or 1;
**R¹** and **R**^{**1**'} are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, acyl, hydroxy-C₁₋₆-alk(en/yn)yl, hydroxy-C₃₋₈-cycloalk(en)yl, halo-C₁₋₆-alk(en/yn)yl and halo-C₃₋₈-cyloalk(en)yl;
**R²** is selected from the group consisting of hydrogen, halogen, C₁₋₆-alk(en/yn)yl, C₃₋₈-cycloalk(en)yl, C₃₋₈-cycloalk(en)yl-C₁₋₆-alk(en/yn)yl, Ar, Ar-C₁₋₆-alk(en/yn)yl, Ar-C₃₋₈-cycloalk(en)yl, acyl, hydroxy-C₁₋₆-alk(en/yn)yl, hydroxy-C₃₋₈-cycloalk(en)yl, halo-C₁₋₆-alk(en/yn)yl, halo-C₃₋₈-cycloalk(en)yl and cyano; provided that when **R²** is halogen or cyano, then s is 0;
when s is 1 and **U** is N**R**^{**2**'} then **R^{2'}** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar, Ar-C₁₋₆-alk(en/yn)yl, Ar-C₃₋₈-cycloalk(en)yl, acyl, hydroxy-C₁-₆-alk(en/yn)yl, hydroxy-C₃₋₈-cycloalk(en)yl, halo-C₁₋₆-alk(en/yn)yl and halo-C₃₋₈-cycloalk(en)yl; or **R²** and **R**^{**2**'} together form a 5-8 membered saturated or unsaturated ring which optionally contains one further heteroatom;
**R**³ is selected from the group consisting of C₁₋₆-alk(en/yn)yl, C₃₋₈-cycloalk(en)yl, C₃₋₈-cycloalk(en)yl-C₁₋₆-alk(en/yn)yl, Ar, Ar-C₁₋₆-alk(en/yn)yl, Ar-C₃₋₈-cycloalk(en)yl, hydroxy-C₁₋₆-alk(en/yn)yl, hydroxy-C₃₋₈-cycloalk(en)yl, halo-C₁₋₆-alk(en/yn)yl and halo-C₃₋₈-cycloalk(en)yl;
and
**Y** represents a group of formulae **VI**, **VII, VIII**, **IX** or **XXX:** 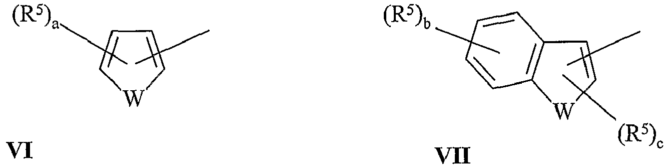 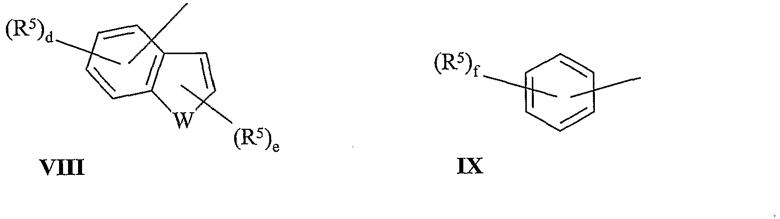 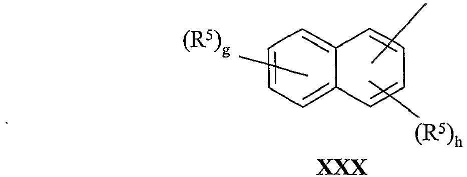 wherein
the line represents a bond attaching the group represented by Y to the nitrogen atom;
**W** is O or S;
**a** is 0, 1, 2 or 3;
**b** is 0, 1, 2, 3 or 4;
**c** is 0 or 1;
**d** is 0, 1, 2 or 3;
**e** is 0, 1 or 2;
**f** is 0, 1, 2, 3, 4 or 5;
**g** is 0, 1, 2, 3 or 4;
**h** is 0, 1, 2 or 3; and
each **R⁵** is independently selected from the group consisting of a C₁₋₆-alk(en/yn)yl, C₃₋₈-cycloalk(en)yl, Ar, C₃₋₈-cycloalk(en)yl-C₁₋₆-alk(en/yn)yl, ArC₁₋₆-alk(en/yn)yl, acyl, C₁₋₆-alk(an/en/yn)yloxy, halogen, halo-C₁₋₆-alk(en/yn)yl, -CO-**NR⁶R**^{**6**'}, cyano, nitro, -N**R⁷R^{7'}**, -S-**R⁸**, -SO₂**R⁸** and SO₂O**R⁸**, or two substituents together form a 5-8 membered saturated or unsaturated ring which optionally contains one or two heteroatoms;
**R⁶** and **R^{6'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl and Ar;
**R⁷** and **R^{7'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar and acyl; and
**R⁸** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar and -NR⁹R^{9'}; wherein **R⁹** and **R^{9'}** are independently selected from the group consisting of hydrogen, C₁₋₆-alk(en/yn)yl, C₃₋₈-cycloalk(en)yl and C₃₋₈-cycloalk(en)yl-C₁₋₆-alk(en/yn)yl; with the provisos that when **R⁵** is SO₂O**R⁸** then **R⁸** is not -N**R⁹R^{9'}** and when **R⁵** is SO₂R⁸ , then **R⁸** is not a hydrogen atom;
or pharmaceutically acceptable salts thereof; and
where formula 4 is: 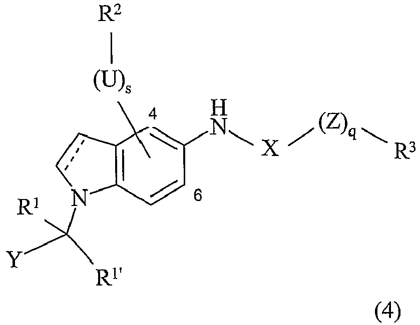 wherein
the dotted line represents an optional bond;
**R¹** and **R^{1'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, hydroxy-C₁₋₆-alk(en/yn)yl, hydroxy-C₃₋₈-cycloalk(en)yl, hydroxy-C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, cyano-C₁₋₆-alk(en/yn)yl, cyano-C₃₋₈-cycloalk(en)yl and cyano-C₃₋₈-cycloalk(en)yl-C₁₋₆-alk(en/yn)yl; or **R¹** and **R^{1'}** together with the carbon atom to which they are attached form a 3-8 membered saturated or unsaturated ring which optionally contains 1 or 2 heteroatoms;
**s** is 0 or 1;
**U** is O, N**R¹¹**, S, SO₂, SO₂N**R¹¹**, CO-O or CO-N**R¹¹**; wherein **R¹¹** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl; or **R²** and **R¹¹** together with the nitrogen atom to which they are attached form a 4-8 membered saturated or unsaturated ring which optionally contains 1, 2 or 3 further heteroatoms;
**R²** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar, Ar-C₁₋₆-alk(en/yn)yl, Ar-C₃₋₈-cycloalk(en)yl, Ar-C₃₋₈-cycloalk(en)yl-C₁₋₆-alk(en/yn)yl, acyl, hydroxy-C₁₋₆-alk(en/yn)yl, hydroxy-C₃₋₈-cycloalk(en)yl, hydroxy-C₃₋₈-cycloalk(en)yl-C₁₋₆-alk(en/yn)yl, halogen, halo-C₁₋₆-alk(en/yn)yl, halo-C₃₋₈-cycloalk(en)yl, halo-C₃₋₈-cyloalk(en)yl-C₁₋₆-alk(en/yn)yl, cyano, cyano-C₁₋₆-alk(en/yn)yl, cyano-C₃₋₈-cycloalk(en)yl, cyano-C₃₋₈-cycloalk(en)yl-C₁₋₆-alk(en/yn)yl, -NO₂, N**R¹⁰R^{10'}**-C₁₋₆-alk(en/yn)yl, N**R¹⁰R¹⁰**-C₃₋₈-cycloalk(en)yl and N**R¹⁰R**^{**10**'}-C₃₋₈-cycloalk(en)yl-C₁₋₆-alk(en/yn)yl; wherein
**R¹⁰** and **R^{10'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, hydroxy-C₁₋₆-alk(en/yn)yl, hydroxy-C₃₋₈-cycloalk(en)yl, hydroxy-C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, cyano-C₁₋₆-alk(en/yn)yl, cyano-C₃₋₈-cycloalk(en)yl and cyano-C₃₋₈-cycloalk(en)yl-C₁₋₆-alk(en/yn)yl, or **R¹⁰** and **R^{10'}** together with the nitrogen atom to which they are attached form a 4-8 membered saturated or unsaturated ring which optionally contains 1, 2 or 3 further heteroatoms;
with the proviso that when **R²** is NO₂**,** halogen or cyano then **s** is 0; and with the proviso that when **R²** is a hydrogen atom or acyl and s is 1 then **U** is N**R¹¹**, O or S;
wherein the group **-(U)ₛ-R²** is linked to position 4 or 6 of the indole or indoline;
**q** is 0 or 1;
**Z** is O or S;
**X** is CO or SO₂; with the proviso that **q** is 0 when **X** is SO₂;
**R³** is selected from the group consisting of C₁₋₆₋alk(en/yn)yl, C₃₋₈-cycloalk(en)yl, heterocycloalk(en)yl, C₃₋₈-cycloalk(en)yl-C₁₋₆-alk(en/yn)yl, C₁₋₆-alk(en/yn)yl-C₃₋₈-cycloalk(en)yl, C₁₋₆-alk(en/yn)yl-heterocycloalk(en)yl, Ar, Ar-C₁₋₆-alk(en/yn)yl, Ar-C₃₋₈-cycloalk(en)yl, Ar-heterocycloalk(en)yl, Ar-C₃₋₈-cycloalk(en)yl-C₁₋₆-alk(en/yn)yl, Ar-C₁₋₆-alk(en/yn)yl-C₃₋₈-cycloalk(en)yl, Ar-C₁₋₆-alk(en/yn)yl-heterocycloalk(en)yl, C₁₋₆-alk(en/yn)yloxy-C₁₋₆-alk(en/yn)yl, C₃₋₈-cycloalk(en)yloxy-C₁₋₆-alk(en/yn)yl, C₁₋₆-alk(en/yn)yloxy-C₃₋₈-cycloalk(en)yl, C₁₋₆-alk(en/yn)yloxy-heterocycloalk(en)yl, Ar-oxy-C₁₋₆-alk(en/yn)yl, Ar-C₁₋₆-alk(en/yn)yloxy-C₁₋₆-alk(en/yn)yl, C₁₋₆-alk(en/yn)yloxy-carbonyl-C₁₋₆-alk(en/yn)yl, C₃₋₈-cycloalk(en)yloxy-carbonyl-C₁₋₆-alk(en/yn)yl, C₃₋₈-cycloalk(en)yl-C₁₋₆-alk(en/yn)yloxy-carbonyl-C₁₋₆-alk(en/yn)yl, hydroxy-C₁₋₆-alk(en/yn)yl, hydroxy-C₃₋₈-cycloalk(en)yl, hydroxy-heterocycloalk(en)yl, hydroxy-C₃₋₈-cyloalk(en)yl-C₁₋₆-alk(en/yn)yl, hydroxy-C₁₋₆-alk(en/yn)yl-C₃₋₈-cycloalk(en)yl, hydroxy-C₁₋₆-alk(en/yn)yl-heterocycloalk(en)yl, halo-C₁₋₆-alk(en/yn)yl, halo-C₃₋₈-cycloalk(en)yl, halo-heterocycloalk(en)yl, halo-C₃₋₈-cycloalk(en)yl-C₁₋₆-alk(en/yn)yl, halo-C₁₋₆-alk(en/yn)yl-C₃₋₈-cycloalk(en)yl, halo-C₁₋₆-alk(en/yn)yl-heterocycloalk(en)yl, halo-C₁₋₆-alk(en/yn)yl-Ar, halo-C₃₋₈-cycloalk(en)yl-Ar, halo-C₃₋₈-cycloalk(en)yl-C₁₋₆-alk(en/yn)yl-Ar, halo-C₁₋₆-alk(en/yn)yl-C₃₋₈-cycloalk(en)yl-Ar, cyano-C₁₋₆-alk(en/yn)yl, cyano-C₃₋₈-cycloalk(en)yl, cyano-heterocycloalk(en)yl, cyano-C₃₋₈-cycloalk(en)yl-C₁₋₆-alk(en/yn)yl, cyano-C₁₋₆-alk(en/yn)yl-C₃₋₈-cycloalk(en)yl, cyano-C₁₋₆-alk(en/yn)yl-heterocycloalk(en)yl, acyl-C₁₋₆-alk(en/yn)yl, acyl-C₃₋₈-cycloalk(en)yl, acyl-heterocycloalk(en)yl, acyl-C₃₋₈-cycloalk(en)yl-C₁₋₆-alk(en/yn)yl, acyl-C₁₋₆-alk(en/yn)yl-C₃₋₈-cycloalk(en)yl, acyl-C₁₋₆-alk-(en/yn)yl-heterocycloalk(en)yl and -N**R¹²R^{12'}**, optionally substituted N**R¹²R^{12'}**-C₁₋₆-alk(en/yn)yl, optionally substituted N**R¹²R^{12'}**-C₃₋₈-cycloalk(en)yl, optionally substituted N**R¹²R**^{**12**'}-C₃₋₈-cycloalk(en)yl-C₁₋₆-alk(en/yn)yl; wherein **R¹²** and **R^{12'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar, Ar-C₁₋₆-alk(en/yn)yl, Ar-C₃₋₈-cycloalk(en)yl, Ar-C₃₋₈-cycloalk(en)yl-C₁₋₆-alk(en/yn)yl, hydroxy-C₁₋₆-alk(en/yn)yl, hydroxy-C₃₋₈-cycloalk(en)yl, hydroxy-C₃-s-cycloalk(en)yl-Ci-6-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, cyano-C₁₋₆-alk(en/yn)yl, cyano-C₃₋₈-cycloalk(en)yl and cyano-C₃₋₈-cycloalk(en)yl-C₁₋₆-alk(en/yn)yl, or
**R¹²** and **R^{12'}** together with the nitrogen atom to which they are attached form a 4-8 membered saturated or unsaturated ring which optionally contains 1, 2 or 3 further heteroatoms;
with the proviso that when **R³** is N**R¹²R^{12'}** then **q** is 0;
and
Y represents a group of formula **II, III, IV, V,, VI, XXX** and **XXXI:** 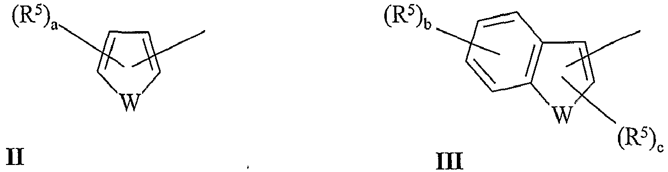 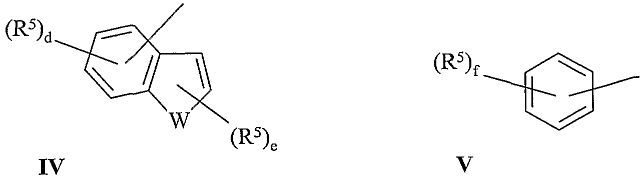 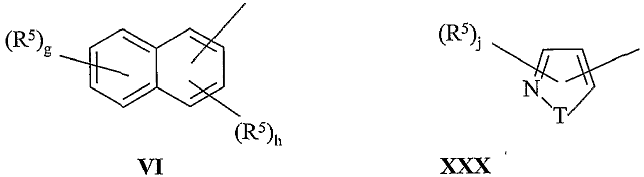 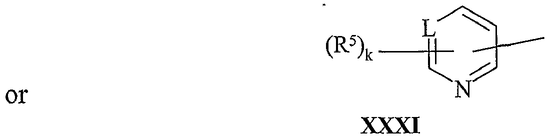 wherein
the line represents a bond attaching the group represented by **Y** to the carbon atom;
**W** is O or S;
**T** is N, NH or O;
**L** is N, C or CH;
**a** is 0, 1, 2 or 3;
**b** is 0, 1, 2, 3 or 4;
**c** is 0 or 1;
**d** is 0, 1, 2 or 3;
**e** is 0, 1 or 2;
**f** is 0, 1, 2, 3, 4 or 5;
**g** is 0, 1, 2, 3 or 4;
**h** is 0, 1, 2 or 3;
**j** is 0, 1, 2 or 3; with the proviso that when T is a nitrogen atom then **j** is 0, 1, 2 or 3; and when **T** is NH or an oxygen atom then **j** is 0, 1 or 2;
**k** is 0, 1, 2, 3 or 4; and
each **R⁵** is independently selected from the group consisting of a C₁₋₆-alk(en/yn)yl, C₃₋₈-cycloalk(en)yl, C₃₋₈-cycloalk(en)yl-C₁₋₆-alk(en/yn)yl, Ar, ArC₁₋₆-alk(en/yn)yl, Ar-thio, Ar-oxy, acyl, C₁₋₆-alk(en/yn)yloxy, C₃₋₈-cycloalk(en)yloxy, C₃₋₈-cycloalk(en)yl-C₁₋₆-alk(en/yn)yloxy, halogen, halo-C₁₋₆-alk(en/yn)yl, halo-C₃₋₈-cycloalk(en)yl, halo-C₃₋₈-cycloalk(en)yl-C₁₋₆-alk(en/yn)yl, -CO-N**R⁶R^{6'}**, cyano, cyano-C₁₋₆-alk(en/yn)yl, cyano-C₃₋₈-cycloalk(en)yl, cyano-C₃₋₈-cycloalk(en)yl-C₁₋₆-alk(en/yn)yl, -N**R⁷R^{7'},** -S-**R⁸** and -SO₂**R⁸,** or
two adjacent **R⁵** together with the aromatic group to which they are attached form a 4-8 membered ring which optionally contains one or two heteroatoms;
**R⁶** and **R^{6'}** are independently selected from the group consisting of hydrogen, C₁-₆-alk(en/yn)yl, C₃₋₈-cycloalk(en)yl, C₃₋₈-cycloalk(en)yl-C₁₋₆-alk(en/yn)yl and Ar;
**R⁷** and **R^{7'}** are independently selected from the group consisting of hydrogen, C₁₋₆-alk(en/yn)yl, C_{3,8}-cycloalk(en)yl, C₃₋₈-cycloalk(en)yl-C₁₋₆-alk(en/yn)yl, Ar and acyl;
and
**R⁸** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar and -N**R⁹R^{9'}**; wherein **R⁹** and **R^{9'}** are independently selected from the group consisting of hydrogen, C₁₋₆-alk(en/yn)yl, C₃₋₈-cycloalk(en)yl and C₃₋₈-cycloalk(en)yl-C₁₋₆-alk(en/yn)yl; provided that when **R⁸** is **-**N**R⁹R^{9'}** then **R⁵** is not **-S-R⁸;**
or pharmaceutically acceptable salts thereof; and
where formula 5 is: 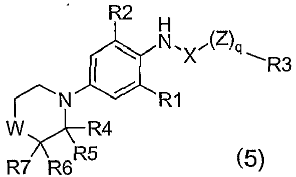 wherein
q is 0 or 1;
W is O or S;
X is CO;
ZisO;
R1 is selected from the group consisting of halogen, cyano, C₁₋₆-alk(en/yn)yl, C₃₋₈-cycloalk(en)yl, C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, C₁₋₆-alk(en/yn)yloxy, C₃₋₈-cycloalk(en)yloxy and C₃₋₈-cycloalk(en)yl-C₁₋₆-alk(en/yn)yloxy;
R2 is selected from the group consisting of halogen, cyano, C₁₋₆-alk(en/yn)yl, C₃₋₈-cycloalk(en)yl, C₃₋₈-cycloalk(en)yl-C₁₋₆-alk(enlyn)yl, halo-C₁₋₆-alk(enlyn)yl, halo-C₃₋₈-cycloallc(en)yl, halo-C₃₋₈-cycloalk(en)yl-C₁₋₆-alk(en/yn)yl, C₁₋₆-alk(en/yn)yloxy, C₃₋₈-cycloalk(en)yloxy, C₃₋₈-cycloalk(en)yl-C₁₋₆-alk(en/yn)yloxy, optionally substituted phenyl and optionally substituted pyridyl; wherein phenyl and pyridyl are optionally substituted with one or more substituents independently being halogen, C₁₋₆-alk(en/yn)yl, C₃₋₈-cyloalk(en)yl or C₃₋₈-cycloalk(en)yl-C₁₋₆-alk(en/yn)yl;
R3 is selected from the group consisting of C₁₋₁₀-alk(en/yn)yl, C₃₋₈-cycloalk(en)yl, C₃₋₈-cycloalk(en)yl-C₁₋₆-alk(en/yn)yl, ArC₁₋₆-alk(en/yn)yl, Ar-C₃₋₈-cycloalk(en)yl, Ar-C₃₋₈-cycloalk(en)yl-C₁₋₆-alk(en/yn)yl and Ar; and
each of R4, R5, R6 and R7 is independently selected from the group consisting of hydrogen and Ar;
or pharmaceutically acceptable salts thereof; and
where formula 6 is: 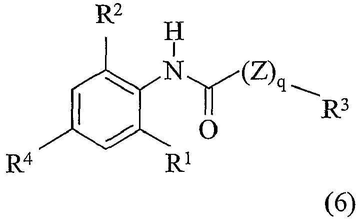 wherein
Z is O or S;
and
q is 0 or 1;
and
each of R¹ and R² is independently selected from the group consisting of halogen, cyano, amino, C₁₋₆-alk(en/yn)yl, C₃₋₈-cycloalk(en)yl, C₃₋₈-cycloalk(en)yl-C₁₋₆-alk(en/yn)yl, C₃₋₈-heterocycloalk(en)yl, Aryl, Heteroaryl, halo-C₁₋₆-alk(en/yn)yl, halo-C₃₋₈-cycloalk(en)yl, halo-C₃₋₈-cycloalk(en)yl-C₁₋₆-alk(en/yn)yl, C₁₋₆-alk(en/yn)yloxy, C₃₋₈-cycloalk(en)yloxy, C₃₋₈-cycloalk(en)yl-C₁₋₆-alk(en/yn)yloxy, C₃₋₈-heterocycloalk(en)yloxy;
and
R³ is selected from the group consisting of C₁₋₈₋alk(en/yn)yl, C₃₋₈-cycloalk(en)yl, C₃₋₈-cycloalk(en)yl-C₁₋₆-alk(en/yn)yl, Aryl-C₁₋₆-alk(en/yn)yl, Aryl-C₃₋₈-cycloalk(en)yl, Aryl-C₃₋₈-cycloalk(en)yl-C₁₋₆-alk(en/un)yl, C₃₋₈-heterocycloalk(en)yl-C₁₋₆-alk(en/yn)yl, C₁₋₆-alk(en/yn)yl-C₃₋₈-heterocycloalk(en)yl-C₁₋₆-alk(en/yn)yl, Heteroaryl-C₁₋₆-alk(en/yn)yl, Heteroaryl-C₃₋₈-cycloalk(en)yl, Heteroaryl-C₃₋₈-cycloalk(en)yl-C₁₋₆-alk(en/yn)yl, amino-C₁₋₆ alk(en/yn)yl, amino-C₃₋₈-cycloalk(en)yl, amino-C₃₋₈-cycloalk(en)yl-C₁₋₆-alk(en/yn)yl, C₁₋₆-alk(en/yn)yloxy-C₁₋₆-alk(en/yn)yl, C₃₋₈-cycloalk(en)yloxy-C₁₋₆-alk(en/yn)yl, C₃₋ₛ-cycloalk(en)yl-C₁₋₆-alk(en/yn)yloxy-C₁₋₆-alk(en/yn)yl, halo-C₁₋₆-alk(en/yn)yl, halo-C₃₋₈-cycloalk(en)yl and halo-C₃₋₈-cycloalk(en)yl-C₁₋₆-alk(en/yn)yl;
and
R⁴ is selected from the group consisting of halogen, cyano, C₁₋₆-alk(en/yn)yl, C₃₋₈-cycloalk(en)yl, C₃₋₈-cycloalk(en)yl-C₁₋₆-alk(en/yn)yl, C₃₋₈-heterocycloalk(en)yl, Aryl, Heteroaryl, Aryl-C₁₋₆-alk(en/yn)yl, Aryl-C₃₋₈-cycloalk(en)yl, Aryl-C₃₋₈₋cycloalk(en)yl-C₁₋₆-allk(en/yn)yl, Aryl-C₃₋₈-heterocycloalk(en)yl, halo-C₁₋₆-alk(en/yn)yl, halo-C₃₋₈-cycloalk(en)yl, halo-C₃₋₈-cycloalk(en)yl-C₁₋₆-alk(en/yn)yl, halo-C₁₋₆-alk(en/yn)yl-C₃₋₈-heterocycloalk(en)yl-C₁₋₆-alk(en/yn)yl, NR⁵R⁶ and R⁷NH-C₁₋₆-alk(en/yn)yl; wherein R⁵ and R⁶ are independently selected from the group consisting of hydrogen, Aryl-C₁₋₆-alk(en/yn)yl, Aryl-C₃₋₈-cycloalk(en)yl, Aryl-C₃₋₈-cycloalk(en)yl-C₁₋₆-alk(en/yn)yl, C₁₋₆-alk(en/yn)yl, C₃₋₈-cycloalk(en)yl, C₃₋₈-cycloalk(en)yl-C₁₋₆-alk(en/yn)yl, Heteroaryl-C₁₋₆-alk(en/yn)yl, Heteroaryl-C₃₋₈-cycloalk(en)yl and Heteroaryl-C₃₋₈-cycloalk(en)yl-C₁₋₆-alk(en/yn)yl with the proviso that R⁵ and R⁶ are not hydrogen at the same time; and R⁷ is selected from the group consisting of C₁₋₆-alk(en/yn)yl, C₃₋₈-cycloalk(en)yl, C₃₋₈-cycloalk(en)yl-C₁₋₆-alk(en/yn)yl, Aryl, halo-C₁₋₆-alk(en/yn)yl, halo-C₃₋₈-cycloalk(en)yl, halo-C₃₋₈-cycloalk(en)yl-C₁₋₆-alk(en/yn)yl, Aryl-C₁₋₆-alk(en/yn)yl, Aryl-C₃₋₈-cycloalk(en)yl and Heteroaryl;
or pharmaceutically acceptable salts thereof; and
where formula 7 is: 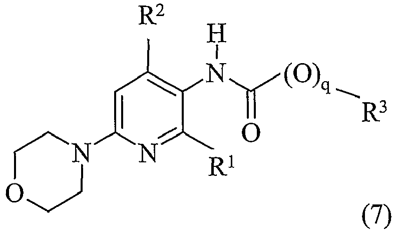 wherein:
q is 0 or 1;
each of R¹ and R² is independently selected from the group consisting of halogen, cyano, C₁₋₆-alk(en/yn)yl, C₃₋₈-cycloalk(en)yl, C₃₋₈-cycloalk(en)yl-C₁₋₆-alk(en/yn)yl, halo-C₁₋₆-alk(en/yn)yl, halo-C₃-₈-cycloalk(en)yl, halo-C₃₋₈-cycloalk(en)yl-C₁₋₆-alk(en/yn)yl, C₁₋₆-alk(en/yn)yloxy, C₃₋₈-cycloalk(en)yloxy and C₃₋₈-cycloalk(en)yl-C₁₋₆-alk(en/yn)yloxy; and
R³ is selected from the group consisting of C₁₋₈-alk(en/yn)yl, C₃₋₈-cycloalk(en)yl, C₃₋₈-cycloalk(en)yl-C₁₋₆-alk(en/yn)yl, optionally substituted Aryl-C₁₋₆-alk(en/yn)yl, optionally substituted Aryl-C₃₋₈-cycloalk(en)yl, optionally substituted Aryl-C₃₋₈-cycloalk(en)yl-C₁₋₆-alk(en/yn)yl, C₁₋₆-alk(en/yn)yl-C₃₋₈-heterocycloalk(en)yl-C₁₋₆-alk(en/yn)yl, C₃₋₈-heterocycloalk(en)yl-C₁₋₆-alk(en/yn)yl, C₁₋₆-alk(en/yn)yl-C₃₋₈-heterocycloalk(en)yl-C₁₋₆-alk(en/yn)yl, Heteroaryl-C₁₋₆-alk(en/yn)yl, Heteroaryl-C₃₋₈-cycloalk(en)yl, Heteroaryl-C₃₋₈-cycloalk(en)yl-C₁₋₆-alk(en/yn)yl, NR⁴R⁵-C₁₋₆-alk(en/yn)yl, NR⁴R⁵-C₃₋₈-cycloalk(en)yl, NR⁴R⁵-C₃₋₈-cycloalk(en)yl-C₁₋₆-alk(en/yn)yl, C₁₋₆-alk(en/yn)yloxy-C₁₋₆-alk(en/yn)yl, C₃₋₈-cycloalk(en)yloxy-C₁₋₆-alk(en/yn)yl, C₃-₈-cycloalk(en)yl-C₁₋₆-alk(en/yn)yloxy-C₁₋₆-alk(en/yn)yl, halo-C₁₋₆-alk(en/yn)yl, halo-C₃₋₈-cycloalk(en)yl and halo-C₃₋₈-cycloalk(en)yl-C₁₋₆-alk(en/yn)yl; wherein
each of R⁴ and R⁵ is independently selected from the group consisting of hydrogen, C₁₋₆-alk(en/yn)yl, C₃₋₈-cycloalk(en)yl and C₃₋₈-cycloalk(en)yl-C₁₋₆-alk(en/yn)yl;
or pharmaceutically acceptable salts thereof; and
where formula 8 is: 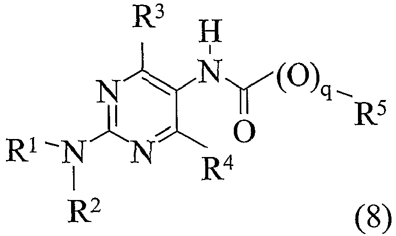 wherein: q is 0 or 1;
R¹ and R² are independently selected from the group consisting of hydrogen and optionally substituted aryl-C₁₋₆-alk(en/yn)yl, provided that R¹ and R² are not both hydrogen, or R¹ and R² together with the nitrogen to which they are attached form a 5 to 7 membered ring optionally containing a further heteroatom;
R³ and R⁴ are independently selected from hydrogen, halogen, cyano, amino, C₁₋₆-alk(en/yn)yl, C₃₋₈-cycloalk(en)yl, halo-C₁₋₆-alk(en/yn)yl, halo-C₃₋₈-cycloalk(en)yl, C₁₋₆-alk(en/yn)yloxy, C₃₋₈-cycloalk(en)yloxy, C₃₋₈-cycloalk(en)yl-C₁₋₆-alk(en/yn)yloxy, halo-C₁₋₆-alk(en/yn)yloxy, halo-C₃₋₈-cycloalk(en)yloxy and halo-C₃₋₈-cycloalk(en)yl-C₁₋₆-alk(en/yn)yloxy, provided that R³ and R⁴ are not both hydrogen;
R⁵ is selected from the group consisting of C₁₋₁₀-alk(en/yn)yl, C₃₋₈-cycloalk(en)yl-C₁₋₆-alk(en/yn)yl, optionally substituted aryl-C₁₋₆-alk(en/yn)yl and optionally substituted aryl;
or pharmaceutically acceptable salts thereof; and
where formula 9 is: 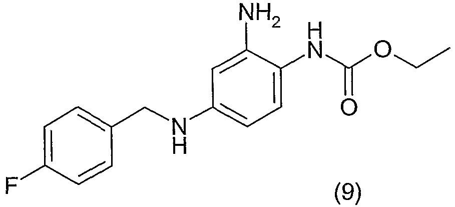 or a pharmaceutically acceptable salt thereof.

In a seventh aspect the present invention, relates to the use of a selective KCNQ potassium channel opener for the preparation of a pharmaceutical composition for the treatment of schizophrenia.

In an eight aspect the present invention, relates to a method of screening for a compound, which is a selective KCNQ channel opener and which is capable of having an anti-psychotic potential comprising the steps of:
a. screening for a KCNQ opener;
b. contra-screening against other channels and/or receptors, and
c. testing the compound in a model predictive for an anti-psychotic potential.

In a ninth aspect the present invention relates to a method for treating or reducing the symptoms of depression, bipolar disorders, bipolar depression or major depression, said method comprising administering to a host in need thereof an effective amount of a compound able to selectively increase the ion flow through KCNQ potassium channels.

In a tenth aspect the present invention relates to a method for treating or reducing the symptoms of schizophrenia, said method comprising administering to a host in need thereof an effective amount of a compound able to selectively increase the ion flow through KCNQ potassium channels and one or more antipsychotic compounds, for example Asenapine, Blonanserin, Iloperidone, Paliperidone, Bifeprunox, Lurasidone, Ocaperidone, Talnetant, ACP 104, SLV 310, ACR 16, YKP 1358, GW 773812, RGH 188, SLV 314, Y-931, BL 1020, Chlorpromazine, Levomepromazine, Promazine, Acepromazine, Triflupromazine, Cyamemazine, Chlorproethazine, Dixyrazine, Fluphenazine, Perphenazine, Prochlorperazine, Thiopropazate, Trifluoperazine, Acetophenazine, Thioproperazine, Butaperazine, Perazine, Periciazine, Thioridazine, Mesoridazine, Pipotiazine, Haloperidol, Trifluperidol, Melperone, Moperone, Pipamperone, Bromperidol, Benperidol, Droperidol, Fluanisone, Oxypertine, Molindone, Sertindole, Ziprasidone, Flupentixol, Clopenthixol, Chlorprothixene, Tiotixene, Zuclopenthixol, Fluspirilene, Pimozide, Penfluridol, Loxapine, Clozapine, Olanzapine, Quetiapine, Sulpiride, Sultopride, Tiapride, Remoxipride, Amisulpride, Veralipride, Levosulpiride, Prothipendyl, Risperidone, Clotiapine, Mosapramine, Zotepine or Aripiprazole.

### Description of the invention

The pharmacological profile of the compounds of the invention is highly novel compared with existing antipsychotic compounds and would therefore be expected to be devoid of the side effects induced by these drugs. In addition compounds that activate KCNQ channels may have a fast onset of action. Furthermore, the distinct and novel mechanism of action may have significantly greater efficacy in treating the positive, the negative and the cognitive symptoms of schizophrenia and may treat a greater percentage of patients than currently benefit from existing antipsychotic drugs. Additionally compliance may be improved. In addition, compounds that activate KCNQ channels may show improved utility in treating depression or bipolar disorder. They would, therefore, offer a significant advance in the treatment of schizophrenia, depression, bipolar disorder and related diseases and disorders.

In one embodiment, the invention relates to a method wherein positive symptoms of schizophrenia are reduced, wherein said positive symptoms cover a pattern of psychotic features including one or more of, but not limited to, hallucinations (typically auditory), delusions, thought disorders, distortions or exaggerations in language and communication, disorganized speech, disorganized behaviour, catatonic behaviour and agitation.

In another embodiment, the invention relates to a method wherein negative symptoms of schizophrenia are reduced, wherein said negative symptoms typically refer to a syndrome characterised by one or more of, but not limited to, blunted affect, aphasia, asociality, anhedonia (lack of pleasure), avolition (restrictions in the initiation of goal-directed behaviour), emotional withdrawal, difficulty in abstract thinking, lack of spontaneity, stereotyped thinking, alogia (restrictions in the fluency and productivity of thought and speech) and attentional impairment.

In yet another embodiment, the invention relates to a method wherein cognitive symptoms of schizophrenia are reduced, wherein said cognitive symptoms refer to, but limited to, dysfunction across many cognition domains including attention, memory and executive function.

In yet another embodiment, the invention relates to a method wherein one or more of positive, negative and cognitive symptoms of schizophrenia are reduced.

In yet another embodiment, the invention relates to a method wherein the symptoms of one or more of the schizophrenia subtypes selected from the group consisting of the catatonic-subtype, the paranoid-subtype, the disorganized-subtype and the residual-subtype are reduced.

In yet another embodiment, the invention relates to a method wherein said compound able to selectively increase the ion flow through KCNQ potassium channels is effective in a model predictive for an anti-psychotic potential of said compound.

In yet another embodiment, the invention relates to a method wherein said model is selected from the group consisting of the acute stimulant-induced hyperactivity test, the sensitised amphetamine-induced hyperactivity test, the conditioned avoidance test, the spontaneous firing of mesolimbic DA cells test and the mouse forced swim test. In yet another embodiment, the invention relates to a method wherein said compound is effective in more than one model predictive for an anti-psychotic potential of said compound.

In yet another embodiment, the invention relates to a method wherein said compound does not to any reasonably extent manifest any side-effects associated with the mechanism of action of compounds known to treat schizophrenia.

In yet another embodiment, the invention relates to a method wherein said side effects associated with compounds known to treat schizophrenia is mediated directly through dopamine D2 receptor modulation.

In yet another embodiment, the invention relates to a method wherein said compound is administered in an amount of more than 1 mg/day.

In yet another embodiment, the invention relates to a method wherein said compound is administered in an amount of more than 5 mg/day, more than 10 mg/day or more than 50 mg/day.

In yet another embodiment, the invention relates to a method wherein said amount is administered once daily or more than once daily.

In yet another embodiment, the invention relates to a method wherein said compound has a fast-onset of action.

In yet another embodiment, the invention relates to a method wherein the symptoms of schizophrenia are reduced faster than known compounds for treating said symptoms of schizophrenia.

In yet another embodiment, the invention relates to a method wherein the said symptoms of schizophrenia are reduced after two weeks, preferably after one week, even more preferred within one week, even more preferred after two days, even more preferred within two days and most preferably after a day.

In yet another embodiment, the invention relates to acute treatment.

In yet another embodiment, the invention relates to long-term treatment.

In yet another embodiment, the invention relates to a method wherein said compound is a compound according to formula 1, 2, 3, 4, 5, 6, 7, 8 or 9,
where formula 1 is: 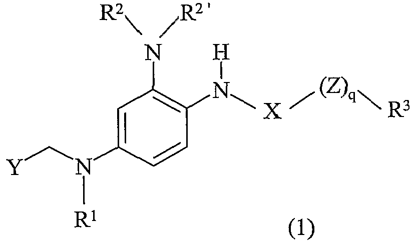 wherein
**R¹** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, acyl, hydroxy-C₁₋₆-alk(en/yn)yl and hydroxy-C₃₋₈-cycloalk(en)yl;
**R²** and **R^{2'}** are independently selected from the group consisting of hydrogen, C₁-6-alk(en/yn)yl, C₃₋₈-cycloalk(en)yl, aryl, C₃₋₈-cycloalk(en)yl-C₁₋₆-alk(en/yn)yl, aryl-C₁₋₆-alk(en/yn)yl, acyl, hydroxy-C₁₋₆-alk(en/yn)yl and hydroxy-C₃₋₈-cycloalk(en)yl;
**R³** is selected from the group consisting of hydrogen, C₁₋₆-alk(en/yn)yl, C₃₋₈-cycloalk(en)yl, aryl, C₃₋₈-cycloallc(en)yl-C₁₋₆-alk(en/yn)yl, aryl-C₁₋₆-alk(en/yn)yl, hydroxy-C₁₋₆-alk(en/yn)yl, aryl-C₃₋₈-cycloalk(en)yl, NR¹⁰R^{10'}-C₁₋₆-alk(en/yn)yl, NR¹⁰R^{10'}-C₃₋₈-cycloalk(en)yl and hydroxy-C₃₋₈-cycloalk(en)yl; wherein
**R¹⁰** and **R^{10'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, hydroxy-C₁₋₆-alk(en/yn)yl, hydroxy-C₃₋₈-cycloalk(en)yl, hydroxy-C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, cyano-C₁₋₆-alk(en/yn)yl, cyano-C₃₋₈-cycloalk(en)yl and cyano-C₃-₈-cycloalk(en)yl-C₁₋₆-alk(en/yn)yl, or **R¹⁰** and **R¹⁰'** together with the nitrogen atom to which they are attached form a 4-8 membered saturated or unsaturated ring which optionally contains 1, 2 or 3 further heteroatoms;
**X** is CO or SO₂;
**Z** is O or NR⁴, wherein
**R⁴** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, hydroxy-C₁₋₆-alk(en/yn)yl and hydroxy-C₃₋₈-cycloalk(en)yl; or
**R³** and **R⁴** together with the nitrogen atom to which they are attached form a 4-8 membered saturated or unsaturated ring which optionally contains 1, 2 or 3 further heteroatoms, the ring formed by **R³** and **R⁴** and the nitrogen atom is optionally substituted with one or more substituents independently selected from C₁₋₆-alk(en/yn)yl, aryl and aryl-C₁₋₆-alk(en/yn)yl;
**q** is 0 or 1;
and
**Y** represents a heteroaryl of formula II or III 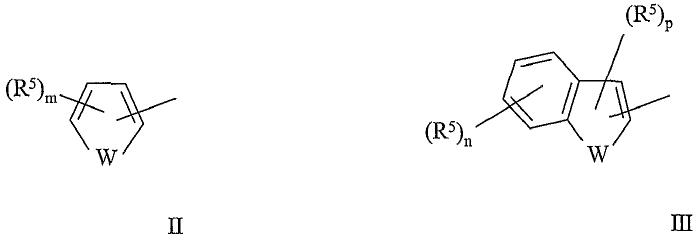 wherein
**W** is O or S;
**m** is 0,1, 2 or 3;
**n** is 0, 1, 2, 3 or 4;
**p** is 0 or 1; and
each **R**⁵ is independently selected from the group consisting of C₁₋₆-alk(en/yn)yl, C₃₋₈-cycloalk(en)yl, aryl, C₃₋₈-cycloalk(en)yl-C₁₋₆-alk(en/yn)yl, aryl-C₁₋₆-alk(en/yn)yl, acyl, halogen, halo-C₁₋₆-alk(en/yn)yl, C₁₋₆-alk(en/yn)yloxy, -CO-NR⁶R^{6'}, cyano, nitro, -NR⁷R^{7'}, -S-R⁸, -SO₂R⁸, SO₂OR⁸;
wherein
**R⁶** and **R^{6'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl and aryl;
**R⁷** and **R^{7'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, aryl and acyl; and
**R⁸** is selected from the group consisting of C₁₋₆-alk(en/yn)yl, C₃₋₈-cycloalk(en)yl, C₃₋₈-cycloalk(en)yl-C₁₋₆-alk(en/yn)yl, aryl and -NR⁹R^{9'}; wherein
**R⁹** and **R^{9'}** are independently selected from the group consisting of hydrogen, C₁₋₆ -alk(en/yn)yl, C₃₋₈-cycloalk(en)yl and C₃₋₈-cycloalk(en)yl-C₁₋₆-alk(en/yn)yl;
or pharmaceutically acceptable salts thereof; and
where fonnula 2 is: 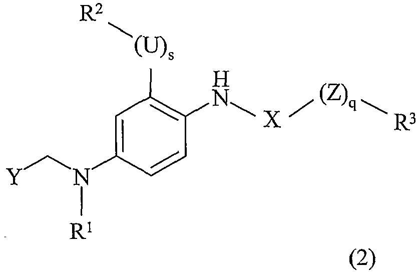 wherein
s is 0 or 1;
**U** is O, S, SO₂ SO₂N**R¹¹**, CO-O or CON**R¹¹**_{;} wherein
**R¹¹** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl; or **R²** and **R¹¹** together with the nitrogen atom form a 5-8 membered saturated or unsaturated ring which optionally contains 1, 2 or 3 further heteroatoms;
**q** is 0 or 1;
**X** is CO or SO₂; with the proviso that q is 0 when **X** is SO₂;
**Z** is O or S;
**R¹** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, acyl, hydroxy-C₁-₆-alk(en/yn)yl, hydroxy-C₃₋₈-cycloalk(en)yl, hydroxy-C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, cyano-C₁₋₆-alk(en/yn)yl, cyano-C₃₋₈-cycloalk(en)yl and cyano-C₃₋₈-cycloallc(en)yl-C₁₋₆-alk(en/yn)yl;
**R²** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar, Ar-C₁₋₆-alk(en/yn)yl, Ar-C₃₋₈-cycloalk(en)yl, Ar-C₃₋₈-cycloalk(en)yl-C₁₋₆-alk(en/yn)yl, acyl, hydroxy-C₁₋₆-alk(en/yn)yl, hydroxy-C₃₋₈-cycloalk(en)yl, hydroxy-C₃₋₈-cycloalk(en)yl-C₁₋₆-alk(en/yn)yl, halogen, halo-C₁₋₆-alk(en/yn)yl, halo-C₃₋₈-cycloalk(en)yl, halo-C₃₋₈-cycloalk(en)yl-C₁₋₆-alk(en/yn)yl, cyano, cyano-C₁₋₆-alk(en/yn)yl, cyano-C₃₋₈-cycloalk(en)yl, cyano-C₃₋₈-cycloalk(en)yl-C₁₋₆-alk(en/yn)yl, N**R¹⁰R**^{**10**'}-C₁₋₆-alk(en/yn)yl, N**R¹⁰R**^{**10**'}-C₃₋₈-cycloalk(en)yl and N**R¹⁰R**^{**10**'}-C₃₋₈-cycloalk(en)yl-C₁₋₆ -alk(en/yn)yl; wherein
**R¹⁰** and **R^{10'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, hydroxy-C₁₋₆-alk(en/yn)yl, hydroxy-C₃₋₈-cycloalk(en)yl, hydroxy-C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, cyano-C₁₋₆-alk(en/yn)yl, cyano-C₃₋₈-cycloalk(en)yl and cyano-C₃₋₈-cycloalk(en)yl-C₁₋₆-alk(en/yn)yl, or **R¹⁰** and **R**^{**10**'} together with the nitrogen atom form a 5-8 membered saturated or unsaturated ring which optionally contains 1, 2 or 3 further heteroatoms; provided that when **R²** is halogen or cyano then s is 0; and provided that U is O or S when s is 1 and **R²** is a hydrogen atom or acyl;
**R³** is selected from the group consisting of C₁₋₆-alk(en/yn)yl, C₃₋₈-cycloalk(en)yl, heterocycloalk(en)yl, C₃₋₈-cycloalk(en)yl-C₁₋₆-alk(en/yn)yl, C₁₋₆-alk(en/yn)yl-C₃-8-cycloalk(en)yl, C₁₋₆-alk(en/yn)yl-heterocycloalk(en)yl, heterocycloalk(en)yl-C₁₋₆-alk(en/yn)yl, Ar, Ar-C₁₋₆-alk(en/yn)yl, Ar-C₃₋₈-cycloalk(en)yl, Ar-heterocycloalk(en)yl, Ar-C₃₋₈-cycloalk(en)yl-C₁₋₆-alk(en/yn)yl, Ar-C₁₋₆-alk(en/yn)yl-C₃₋₈-cycloalk(en)yl, Ar-C₁₋₆-alk(en/yn)yl-heterocycloalk(en)yl, C₁₋₆-alk(en/yn)yloxy-C₁₋₆-alk(en/yn)yl, C₃₋₈-cycloalk(en)yloxy-C₁₋₆-alk(en/yn)yl, C₁₋₆-alk(en/yn)yloxy-C₃₋₈-cycloalk(en)yl, C₁₋₆-alk(en/yn)yloxy-heterocycloalk(en)yl, Ar-oxy-C₁₋₆-alk(en/yn)yl, Ar-C₁₋₆-alk(en/yn)yloxy-C₁₋₆-alk(en/yn)yl, C₁₋₆-alk(en/yn)yloxy-carbonyl-C₁₋₆-alk(en/yn)yl, C₃₋₈-cycloalk(en)yloxy-carbonyl-C₁₋₆-alk(en/yn)yl, C₃₋₈-cycloalk(en)yl-C₁₋₆-alk(en/yn)yloxy-carbonyl-C₁₋₆-alk(en/yn)yl, hydroxy-C₁₋₆-alk(en/yn)yl, hydroxy-C₃₋₈-cycloalk(en)yl, hydroxy-heterocycloalk(en)yl, hydroxy-C₃₋₈-cycloalk(en)yl-C₁₋₆-alk(en/yn)yl, hydroxy-C_{1- 6}-alk(en/yn)yl-C₃₋₈-cycloalk(en)yl, hydroxy-C₁₋₆-alk(en/yn)yl-heterocycloalk(en)yl, halo-C₁₋₆-alk(en/yn)yl, halo-C₃₋₈-cycloalk(en)yl, halo-heterocycloalk(en)yl, halo-C₃₋₈-cycloalk(en)yl-C₁₋₆-alk(en/yn)yl, halo-C₁₋₆-alk(en/yn)yl-C₃₋₈-cycloalk(en)yl, halo-C₁₋₆-alk(en/yn)yl-heterocycloalk(en)yl, halo-C₁₋₆-alk(en/yn)yl-Ar, halo-C₃₋₈-cycloalk(en)yl-Ar, halo-C₃₋₈-cycloalk(en)yl-C₁₋₆-alk(en/yn)yl-Ar, halo-C₁₋₆-alk(en/yn)yl-C₃₋₈-cycloalk(en)yl-Ar, cyano-C₁₋₆-alk(en/yn)yl, cyano-C₃₋₈-cycloalk(en)yl, cyano-heterocycloalk(en)yl, cyano-C₃₋₈-cycloalk(en)yl-C₁₋₆-alk(en/yn)yl, cyano-C₁₋₆-alk(en/yn)yl-C₃₋₈-cycloalk(en)yl, cyano-C₁₋₆-alk(en/yn)yl-heterocycloalk(en)yl, acyl-C₁₋₆-alk(en/yn)yl, acyl-C₃₋₈-cycloalk(en)yl, acyl-heterocycloalk(en)yl, acyl-C₃₋₈-cycloalk(en)yl-C₁₋₆-alk(en/yn)yl, acyl-C₁₋₆-alk(en/yn)yl-C₃₋₈-cycloalk(en)yl, acyl-C₁₋₆-alk(en/yn)yl-heterocycloalk(en)yl, N**R¹²R^{12'}**, optionally substituted N**R¹²R**^{**12**'}-C₁₋₆-alk(en/yn)yl, optionally substituted N**R¹²R**^{**12**'}-C₃₋₈-cycloalk(en)yl, optionally substituted N**R¹²R**^{**12**'}-C₃₋₈-cycloalk(en)yl-C₁₋₆-alk(en/yn)yl; wherein **R¹²** and **R**^{**12**'} are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar, Ar-C₁₋₆-alk(en/yn)yl, Ar-C₃₋₈-cycloalk(en)yl, Ar-C₃₋₈-cycloalk(en)yl-C₁₋₆-alk(en/yn)yl, Ar-heterocycloalk(en)yl, Ar-oxy-C₁₋₆-alk(en/yn)yl, Ar-oxy-C₃₋₈-cycloalk(en)yl, Ar-oxy-C₃₋₈-cycloalk(en)yl-C₁₋₆-alk(en/yn)yl, Ar-oxy-heterocycloalk(en)yl, hydroxy-C₁₋₆-alk(en/yn)yl, hydroxy-C₃₋₈-cycloalk(en)yl, hydroxy-C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, cyano-C₁₋₆-alk(en/yn)yl, cyano-C₃₋₈-cycloalk(en)yl and cyano-C₃₋₈-cycloalk(en)yl-C₁₋₆-alk(en/yn)yl, or
**R¹²** and **R^{12'}** together with the nitrogen atom form a 5-8 membered saturated or unsaturated ring which optionally contains 1, 2 or 3 further heteroatoms;
with the proviso that when **R³** is N**R¹²R^{12'}** then q is 0;
and
Y represents a group of formula **XXIV, XXV, XXVI, XXVII, XXVIII, XXXXI** or **XXXXII:** 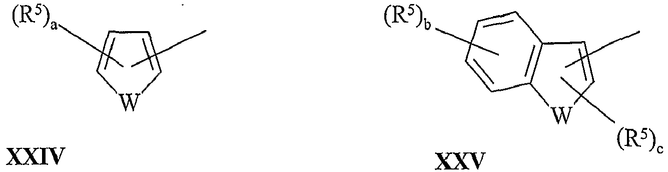 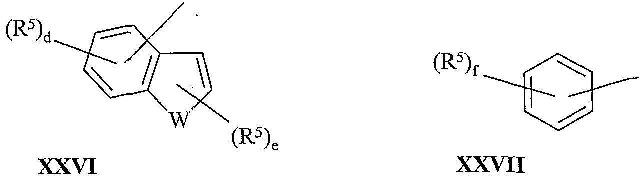 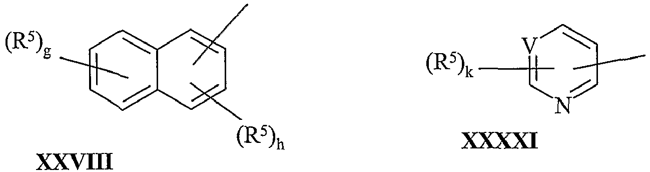 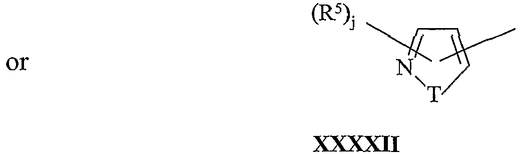 wherein
the line represents a bond attaching the group represented by Y to the carbon atom;
**W** is O or S;
**V** is N, C or CH;
**T** is N, NH or O;
**a** is 0, 1, 2 or 3;
**b** is 0, 1, 2, 3 or 4;
**c** is 0 or 1;
**d** is 0, 1, 2 or 3;
**e** is 0, 1 or 2;
**f** is 0, 1, 2, 3, 4 or 5;
**g** is 0, 1, 2, 3 or 4;
**h** is 0, 1, 2 or 3;
j is 0, 1 or 2;
**k** is 0, 1, 2 or 3; and
each **R⁵** is independently selected from the group consisting of a C₁₋₆-alk(en/yn)yl, C₃₋₈-cycloalk(en)yl, C₃₋₈-cycloalk(en)yl-C₁₋₆-alk(en/yn)yl, Ar, ArC₁₋₆-alk(en/yn)yl, Ar-C₃₋₈-cycloalk(en)yl, Ar-C₃₋₈-cycloalk(en)yl-C₁₋₆-alk(en/yn)yl, Ar-oxy, Ar-oxy-C₁₋₆-alk(en/yn)yl, Ar-oxy-C₃₋₈-cycloalk(en)yl, C₁₋₆-alk(en/yn)yl-heterocycloalk(en)yl, Ar-oxy-C₃₋₈-cycloalk(en)yl-C₁₋₆-alk(en/yn)yl, acyl, C₁₋₆-alk(en/yn)yloxy, C₃₋₈-cycloalk(en)yloxy, C₃₋₈-cycloalk(en)yl-C₁₋₆-alk(en/yn)yloxy, C₁₋₆-alk(en/yn)yloxy-carbonyl, halogen, halo-C₁₋₆-alk(en/yn)yl, halo-C₃₋₈-cycloalk(en)yl, halo-C₃₋₈-cycloalk(en)yl-C₁₋₆-alk(en/yn)yl, -CO-NR⁶R^{6'}, cyano, cyano-C₁₋₆-alk(en/yn)yl, cyano-C₃₋₈-cycloalk(en)yl, cyano-C₃₋₈-cycloalk(en)yl-C₁₋₆-alk(en/yn)yl, N**R⁷R^{7'}**, S-**R⁸** and SO₂**R⁸**, or two adjacent **R⁵** together with the aromatic group form a 5-8 membered ring which optionally contains one or two heteroatoms;
**R⁶** and **R^{6'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl and Ar;
**R⁷** and **R^{7'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar, heterocycloalk(en)yl-C₁₋₆-alk(en/yn)yl, heterocycloalk(en)yl-C₃₋₈-cycloalk(en)yl, heterocycloalk(en)yl-C₃₋₈-cycloalk(en)yl-C₁₋₆-alk(en/yn)yl, heterocycloalk(en)yl-Ar and acyl; or
**R⁷** and **R^{7'}** together with the nitrogen atom form a 5-8 membered saturated or unsaturated ring which optionally contains 1, 2 or 3 further heteroatoms; and
**R⁸** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar and -N**R⁹R**^{**9**'}; wherein **R⁹** and **R^{9'}** are independently selected from the group consisting of hydrogen, C₁₋₆-alk(en/yn)yl, C₃₋₈-cycloalk(en)yl and C₃₋₈-cycloalk(en)yl-C₁₋₆-alk(en/yn)yl;
or pharmaceutically acceptable salts thereof; and
where formula 3 is: 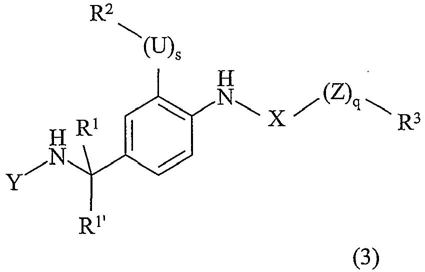 wherein
**U** is O, S or N**R**^{**2**'}_{;}
**s** is 0 or 1;
**X** is CO or SO₂;
Z is O, S or N**R⁴**, wherein **R⁴** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, hydroxy-C₁₋₆-alk(en/yn)yl and hydroxy-C₃₋₈-cycloalk(en)yl;
**q** is 0 or 1;
**R¹** and **R**^{**1**'} are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, acyl, hydroxy-C₁₋₆-alk(en/yn)yl, hydroxy-C₃₋₈-cycloalk(en)yl, halo-C₁₋₆-alk(en/yn)yl and halo-C₃₋₈-cycloalk(en)yl;
**R²** is selected from the group consisting of hydrogen, halogen, C₁₋₆-alk(en/yn)yl, C₃₋₈-cycloalk(en)yl, C₃₋₈-cycloalk(en)yl-C₁₋₆-alk(en/yn)yl, Ar, Ar-C₁₋₆-alk(enyn)yl, Ar-C₃₋₈-cycloalk(en)yl, acyl, hydroxy-C₁₋₆-alk(en/yn)yl, hydroxy-C₃₋₈-cycloalk(en)yl, halo-C₁₋₆-alk(en/yn)yl, halo-C₃₋₈-cycloalk(en)yl and cyano; provided that when **R²** is halogen or cyano, then s is 0;
when s is 1 and U is N**R^{2'}** then **R**^{**2**'} is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar, Ar-C₁₋₆-alk(en/yn)yl, Ar-C₃₋₈-cycloalk(en)yl, acyl, hydroxy-C₁₋₆-alk(en/yn)yl, hydroxy-C₃₋₈-cycloalk(en)yl, halo-C₁₋₆-alk(en/yn)yl and halo-C₃₋₈-cycloalk(en)yl; or **R²** and **R**^{**2**'} together form a 5-8 membered saturated or unsaturated ring which optionally contains one further heteroatom;
**R³** is selected from the group consisting of C₁₋₆-alk(en/yn)yl, C₃₋₈-cycloalk(en)yl, C₃₋₈-cycloalk(en)yl-C₁₋₆-alk(en/yn)yl, Ar, Ar-C₁₋₆-alk(en/yn)yl, Ar-C₃₋₈-cycloalk(en)yl, hydroxy-C₁₋₆-alk(en/yn)yl, hydroxy-C₃₋₈-cycloalk(en)yl, halo-C₁₋₆-alk(en/yn)yl and halo-C₃₋₈-cycloalk(en)yl;
and
**Y** represents a group of fonnulae VI, **VII, VIII, IX** or **XXX:** 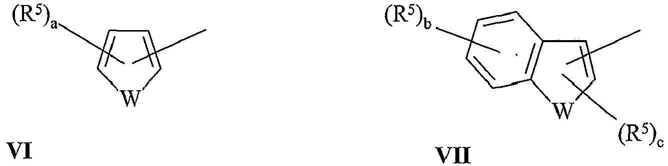 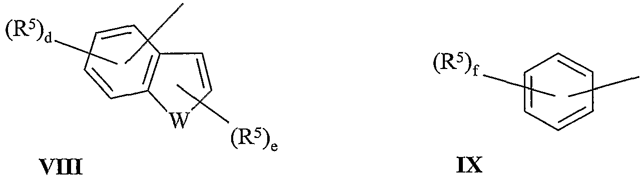 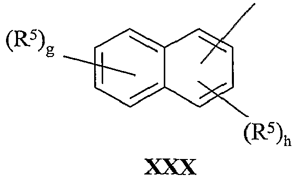 wherein
the line represents a bond attaching the group represented by Y to the nitrogen atom;
**W** is O or S;
**a** is 0, 1, 2 or 3;
**b** is 0, 1, 2, 3 or 4;
**c** is 0 or 1;
**d** is 0, 1, 2 or 3;
**e** is 0, 1 or 2;
**f** is 0, 1, 2, 3, 4 or 5;
**g** is 0, 1, 2, 3 or 4;
**h** is 0, 1, 2 or 3; and
each **R⁵** is independently selected from the group consisting of a C₁₋₆-alk(en/yn)yl, C₃₋₈-cycloalk(en)yl, Ar, C₃₋₈-cycloalk(en)yl-C₁₋₆-alk(en/yn)yl, ArC₁₋₆-alk(en/yn)yl, acyl, C₁₋₆-alk(en/yn)yloxy, halogen, halo-C₁₋₆-alk(en/yn)yl, -CO-**NR⁶R**^{**6**'}, cyano, nitro, -N**R⁷R^{7'}**, -S-**R⁸**, -SO₂**R⁸** and SO₂O**R⁸**, or two substituents together form a 5-8 membered saturated or unsaturated ring which optionally contains one or two heteroatoms;
**R⁶** and **R^{6'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl and Ar;
**R⁷** and **R^{7'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar and acyl; and
**R⁸** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar and -NR⁹R^{9'}; wherein **R⁹** and **R^{9'}** are independently selected from the group consisting of hydrogen, C₁₋₆-alk(en/yn)yl, C₃₋₈-cycloalk(en)yl and C₃₋₈-cycloalk(en)yl-C₁₋₆-alk(en/yn)yl; with the provisos that when **R⁵** is SO₂O**R⁸** then **R⁸** is not -N**R⁹R**^{**9**'} and when **R⁵** is SO₂**R⁸**, then **R⁸** is not a hydrogen atom;
or pharmaceutically acceptable salts thereof; and
where formula 4 is: 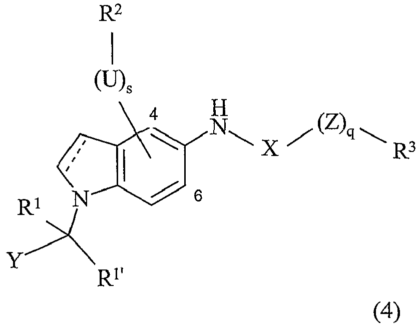 wherein
the dotted line represents an optional bond;
**R¹** and **R^{1'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, hydroxy-C₁₋₆-alk(en/yn)yl, hydroxy-C₃₋₈-cycloalk(en)yl, hydroxy-C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, cyano-C₁₋₆-alk(en/yn)yl, cyano-C₃₋₈-cycloalk(en)yl and cyano-C₃₋₈-cycloalk(en)yl-C₁₋₆-alk(en/yn)yl; or **R¹** and **R^{1'}** together with the carbon atom to which they are attached form a 3-8 membered saturated or unsaturated ring which optionally contains 1 or 2 heteroatoms;
**s** is 0 or 1;
U is O, N**R¹¹**, S, SO₂, SO₂N**R¹¹**, CO-O or CO-N**R¹¹**; wherein **R¹¹** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl; or **R²** and **R¹¹** together with the nitrogen atom to which they are attached form a 4-8 membered saturated or unsaturated ring which optionally contains 1, 2 or 3 further heteroatoms;
**R²** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar, Ar-C₁₋₆-alk(en/yn)yl, Ar-C₃₋₈-cycloalk(en)yl, Ar-C₃₋₈-cycloalk(en)yl-C₁₋₆-alk(en/yn)yl, acyl, hydroxy-C₁₋₆-alk(en/yn)yl, hydroxy-C₃₋₈-cycloalk(en)yl, hydroxy-C₃₋₈-cycloalk(en)yl-C₁₋₆-alk(en/yn)yl, halogen, halo-C₁₋₆-alk(en/yn)yl, halo-C₃₋₈-cycloalk(en)yl, halo-C₃₋₈-cycloalk(en)yl-C₁₋₆-alk(en/yn)yl, cyano, cyano-C₁₋₆-alk(en/yn)yl, cyano-C₃₋₈-cycloalk(en)yl, cyano-C₃₋₈-cycloalk(en)yl-C₁₋₆-alk(en/yn)yl, -NO₂, N**R¹⁰R**^{**10**'}-C₁₋₆-alk(en/yn)yl, N**R¹⁰R¹⁰'**-C₃₋₈-cycloalk(en)yl and N**R¹⁰R**^{**10**'}-C₃₋₈-cycloalk(en)yl-C₁₋₆-alk(en/yn)yl; wherein
**R¹⁰** and **R**^{**10**'} are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, hydroxy-C₁₋₆-alk(en/yn)yl, hydroxy-C₃₋₈-cycloalk(en)yl, hydroxy-C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, cyano-C₁₋₆-alk(en/yn)yl, cyano-C₃₋₈-cycloalk(en)yl and cyano-C₃₋₈-cycloalk(en)yl-C₁₋₆-alk(en/yn)yl, or **R¹⁰** and **R**^{**10**'} together with the nitrogen atom to which they are attached form a 4-8 membered saturated or unsaturated ring which optionally contains 1, 2 or 3 further heteroatoms;
with the proviso that when **R²** is NO₂, halogen or cyano then s is 0; and with the proviso that when **R²** is a hydrogen atom or acyl and s is 1 then U is N**R¹¹** O or S;
wherein the group -(U)ₛ-**R²** is linked to position 4 or 6 of the indole or indoline;
**q** is 0 or 1;
**Z** is O or S;
**X** is CO or SO₂; with the proviso that q is 0 when **X** is SO₂;
**R³** is selected from the group consisting of C₁₋₆-alk(en/yn)yl, C₃₋₈-cycloalk(en)yl, heterocycloalk(en)yl, C₃₋₈-cycloalk(en)yl-C₁₋₆-alk(en/yn)yl, C₁₋₆-alk(en/yn)yl-C₃₋₈-cycloalk(en)yl, C₁₋₆-alk(en/yn)yl-heterocycloalk(en)yl, Ar, Ar-C₁₋₆-alk(en/yn)yl, Ar-C₃₋₈-cycloalk(en)yl, Ar-heterocycloalk(en)yl, Ar-C₃₋₈-cycloalk(en)yl-C₁₋₆-alk(en/yn)yl, Ar-C₁₋₆-alk(en/yn)yl-C₃₋₈-cycloalk(en)yl, Ar-C₁₋₆-alk(en/yn)yl-heterocycloalk(en)yl, C₁₋₆-alk(en/yn)yloxy-C₁₋₆-alk(en/yn)yl, C₃₋₈-cycloalk(en)yloxy-C₁₋₆-alk(en/yn)yl, C₁₋₆-alk(en/yn)yloxy-C₃₋₈-cycloalk(en)yl, C₁₋₆-alk(en/yn)yloxy-heterocycloalk(en)yl, Ar-oxy-C₁₋₆-alk(en/yn)yl, Ar-C₁₋₆-alk(en/yn)yloxy-C₁₋₆-alk(en/yn)yl, C₁₋₆-alk(en/yn)yloxy-carbonyl-C₁₋₆-alk(en/yn)yl, C₃₋₈-cycloalk(en)yloxy-carbonyl-C₁₋₆-alk(en/yn)yl, C₃₋₈-cycloalk(en)yl-C₁₋₆-alk(en/yn)yloxy-carbonyl-C₁₋₆-alk(en/yn)yl, hydroxy-C₁₋₆-alk(en/yn)yl, hydroxy-C₃₋₈-cycloalk(en)yl, hydroxy-heterocycloalk(en)yl, hydroxy-C₃₋₈-cycloalk(en)yl-C₁₋₆-alk(en/yn)yl, hydroxy-C₁₋₆-alk(en/yn)yl-C₃₋₈-cycloalk(en)yl, hydroxy-C₁₋₆-alk(en/yn)yl-heterocycloalk(en)yl, halo-C₁₋₆-alk(en/yn)yl, halo-C₃₋₈-cycloalk(en)yl, halo-heterocycloalk(en)yl, halo-C₃₋₈-cycloalk(en)yl-C₁₋₆-alk(en/yn)yl, halo-C₁₋₆-alk(en/yn)yl-C₃₋₈-cycloalk(en)yl, halo-C₁₋₆-alk(en/yn)yl-heterocycloalk(en)yl, halo-C₁₋₆-alk(en/yn)yl-Ar, halo-C₃₋₈-cycloalk(en)yl-Ar, halo-C₃₋₈-cycloalk(en)yl-C₁₋₆-alk(en/yn)yl-Ar, halo-C₁₋₆-alk(en/yn)yl-C₃₋₈-cycloalk(en)yl-Ar, cyano-C₁₋₆-alk(en/yn)yl, cyano-C₃₋₈-cycloalk(en)yl, cyano-heterocycloalk(en)yl, cyano-C₃₋₈-cycloalk(en)yl-C₁₋₆-alk(en/yn)yl, cyano-C₁₋₆-alk(en/yn)yl-C₃₋₈-cycloalk(en)yl, cyano-C₁₋₆-alk(en/yn)yl-heterocycloalk(en)yl, acyl-C₁₋₆-alk(en/yn)yl, acyl-C₃₋₈-cycloalk(en)yl, acyl-heterocycloalk(en)yl, acyl-C₃₋₈-cycloalk(en)yl-C₁₋₆-alk(en/yn)yl, acyl-C₁₋₆-alk(en/yn)yl-C₃₋₈-cycloalk(en)yl, acyl-C₁₋₆-alk(en/yn)yl-heterocycloalk(en)yl and -N**R¹²R**^{**12**'}, optionally substituted N**R¹²R**^{**12**'}-C₁₋₆-alk(en/yn)yl, optionally substituted N**R¹²R**^{**12**'}-C₃₋₈-cycloalk(en)yl, optionally substituted N**R¹²R**^{**12**'}-C₃₋₈-cycloalk(en)yl-C₁₋₆-alk(en/yn)yl; wherein **R¹²** and **R**^{**12**'} are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar, Ar-C₁₋₆-alk(en/yn)yl, Ar-C₃₋₈-cycloalk(en)yl, Ar-C₃₋₈-cycloalk(en)yl-C₁₋₆-alk(en/yn)yl, hydroxy-C₁₋₆-alk(en/yn)yl, hydroxy-C₃₋₈-cycloalk(en)yl, hydroxy-C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, cyano-C₁₋₆-alk(en/yn)yl, cyano-C₃₋₈-cycloalk(en)yl and cyano-C₃₋₈-cycloalk(en)yl-C₁₋₆-alk(en/yn)yl, or
**R¹²** and **R^{12'}** together with the nitrogen atom to which they are attached form a 4-8 membered saturated or unsaturated ring which optionally contains 1, 2 or 3 further heteroatoms;
with the proviso that when **R³** is N**R¹²R^{12'}** then q is 0;
and
Y represents a group of formula **II, III, IV,** V, , VI, **XXX** and **XXXI:** 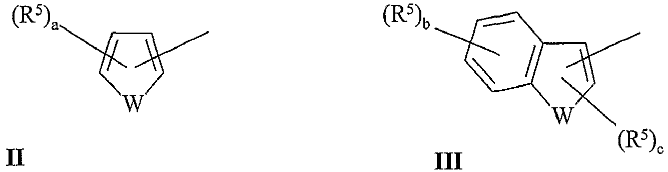 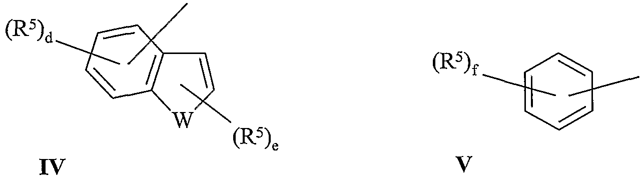 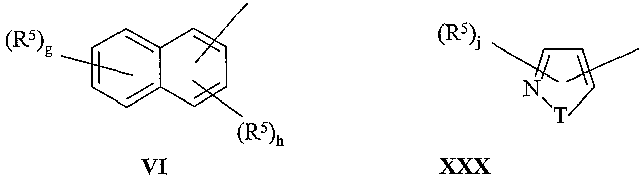 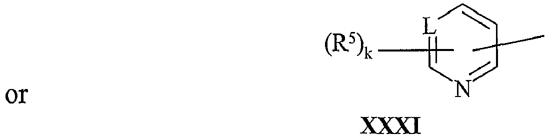 wherein
the line represents a bond attaching the group represented by Y to the carbon atom;
**W** is O or S;
**T** is N, NH or O;
**L** is N, C or CH;
**a** is 0, 1, 2 or 3;
**b** is 0, 1, 2, 3 or 4;
**c** is 0 or 1;
**d** is 0, 1, 2 or 3;
**e** is 0, 1 or 2;
**f** is 0, 1, 2, 3, 4 or 5;
**g** is 0, 1, 2, 3 or 4;
**h** is 0, 1, 2 or 3;
**j** is 0, 1, 2 or 3; with the proviso that when **T** is a nitrogen atom then **j** is 0, 1, 2 or 3; and when **T** is NH or an oxygen atom then **j** is 0, 1 or 2;
**k** is 0, 1, 2, 3 or 4; and
each **R⁵** is independently selected from the group consisting of a C₁₋₆-alk(en/yn)yl, C₃₋₈-cycloalk(en)yl, C₃₋₈-cycloalk(en)yl-C₁₋₆-alk(en/yn)yl, Ar, ArC₁₋₆-alk(en/yn)yl, Ar-thio, Ar-oxy, acyl, C₁₋₆-alk(en/yn)yloxy, C₃₋₈-cycloalk(en)yloxy, C₃₋₈-cycloalk(en)yl-C₁₋₆-alk(en/yn)yloxy, halogen, halo-C₁₋₆-alk(en/yn)yl, halo-C₃₋₈-cycloalk(en)yl, halo-C₃₋₈-cycloalk(en)yl-C₁₋₆-alk(en/yn)yl, -CO-N**R⁶R^{6'}**, cyano, cyano-C₁₋₆-alk(en/yn)yl, cyano-C₃₋₈-cycloalk(en)yl, cyano-C₃₋₈-cycloalk(en)yl-C₁₋₆-alk(en/yn)yl, -N**R⁷R^{7'}**, -S-**R⁸** and -SO₂**R⁸**, or
two adjacent **R⁵** together with the aromatic group to which they are attached form a 4-8 membered ring which optionally contains one or two heteroatoms;
**R⁶** and **R⁶'** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl and Ar;
**R⁷** and **R^{7'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar and acyl;
and
**R⁸** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar and -N**R⁹R^{9'}**; wherein **R⁹** and **R^{9'}** are independently selected from the group consisting of hydrogen, C₁₋₆-alk(en/yn)yl, C₃₋₈-cycloalk(en)yl and C₃₋₈-cycloalk(en)yl-C₁₋₆-alk(en/yn)yl; provided that when **R⁸** is -N**R⁹R^{9'}** then **R⁵** is not -S-**R⁸**;
or pharmaceutically acceptable salts thereof; and
where formula 5 is: 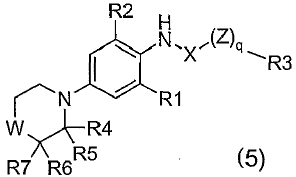 wherein
q is 0 or 1;
WisOorS;
X is CO;
ZisO;
R1 is selected from the group consisting of halogen, cyano, C₁₋₆-alk(en/yn)yl, C₃₋₈-cycloalk(en)yl, C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, C₁₋₆-alk(en/yn)yloxy, C₃₋₈-cycloalk(en)yloxy and C₃₋₈-cycloalk(en)yl-C₁₋₆-alk(en/yn)yloxy;
R2 is selected from the group consisting of halogen, cyano, C₁₋₆-alk(en/yn)yl, C₃₋₈-cycloalk(en)yl, C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, C₁₋₆-alk(en/yn)yloxy, C₃₋₈-cycloalk(en)yloxy, C₃₋₈-cycloalk(en)yl-C₁₋₆-alk(en/yn)yloxy, optionally substituted phenyl and optionally substituted pyridyl; wherein phenyl and pyridyl are optionally substituted with one or more substituents independently being halogen, C₁₋₆-alk(en/yn)yl, C₃₋₈-cycloalk(en)yl or C₃₋₈-cycloalk(en)yl-C₁₋₆-alk(en/yn)yl;
R3 is selected from the group consisting of C₁₋₁₀-alk(en/yn)yl, C₃₋₈-cycloalk(en)yl, C₃₋₈-cycloalk(en)yl-C₁₋₆-alk(en/yn)yl, ArC₁₋₆-alk(en/yn)yl, Ar-C₃₋₈-cycloalk(en)yl, Ar-C₃₋₈-cycloalk(en)yl-C₁₋₆-alk(en/yn)yl and Ar; and
each of R4, R5, R6 and R7 is independently selected from the group consisting of hydrogen and Ar;
or pharmaceutically acceptable salts thereof; and
where formula 6 is: 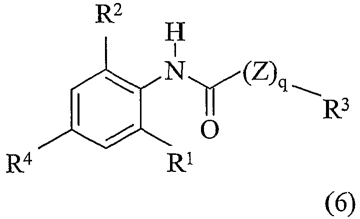 wherein
Z is O or S;
and
q is 0 or 1;
and
each of R¹ and R² is independently selected from the group consisting of halogen, cyano, amino, C₁₋₆-alk(en/yn)yl, C₃₋₈-cycloalk(en)yl, C₃₋₈-cycloalk(en)yl-C₁₋₆-alk(en/yn)yl, C₃₋₈-heterocycloalk(en)yl, Aryl, Heteroaryl, halo-C₁₋₆-alk(en/yn)yl, halo-C₃₋₈-cycloalk(en)yl, halo-C₃₋₈-cycloalk(en)yl-C₁₋₆-alk(en/yn)yl, C₁₋₆-alk(en/yn)yloxy, C₃₋₈-cycloalk(en)yloxy, C₃₋₈-cycloalk(en)yl-C₁₋₆-alk(en/yn)yloxy, C₃-ₛ-heterocycloalk(en)yloxy;
and
R³ is selected from the group consisting of C₁₋₈-alk(en/yn)yl, C₃₋₈-cycloalk(en)yl, C₃₋₈-cycloalk(en)yl-C₁₋₆-alk(en/yn)yl, Aryl-C₁₋₆-alk(en/yn)yl, Aryl-C₃₋₈-cycloalk(en)yl, Aryl-C₃₋₈-cycloalk(en)yl-C₁₋₆-alk(en/yn)yl, C₃₋₈-heterocycloalk(en)yl-C₁₋₆-alk(en/yn)yl, C₁₋₆-alk(en/yn)yl-C₃₋₈-heterocycloalk(en)yl-C₁₋₆-alk(en/yn)yl, Heteroaryl-C₁₋₆-alk(en/yn)yl, Heteroaryl-C₃₋₈-cycloalk(en)yl, Heteroaryl-C₃₋₈-cycloalk(en)yl-C₁₋₆-alk(en/yn)yl, amino-C₁₋₆-alk(en/yn)yl, amino-C₃₋₈-cycloalk(en)yl, amino-C₃₋₈-cycloalk(en)yl-C₁₋₆-alk(en/yn)yl, C₁₋₆-alk(en/yn)yloxy-C₁₋₆-alk(en/yn)yl, C₃₋₈-cycloalk(en)yloxy-C₁₋₆-alk(en/yn)yl, C₃₋₈-cycloalk(en)yl-C₁₋₆-alk(en/yn)yloxy-C₁₋₆-alk(en/yn)yl, halo-C₁₋₆-alk(en/yn)yl, halo-C₃₋₈-cycloalk(en)yl and halo-C₃₋₈-cycloalk(en)yl-C₁₋₆-alk(en/yn)yl;
and
R⁴ is selected from the group consisting of halogen, cyano, C₁₋₆-alk(en/yn)yl, C₃₋₈-cycloalk(en)yl, C₃₋₈-cycloalk(en)yl-C₁₋₆-alk(en/yn)yl, C₃₋₈-heterocycloalk(en)yl, Aryl, Heteroaryl, Aryl-C₁₋₆-alk(en/yn)yl, Aryl-C₃₋₈-cycloalk(en)yl, Aryl-C₃₋₈-cycloalk(en)yl-C₁₋₆-alk(en/yn)yl, Aryl-C₃₋₈-heterocycloalk(en)yl, halo-C₁₋₆-alk(en/yn)yl, halo-C₃₋₈-cycloalk(en)yl, halo-C₃₋₈-cycloalk(en)yl-C₁₋₆-alk(en/yn)yl, halo-C₁₋₆-alk(en/yn)yl-C₃₋₈-heterocycloalk(en)yl-C₁₋₆-alk(en/yn)yl, NR⁵R⁶ and R⁷NH-C₁₋₆-alk(en/yn)yl; wherein R⁵ and R⁶ are independently selected from the group consisting of hydrogen, Aryl-C₁₋₆-alk(en/yn)yl, Aryl-C₃₋₈-cycloalk(en)yl, Aryl-C₃₋₈-cycloalk(en)yl-C₁₋₆-alk(en/yn)yl, C₁₋₆-alk(en/yn)yl, C₃₋₈-cycloalk(en)yl, C₃₋₈-cycloalk(en)yl-C₁₋₆-alk(en/yn)yl, Heteroaryl-C₁₋₆-alk(en/yn)yl, Heteroaryl-C₃₋₈-cycloalk(en)yl and Heteroaryl-C₃₋₈-cycloalk(en)yl-C₁₋₆-alk(en/yn)yl with the proviso that R⁵ and R⁶ are not hydrogen at the same time; and R⁷ is selected from the group consisting of C₁₋₆-alk(en/yn)yl, C₃₋₈-cycloalk(en)yl, C₃₋₈-cycloalk(en)yl-C₁₋₆-alk(en/yn)yl, Aryl, halo-C₁₋₆-alk(en/yn)yl, halo-C₃₋₈-cycloalk(en)yl, halo-C₃₋₈-cycloalk(en)yl-C₁₋₆-alk(en/yn)yl, Aryl-C₁₋₆-alk(en/yn)yl, Aryl-C₃₋₈-cycloalk(en)yl and Heteroaryl;
or pharmaceutically acceptable salts thereof; and
where formula 7 is: 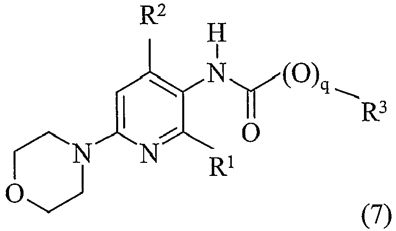 wherein:
q is 0 or 1;
each of R¹ and R² is independently selected from the group consisting of halogen, cyano, C₁₋₆-alk(en/yn)yl, C₃₋₈-cycloalk(en)yl, C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, C₁₋₆-alk(en/yn)yloxy, C₃₋₈-cycloalk(en)yloxy and C₃₋₈-cycloalk(en)yl-C₁₋₆-alk(en/yn)yloxy; and
R³ is selected from the group consisting of C₁₋₈-alk(en/yn)yl, C₃₋₈-cycloalk(en)yl, C₃₋₈-cycloalk(en)yl-C₁₋₆-alk(en/yn)yl, optionally substituted Aryl-C₁₋₆-alk(en/yn)yl, optionally substituted Aryl-C₃₋₈-cycloalk(en)yl, optionally substituted Axyl-C₃₋₈-cycloalk(en)yl-C₁₋₆-alk(en/yn)yl, C₁₋₆-alk(en/yn)yl-C₃₋₈-heterocycloalk(en)yl-C₁₋₆-alk(en/yn)yl, C₃₋₈-heterocycloalk(en)yl-C₁₋₆-alk(en/yn)yl, C₁₋₆-alk(en/yn)yl-C₃₋₈-heterocycloalk(en)yl-C₁₋₆-alk(en/yn)yl, Heteroaryl-C₁₋₆-alk(en/yn)yl, Heteroaryl-C₃₋₈-cycloalk(en)yl, Heteroaryl-C₃₋₈-cycloalk(en)yl-C₁₋₆-alk(en/yn)yl, NR⁴R⁵-C₁₋₆-alk(en/yn)yl, NR⁴R⁵-C₃₋₈-cycloalk(en)yl, NR⁴R⁵-C₃₋₈-cycloalk(en)yl-C₁₋₆-alk(en/yn)yl, C₁₋₆-alk(en/yn)yloxy-C₁₋₆-alk(en/yn)yl, C₃₋₈-cycloalk(en)yloxy-C₁₋₆-alk(en/yn)yl, C₃₋₈-cycloalk(en)yl-C₁₋₆-alk(en/yn)yloxy-C₁₋₆-alk(en/yn)yl, halo-C₁₋₆-alk(en/yn)yl, halo-C₃₋₈-cycloalk(en)yl and halo-C₃₋₈-cycloalk(en)yl-C₁₋₆-alk(en/yn)yl; wherein
each of R⁴ and R⁵ is independently selected from the group consisting of hydrogen, C₁₋₆-alk(en/yn)yl, C₃₋₈-cycloalk(en)yl and C₃₋₈-cycloalk(en)yl-C₁₋₆-alk(en/yn)yl;
or pharmaceutically acceptable salts thereof; and
where formula 8 is: 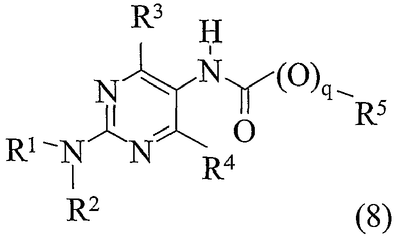 wherein: q is 0 or 1;
R¹ and R² are independently selected from the group consisting of hydrogen and optionally substituted aryl-C₁₋₆-alk(en/yn)yl, provided that R¹ and R² are not both hydrogen, or R¹ and R² together with the nitrogen to which they are attached form a 5 to 7 membered ring optionally containing a further heteroatom;
R³ and R⁴ are independently selected from hydrogen, halogen, cyano, amino, C₁₋₆-alk(en/yn)yl, C₃₋₈-cycloalk(en)yl, halo-C₁₋₆-alk(en/yn)yl, halo-C₃₋₈-cycloalk(en)yl, C₁₋₆-alk(en/yn)yloxy, C₃₋₈-cycloalk(en)yloxy, C₃₋₈-cycloalk(en)yl-C₁₋₆-alk(en/yn)yloxy, halo-C₁₋₆-alk(en/yn)yloxy, halo-C₃₋₈-cycloalk(en)yloxy and halo-C₃₋₈-cycloalk(en)yl-C₁₋₆-alk(en/yn)yloxy, provided that R³ and R⁴ are not both hydrogen;
R⁵ is selected from the group consisting of C₁₋₁₀-alk(en/yn)yl, C₃₋₈-cycloalk(en)yl-C₁₋₆-alk(en/yn)yl, optionally substituted aryl-C₁₋₆-alk(en/yn)yl and optionally substituted aryl;
or pharmaceutically acceptable salts thereof; and
where formula 9 is: 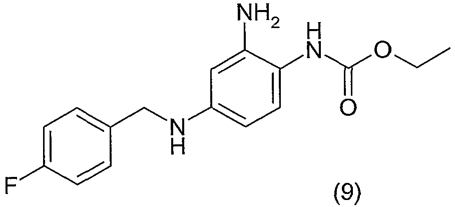 or a pharmaceutically acceptable salt thereof.

In yet another embodiment, the invention relates to a method wherein the compound is selected from the group consisting of: N-(2-amino-4-(4-fluorobenzylamino)-phenyl) carbamic acid ethyl ester;
2-Cyclopentyl-N-(2,6-dimethyl-4-morpholin-4-yl-phenyl)-acetamide; N-(2,6-Dimethyl-4-morpholin-4-yl-phenyl)-3,3-dimethyl-butyramide; N-(4,6-Dimethyl-2-morpholin-4-yl-pyrimidin-5-yl)-2-(4-fluoro-henyl)-acetamide; Hexanoic acid (2,6-difluoro-4-morpholin-4-yl-phenyl)-amide; 2-Cyclopentyl-N-(4,6-dimethyl-2-morpholin-4-yl-pyrimidin-5-yl)-acetamide; N-(2-Bromo-4-morpholin-4-yl-6-trifluoromethyl-phenyl)-propionamide; N-(2,4-Dimethyl-6-morpholin-4-yl-pyridin-3-yl)-3,3-dimethyl-butyramide; [2-Amino-4-(2,4,6-trimethyl-benzylamino)-phenyl]-carbamic acid ethyl ester; and 2-Cyclopentyl-N-(2-methoxy-6-methyl-4-morpholin-4-yl-phenyl)-acetamide.

The term "KCNQ potassium channel" refers to homomeric or heteromeric potassium channels composed of at least one subunit of one of the KCNQ channels selected from the group of KCNQ2, KCNQ3, KCNQ4 and KCNQ5.

The term "a compound able to selectively increase the ion flow through KCNQ potassium channels" refers to compounds that opens a KCNQ potassium channel but not to any significant degree other potassium channels and preferably does not to any significant degree modulate any other channels or receptors.

The term "modulate" or "modulation" in respect of a channel or a receptor refers to an antagonistic or agonist effect on said channel or receptor.

The term "anti-psychotic potential" in relation to a compound refers to a compound that has the potential to treat or reduce one or more symptoms of a psychotic disorder. One such psychotic disorder is schizophrenia.

The term "treatment" as used herein in connection with a disease or disorders includes also prevention, inhibition and amelioration as the case may be.

The term "acute treatment" refers to the introduction or reintroduction of a compound according to the invention to alleviate (or at least palliate) an exacerbation of psychosis.

The term "long-term treatment" refers to maintanence or life-long treatment.

The term "host" refers refers to any mammal. The host, such as a human, to be treated with a compound according to the invention may in fact be any subject of the human population, male or female, which may be divided into children, adults, or elderly. Any one of these patient groups relates to an embodiment of the invention.

The term "effective amount" refers to to the amount/dose of a compound or pharmaceutical composition that is sufficient to produce an effective response (i.e., a biological or medical response of a tissue, system, animal or human sought by a researcher, veterinarian, medical doctor or other clinician) upon administration to a subject. The "effective amount" will vary depending on *inter alia* the disease and its severity, and the age, weight, physical condition and responsiveness of the subject to be treated.

A potential of a compound to treat anti-psychotic disorders, where one such compound is able to control agitated psychotic behavior, alleviate acute psychotic states, reduce psychotic symptoms, and exert a quieting effect, is supported by in vivo behavioural tests reflective of antipsychotic-like behaviour such as inhibition of stimulant-induced hyperactivity, inhibition of a sensitised response (hyperactivity) to amphetamine, and inhibition of conditioned avoidance responses.

A potential of a compound to treat the positive symptoms of schizophrenia, where positive symptoms is defined as a symptom cluster of schizophrenia comprising delusion formation and hallucinations (visceral, visual, auditory), is supported by in vivo behavioural tests reflective of antipsychotic-like behaviour such as inhibition of stimulant-induced hyperactivity, inhibition of a sensitised response (hyperactivity) to amphetamine, conditioned avoidance response.

A potential of a compound to treat the negative symptoms of schizophrenia, where negative symptoms is defined as a symptom cluster of schizophrenia comprising emotional disharmony and regressive behaviour, is supported by positive effects in the forced swim test, an in vivo behavioural test reflective of antidepressant-like behaviour.

A potential of a compound for fast-onset of therapeutic efficacy is defined as the potential for a compound to exert a fast onset of clinical therapeutic efficacy i.e. a faster onset than seen with clinically used compounds within a given indication area, is supported by in vivo electrophysiological assessments of the spontaneous firing rate of dopamine cells in the ventral tegmental area, showing acute inhibitory effects of compound (as opposed to inhibitory effects only after chronic dosing).

Lack of D2 antagonism related side-effects is defined as avoidance of D2 receptor-related side effects given the lack of direct involvement of D2 receptors in the mechanism of action of the mentioned compounds.

Antibipolar disorder potential is defined as a potential to treat bipolar disorder, a major affective disorder that is characterised by severe mood swings (mania and/or depression) and a tendency to remission and recurrence.

Antimanic potential is defined as a potential to treat mania, a part of the bipolar disorder episode spectrum, that is supported by in vivo behavioural tests reflective of antimanic-like behaviour such as inhibition of stimulant-induced hyperactivity and inhibition of a sensitised response (hyperactivity) to amphetamine.

Anti-bipolar depression potential is defined as a potential to treat bipolar depression, a part of the bipolar disorder episode spectrum that is supported by positive effects in the the forced swim test, an in vivo behavioural test reflective of antidepressant-like behaviour.

Antidepressant potential is defined as a potential to treat patients suffering from major depression, this is supported by positive effects in the forced swim test, an in vivo behavioural test reflective of antidepressant-like behaviour.

The acute stimulant-induced hyperactivity test is defined as an in vivo test involving rats that a given an acute s.c. injection of amphetamine-sulphate causing increased locomotor activitiy (psychotic-like behaviour) that can be reversed by anti-psychotic and anti-manic compounds.

The sensitised amphetamine-induced hyperactivity test is defined as an in vivo test involving mice that have been treated intermittently with amphetamine and thus become sensitised (exaggerated locomotor activity response) to sub-sequent doses of amphetamine-sulphate. The exaggerated response can be reversed by anti-psychotic and anti-manic compounds.

The spontaneous firing of mesolimbic DA cells test is defined as an in vivo test involving anaesthetised rats where the spontaneous firing rate of dopamine neurons in the ventral tegmental area is assessed.

The forced swim test is defined as an in vivo test involving mice where the time spent immobile while immersed in water is assessed during a short experimental period (minutes). Antidepressant compounds reduce this behaviour.

Compounds according to formula 1 can be prepared as described in WO2004/058739. Compounds according to formula 2 can be prepared as described in WO2004/082677. Compounds according to formula 3 can be prepared as described in WO2004/080950. Compounds according to formula 4 can be prepared as described in WO2004/096767. Compounds according to formula 5 can be prepared as described in WO2005/087754. Compounds according to formula 6 can be prepared as described in WO2006/029623. Compounds according to formula 7 can be prepared as described in WO2006/092143. Compounds according to formula 8 can be prepared as described in PCT/DK06/050039.

The compound according to formula 9 can be prepared as described in EP554543.

In another embodiment, the invention relates a compound according to formula 1 wherein said compound is selected from the group of:
{2-Amino-4-[(5-chloro-thiophen-2-ylmethyl)-methyl-amino]-phenyl}-carbamic acid ethyl ester; {2-Amino-4-[(5-chloro-thiophen-2-ylmethyl)-amino]-phenyl}-carbamic acid ethyl ester; {2-Amino-4-[(5-methyl-thiophen-2-ylmethyl)-methyl-amino]-phenyl}-carbamic acid ethyl ester; {2-Amino-4-[(5-bromo-thiophen-2-ylmethyl)-amino]-phenyl}-carbamic acid ethyl ester; {2-Amino-4-[(6-chloro-3-methoxy-benzo[b]thiophen-2-ylmethyl)-amino]-phenyl}-carbamic acid ethyl ester; {2-Amino-4-[(benzo[b]thiophen-2-ylmethyl)-amino]-phenyl}-carbamic acid ethyl ester; {2-Amino-4-[(5-methyl-thiophen-2-ylmethyl)-amino]-phenyl}-carbamic acid ethyl ester;
{2-Amino-4-[(4-bromo-3-methoxy-thiophen-2-ylmethyl)-amino]-phenyl}-carbamic acid ethyl ester; {2-Amino-4-[(5-phenyl-thiophen-2-ylmethyl)-amino]-phenyl}-carbamic acid ethyl ester; {2-Amino-4-[(3-chloro-thiophen-2-ylmethyl)-amino]-phenyl}-carbamic acid ethyl ester; (2-Amino-4-{[4-(4-chloro-benzenesulfonyl)-3-methyl-thiophen-2-ylmethyl]-amino}-phenyl)-carbamic acid ethyl ester; {2-Amino-4-[(3-methyl-thiophen-2-ylmethyl)-amino]-phenyl}-carbamic acid ethyl ester; {2-Amino-4-[(5-fluoro-benzofuran-3-ylmethyl)-amino]-phenyl}-carbamic acid ethyl ester; {2-Amino-4-[(thiophen-2-ylmethyl)-amino]-phenyl}-carbamic acid ethyl ester;
{2-Amino-4-[(4-bromo-thiophen-2-ylmethyl)-amino]-phenyl}-carbamic acid ethyl ester; {2-Amino-4-[(5-ethyl-thiophen-2-ylmethyl)-amino]-phenyl}-carbamic acid ethyl ester; {2-Amino-4-[(thiophen-3-ylmethyl)-amino]-phenyl}-carbamic acid ethyl ester; {2-Amino-4-[(5-chloro-thiophen-2-ylmethyl)-ethyl-amino]-phenyl}-carbamic acid ethyl ester; {2-Amino-4-[(benzo[b]thiophen-3-ylmethyl)-amino]-phenyl}-carbamic acid ethyl ester; {2-Amino-4-[(5-dimethyl-amino-benzo[b]thiophen-3-ylmethyl)-amino]-phenyl}-carbamic acid ethyl ester; {2-Amino-4-[(5-dimethy-lamino-3-methyl-benzo[b]thiophen-2-ylmethyl)-amino]-phenyl}-carbamic acid ethyl ester; {2-Amino-4-[(5-fluoro-thiophen-2-ylmethyl)-amino]-phenyl}-carbamic acid ethyl ester; {2-Amino-4-[(benzo[b]thiophen-2-ylmethyl)-amino]-phenyl}-carbamic acid propyl ester; {2-Amino-4-[(benzo[b]thiophen-3-ylmethyl)-amino]-phenyl}-carbamic acid propyl ester; N-{2-Amino-4-[(5-chloro-thiophen-2-ylmethyl)amino]phenyl}-2-(4-fluoro-phenyl)-acetamide; and N-{2-Amino-4-[(5-chloro-thiophen-2-ylmethyl)amino]phenyl} -3,3-dimethyl-butyramide, or a pharmaceutically acceptable salt thereof.

In another embodiment, the invention relates a compound according to formula 2 wherein said compound is selected from the group of:
{4-[(Benzofuran-2-ylmethyl)-amino]-2-methylphenyl}-carbamic acid propyl ester;
{4-[(5-Chloro-thiophen-2-ylmethyl)-amino]-2-methylphenyl}-carbamic acid ethyl ester; {4-[(Benzo[b]thiophen-2-ylmethyl)-amino]-2-methylphenyl}-carbamic acid ethyl ester; {2-Methyl-4-[(5-phenyl-thiophen-2-ylmethyl)-amino]-phenyl}-carbamic acid ethyl ester; [4-(4-Isopropyl-benzylamino)-2-methylphenyl]-carbamic acid ethyl ester; [4-(4-Fluoro-benzylamino)-2-methylphenyl]-carbamic acid propyl ester;
(4- {[4-(4-Chloro-benzenesulfonyl)-3-methyl-thiophen-2-ylmethyl]-amino}-2-methylphenyl)-carbamic acid propyl ester; {4-[(5-Methyl-thiophen-2-ylmethyl)-amino]-2-methylphenyl}-carbamic acid propyl ester; {4-[(5-Bromo-thiophen-2-ylmethyl)-amino]-2-methylphenyl}-carbamic acid propyl ester; {4-[(5-Chloro-thiophen-2-ylmethyl)-amino]-2-methylphenyl}-carbamic acid propyl ester; {4-[(Benzo[b]thiophen-2-ylmethyl)-amino]-2-methylphenyl}-carbamic acid propyl ester; {2-Methyl-4-[(5-phenyl-thiophen-2-ylmethyl)-amino]-phenyl} -carbamic acid propyl ester; [4-(4-Isopropyl-benzylamino)-2-methylphenyl]-carbamic acid propyl ester;
{4-[(5-Bromo-thiophen-2-ylmethyl)-amino]-2-chlorophenyl} -carbamic acid ethyl ester; {4-[(5-Chloro-thiophen-2-ylmethyl)-amino]-2-chlorophenyl}-carbamic acid ethyl ester; {4-[(Benzo[b]thiophen-2-ylmethyl)-amino]-2-chlorophenyl}-carbamic acid ethyl ester; [2-Chloro-4-(4-isopropyl-benzylamino)-phenyl]-carbamic acid ethyl ester; [2-Chloro-4-(4-fluoro-benzylamino)-phenyl]-carbamic acid propyl ester; 2-Chloro-4- {[4-(4-chloro-benzenesulfonyl)-3-methyl-thiophen-2-ylmethyl]-amino}-phenyl)-carbamic acid propyl ester; {4-[(5-Methyl-thiophen-2-ylmethyl)-amino]-2-chlorophenyl}-carbamic acid propyl ester; {4-[(5-Bromo-thiophen-2-ylmethyl)-amino]-2-chlorophenyl}-carbamic acid propyl ester; {2-Chloro-4-[(5-chloro-thiophen-2-ylmethyl)-amino]-phenyl}-carbamic acid propyl ester; {4-[(Benzo[b]thiophen-2-ylmethyl)-amino]-2-chlorophenyl}-carbamic acid propyl ester; {4-[(Benzofuran-2-ylmethyl)-amino]-2-chlorophenyl}-carbamic acid propyl ester; {4-[(5-Chloro-thiophen-2-ylmethyl)-amino]-2-cyanophenyl}-carbamic acid ethyl ester; {4-[(Benzo[b]thiophen-2-ylmethyl)-amino]-2-methoxyphenyl}-carbamic acid methyl ester; {4-[(5-Bromo-thiophen-2-ylmethyl)-amino]-2-methoxyphenyl}-carbamic acid isopropyl ester; {4-[(4-Fluoro-benzyl)-(methyl)amino]-2-methoxyphenyl}-carbamic acid propyl ester; [4-(Benzo[b]thiophen-2-ylmethyl-(methyl)amino)-2-methoxy-phenyl]-carbamic acid propyl ester; {4-[(5-Chloro-thiophen-2-ylmethyl)-(methyl)amino]-2-methoxy-phenyl}-carbamic acid propyl ester; {4-[(5-Bromo-thiophen-2-ylmethyl)-(methyl)amino]-2-methoxy-phenyl}-carbamic acid propyl ester;
{2-Methoxy-4-[methyl-(5-methyl-thiophen-2-ylmethyl)-amino]-phenyl}-carbamic acid propyl ester; {4-[(4-Fluorobenzyl)-(methyl)-amino]-2-isopropoxyphenyl}-carbamic acid ethyl ester; [4-(3-Fluorobenzylamino)-2-methoxyphenyl]-carbamic acid ethyl ester; [4-(4-Isopropylbenzylamino)-2-methoxyphenyl]-carbamic acid ethyl ester; {2-Methoxy-4-[(3-methylthiophen-2-ylmethyl)-amino]-phenyl}-carbamic acid ethyl ester; [4-(2,4-Difluorobenzylamino)-2-methoxyphenyl]-carbamic acid ethyl ester; [2-Cyclopentyloxy-4-(4-methoxybenzylamino)-phenyl]-carbamic acid ethylester; [2-Cyclopentyloxy-4-(3-fluoro-2-methylbenzylamino)-phenyl]-carbamic acid ethyl ester; [4-(3-Fluoro-2-methylbenzylamino)-2-phenethyloxyphenyl]-carbamic acid ethyl ester; [2-Benzyloxy-4-(3-fluoro-2-methylbenzylamino)-phenyl]-carbamic acid ethyl ester; [2-Benzyloxy-4-(4-methylsulfanylbenzylamino)-phenyl]-carbamic acid ethyl ester; {4-[(Benzo[b]thiophen-3-ylmethyl)-amino]-2-cyclopentyloxyphenyl}-carbamic acid ethyl ester; [4-(3-Fluoro-2-methylbenzylamino)-2-isopropoxyphenyl]-carbamic acid ethyl ester; [2-Benzyloxy-4-(3-methoxybenzylamino)-phenyl]-carbamic acid ethyl ester; {4-[(Benzo[1,3]dioxol-5-ylmethyl)-amino]-2-isopropoxyphenyl}-carbamic acid ethyl ester; {4-[(5-Bromo-thiophen-2-ylmethyl)-amino]-phenyl}-carbamic acid propyl ester; {4-[(5-Chloro-thiophen-2-ylmethyl)-amino]-phenyl}-carbamic acid propyl ester; [2-Cyano-4-(4-isopropylbenzylamino)-phenyl]-carbamic acid ethyl ester; {4-[(5-Bromo-thiophen-2-ylmethyl)-(methyl)amino]-2-methylphenyl}-carbamic acid propyl ester; {4-[(4-Isopropylbenzyl)-(methyl)amino]-2-methylphenyl}-carbamic acid propyl ester; {2-Methyl-4-[methyl-(4-trifluoromethyl-benzyl)-amino]-phenyl}-carbamic acid propyl ester; {2-Methyl-4-[methyl-(4-methylsulfanyl-benzyl)-amino]-phenyl}-carbamic acid propyl ester; {4-[(4-tert-Butyl-benzyl)-(methyl)amino]-2-chlorophenyl}-carbamic acid ethyl ester; {2-Chloro-4-[methyl-(4-trifluoromethyl-benzyl)-amino]-phenyl}-carbamic acid ethyl ester; {2-Chloro-4-[methyl-(4-methylsulfanyl-benzyl)-amino]-phenyl}-carbamic acid ethyl ester;
{4-[(5-Bromo-thiophen-2-ylmethyl)-(methyl)amino]-2-chlorophenyl}-carbamic acid propyl ester; {2-Chloro-4-[(5-chloro-thiophen-2-ylmethyl)-(methyl)amino]-phenyl}-carbamic acid propyl ester; {4-[(4-tert-Butyl-benzyl)-(methyl)amino]-2-chlorophenyl}-carbamic acid propyl ester; {2-Chloro-4-[methyl-(4-trifluoromethylbenzyl)-amino]-phenyl}-carbamic acid propyl ester; {4-[(5-Bromo-thiophen-2-ylmethyl)-(methyl)amino]-2-trifluoromethyl-phenyl}-carbamic acid ethyl ester; {4-[(5-Chloro-thiophen-2-ylmethyl)-(methyl)amino]-2-trifluoromethyl-phenyl}-carbamic acid ethyl ester; {4-[(4-Isopropyl-benzyl)-(methyl)amino]-2-trifluoromethyl-phenyl}-carbamic acid ethyl ester; {4-[(4-tert-Butyl-benzyl)-(methyl)amino]-2-trifluoromethyl-phenyl}-carbamic acid ethyl ester; {4-[Methyl-(4-trifluoromethyl-benzyl)-amino]-2-trifluoromethyl-phenyl}-carbamic acid ethyl ester; {4-[Methyl-(4-methylsulfanyl-benzyl)-amino]-2-trifluoromethyl-phenyl}-carbamic acid ethyl ester; {4-[(5-Bromo-thiophen-2-ylmethyl)-(methyl)amino]-2-trifluoromethyl-phenyl}-carbamic acid propyl ester; {4-[(5-Chloro-thiophen-2-ylmethyl)-(methyl)amino]-2-trifluoromethyl-phenyl}-carbamic acid propyl ester; {4-[(4-Isopropyl-benzyl)-(methyl)amino]-2-trifluoromethyl-phenyl}-carbamic acid propyl ester; {4-[(4-tert-Butyl-benzyl)-(methyl)amino]-2-trifluoromethyl-phenyl}-carbamic acid propyl ester; {4-[Methyl-(4-trifluoromethyl-benzyl)-amino]-2-trifluoromethyl-phenyl}-carbamic acid propyl ester; {4-[Methyl-(4-methylsulfanyl-benzyl)-amino]-2-trifluoromethyl-phenyl}-carbamic acid propyl ester; {4-[(5-Bromo-thiophen-2-ylmethyl)-(methyl)amino]-2-cyanophenyl}-carbamic acid propyl ester; {4-[(4-tert-Butyl-benzyl)-(methyl)amino]-2-cyanophenyl}-carbamic acid propyl ester; {2-Cyano-4-[methyl-(4-trifluoromethyl-benzyl)-amino]-phenyl}-carbamic acid propyl ester; {2-Bromo-4-[(5-bromo-thiophen-2-ylmethyl)-(methyl)amino]-phenyl}-carbamic acid propyl ester; {2-Bromo-4-[(5-chloro-thiophen-2-ylmethyl)-(methyl)amino]-phenyl}-carbamic acid propyl ester; {2-Bromo-4-[(4-isopropylbenzyl)-(methyl)amino]-phenyl}-carbamic acid propyl ester; {2-Bromo-4-[(4-tert-butyl-benzyl)-(methyl)amino]-phenyl}-carbamic acid propyl ester; {2-Bromo-4-[methyl-(4-trifluoromethyl-benzyl)-amino]-phenyl}-carbamic acid propyl ester; [2-Iodo-4-(4-isopropyl-benzylamino)-phenyl]-carbamic acid propyl ester; [4-(4-tert-Butyl-benzylamino)-2-iodophenyl]-carbamic acid propyl ester; [2-Iodo-4-(4-trifluoromethyl-benzylamino)-phenyl]-carbamic acid propyl ester; [2-Iodo-4-(4-methylsulfanyl-benzylamino)-phenyl]-carbamic acid propyl ester; {2-Iodo-4-[4-(4-methylpiperazin-1-yl)-benzylamino]-phenyl}-carbamic acid propyl ester; {4-[(5-Bromo-thiophen-2-ylmethyl)-amino]-2-trifluoromethyl-phenyl}-carbamic acid ethyl ester; {4-[(5-Chloro-thiophen-2-ylmethyl)-amino]-2-trifluoromethyl-phenyl}-carbamic acid ethyl ester; [4-(4-tert-Butyl-benzylamino)-2-trifluoromethyl-phenyl]-carbamic acid ethyl ester; [4-(4-Methylsulfanyl-benzylamino)-2-trifluoromethylphenyl]-carbamic acid ethyl ester; {4-[(5-Bromo-thiophen-2-ylmethyl)-amino]-2-trifluoromethyl-phenyl}-carbamic acid propyl ester; [4-(4-Isopropylbenzylamino)-2-trifluoromethyl-phenyl]-carbamic acid propyl ester; [4-(4-tert-Butyl-benzylamino)-2-trifluoromethyl-phenyl]-carbamic acid propyl ester; [2-Trifluoromethyl-4-(4-trifluoromethyl-benzylamino)-phenyl]-carbamic acid propyl ester; [4-(4-Dimethylamino-benzylamino)-2-trifluoromethyl-phenyl]-carbamic acid propyl ester; [4-(4-Methylsulfanyl-benzylamino)-2-trifluoromethyl-phenyl]-carbamic acid propyl ester; {4-[(5-Bromo-thiophen-2-ylmethyl)-amino]-2-cyanophenyl}-carbamic acid propyl ester; {4-[(5-Chloro-thiophen-2-ylmethyl)-amino]-2-cyanophenyl}-carbamic acid propyl ester; [2-Cyano-4-(4-trifluoromethyl-benzylamino)-phenyl]-carbamic acid propyl ester; {2-Bromo-4-[(5-bromo-thiophen-2-ylmethyl)-amino]-phenyl}-carbamic acid propyl ester; {2-Bromo-4-[(5-chloro-thiophen-2-ylmethyl)-amino]-phenyl}-carbamic acid propyl ester; [2-Bromo-4-(4-isopropylbenzylamino)-phenyl]-carbamic acid propyl ester; [2-Bromo-4-(4-tert-butyl-benzylamino)-phenyl]-carbamic acid propyl ester; [2-Bromo-4-(4-trifluoromethyl-benzylamino)-phenyl]-carbamic acid propyl ester; [2-Bromo-4-(4-methylsulfanyl-benzylamino)-phenyl]-carbamic acid propyl ester; N-{4-[(5-Bromo-thiophen-2-ylmethyl)-amino]-2-methoxyphenyl}-butyramide; N-{4-[(5-Chloro-thiophen-2-ylmethyl)-amino]-2-methoxyphenyl}-butyramide; N-[4-(4-Isopropylbenzylamino)-2-methoxyphenyl]-butyramide; N-[4-(4-tert-Butyl-benzylamino)-2-methoxyphenyl]-butyramide; N-[2-Methoxy-4-(4-trifluoromethyl-benzylamino)-phenyl]-butyramide; {4-[(5-Chloro-thiophen-2-ylmethyl)-amino]-2-furan-2-yl-phenyl}-carbamic acid propyl ester; [2-Furan-2-yl-4-(4-isopropylbenzylamino)-phenyl]-carbamic acid propyl ester; [5-(4-Fluorobenzylamino)-biphenyl-2-yl]-carbamic acid propyl ester; {5-[(5-Chloro-thiophen-2-ylmethyl)-amino]-biphenyl-2-yl}-carbamic acid propyl ester; [5-(4-Isopropylbenzylamino)-biphenyl-2-yl]-carbamic acid propyl ester; N-{2-Chloro-4-[(5-chloro-thiophen-2-ylmethyl)-(methyl)amino]-phenyl}-2-phenylacetamide; N- {2-Chloro-4-[(5-chloro-thiophen-2-ylmethyl)-(methyl)amino]-phenyl}-3,3-dimethylbutyramide; N-{2-Chloro-4-[(5-chloro-thiophen-2-ylmethyl)-(methyl)amino]-phenyl}-3-phenylpropionamide; N-{2-Chloro-4-[(5-chloro-thiophen-2-ylmethyl)-(methyl)amino]-phenyl}-butyramide; Pentanoic acid {2-chloro-4-[(5-chloro-thiophen-2-ylmethyl)-(methyl)amino]-phenyl}-amide; Cyclopropanecarboxylic acid {2-chloro-4-[(5-chloro-thiophen-2-ylmethyl)-(methyl)amino]-phenyl}-amide; Cyclobutanecarboxylic acid {2-chloro-4-[(5-chloro-thiophen-2-ylmethyl)-(methyl)amino]-phenyl}-amide; Cyclopentanecarboxylic acid {2-chloro-4-[(5-chloro-thiophen-2-ylmethyl)-(methyl)amino]-phenyl}-amide; Cyclohexanecarboxylic acid {2-chloro-4-[(5-chloro-thiophen-2-ylmethyl)-(methyl)amino]-phenyl}-amide; N-{2-Chloro-4-[(5-chloro-thiophen-2-ylmethyl)-(methyl)amino]-phenyl}-2-thiophen-2-yl-acetamide; N-{2-Chloro-4-[(5-chloro-thiophen-2-ylmethyl)-(methyl)amino]-phenyl}-2-(3-methoxy-phenyl)-acetamide; N-{2-Chloro-4-[(5-chloro-thiophen-2-ylmethyl)-(methyl)amino]-phenyl}-2-(4-chlorophenyl)-acetamide; N-{2-Chloro-4-[(5-chloro-thiophen-2-ylmethyl)-(methyl)amino]-phenyl}-2-(4-methoxy-phenyl)-acetamide;
N-{2-Chloro-4-[(5-chloro-thiophen-2-ylmethyl)-(methyl)amino]-phenyl}-2-(4-fluorophenyl)-acetamide; N-{2-Chloro-4-[(5-chloro-thiophen-2-ylmethyl)-(methyl)amino]-phenyl}-3-cyclohexylpropionamide; N-{2-Chloro-4-[(5-chloro-thiophen-2-ylmethyl)-amino]-phenyl}-2,2-dimethylpropionamide; N-{2-Chloro-4-[(5-chloro-thiophen-2-ylmethyl)-amino]-phenyl}-2-phenoxyacetamide; N-{2-Chloro-4-[(5-chloro-thiophen-2-ylmethyl)-amino]-phenyl}-2-phenylacetamide; N-{2-Chloro-4-[(5-chloro-thiophen-2-ylmethyl)-amino]-phenyl}-3,3-dimethylbutyramide; N-{2-Chloro-4-[(5-chloro-thiophen-2-ylmethyl)-amino]-phenyl}-butyramide; Pentanoic acid {2-chloro-4-[(5-chloro-thiophen-2-ylmethyl)-amino]-phenyl}-amide; Cyclopropanecarboxylic acid {2-chloro-4-[(5-chloro-thiophen-2-ylmethyl)-amino]-phenyl}-amide; Cyclobutanecarboxylic acid {2-chloro-4-[(5-chloro-thiophen-2-ylmethyl)-amino]-phenyl}-amide; Cyclopentanecarboxylic acid {2-chloro-4-[(5-chloro-thiophen-2-ylmethyl)-amino]-phenyl}-amide; Cyclohexanecarboxylic acid {2-chloro-4-[(5-chloro-thiophen-2-ylmethyl)-amino]-phenyl}-amide; N-{2-Chloro-4-[(5-chloro-thiophen-2-ylmethyl)-amino]-phenyl}-2-thiophen-2-yl-acetamide; N-{2-Chloro-4-[(5-chloro-thiophen-2-ylmethyl)-amino]-phenyl}-2-(3-methoxyphenyl)-acetamide; N-{2-Chloro-4-[(5-chloro-thiophen-2-ylmethyl)-amino]-phenyl}-2-(4-chlorophenyl)-acetamide; N-{2-Chloro-4-[(5-chloro-thiophen-2-ylmethyl)-amino]-phenyl}-2-(4-methoxyphenyl)-acetamide; N-{2-Chloro-4-[(5-chloro-thiophen-2-ylmethyl)-amino]-phenyl}-2-(4-fluorophenyl)-acetamide; 2,3-Dihydro-benzo[1,4]dioxine-6-carboxylic acid {2-chloro-4-[(5-chloro-thiophen-2-ylmethyl)-amino]-phenyl}-amide; 2,3-Dihydro-benzofuran-5-carboxylic acid {2-chloro-4-[(5-chloro-thiophen-2-ylmethyl)-amino]-phenyl}-amide; N-{2-Chloro-4-[(5-chloro-thiophen-2-ylmethyl)-amino]-phenyl}-3-cyclohexylpropionamide; N- {4-[(5-Chloro-thiophen-2-ylmethyl)-(methyl)amino]-2-methyl-phenyl}-2,2-dimethylpropionamide; N-{4-[(5-Chloro-thiophen-2-ylmethyl)-(methyl)amino]-2-methyl-phenyl} -2-phenylacetamide; N- {4-[(5-Chloro-thiophen-2-ylmethyl)-(methyl)amino]-2-methyl-phenyl}-3,3-dimethylbutyramide; N-{4-[(5-Chloro-thiophen-2-ylmethyl)-(methyl)amino]-2-methyl-phenyl}-3-phenylpropionamide; N-{4-[(5-Chloro-thiophen-2-ylmethyl)-(methyl)amino]-2-methyl-phenyl}-butyramide; 2,2,2-Trichloro-N-{4-[(5-chloro-thiophen-2-ylmethyl)-(methyl)amino]-2-methyl-phenyl}-acetamide; Cyclopropanecarboxylic acid {4-[(5-chloro-thiophen-2-ylmethyl)-(methyl)amino]-2-methyl-phenyl}-amide; Cyclobutanecarboxylic acid {4-[(5-chloro-thiophen-2-ylmethyl)-(methyl)amino]-2-methylphenyl}-amide; Cyclopentanecarboxylic acid {4-[(5-chloro-thiophen-2-ylmethyl)-(methyl)amino]-2-methylphenyl}-amide; Cyclohexanecarboxylic acid {4-[(5-chloro-thiophen-2-ylmethyl)-(methyl)amino]-2-methylphenyl}-amide; N-{4-[(5-Chloro-thiophen-2-ylmethyl)-(methyl)amino]-2-methylphenyl}-2-thiophen-2-yl-acetamide; N- {4-[(5-Chloro-thiophen-2-ylmethyl)-(methyl)amino]-2-methylphenyl}-2-(3-methoxyphenyl)-acetamide; N-{4-[(5-Chloro-thiophen-2-ylmethyl)-(methyl)amino]-2-methylphenyl}-malonamic acid methyl ester; 2-(4-Chlorophenyl)-N- {4-[(5-chloro-thiophen-2-ylmethyl)-(methyl)amino]-2-methylphenyl} -acetamide; N- {4-[(5-Chloro-thiophen-2-ylmethyl)-(methyl)amino]-2-methylphenyl}-2-(4-methoxyphenyl)-acetamide; N- {4-[(5-Chloro-thiophen-2-ylmethyl)-(methyl)amino]-2-methylphenyl}-2-(4-fluorophenyl)-acetamide; N-{4-[(5-Chloro-thiophen-2-ylmethyl)-(methyl)amino]-2-methylphenyl}-3-cyclohexylpropionamide; {2-Chloro-4-[(5-chloro-thiophen-2-ylmethyl)-(methyl)amino]-phenyl}-carbamic acid phenyl ester; {2-Chloro-4-[(5-chloro-thiophen-2-ylmethyl)-(methyl)amino]-phenyl}-carbamic acid benzyl ester; {2-Chloro-4-[(5-chloro-thiophen-2-ylmethyl)-(methyl)amino]-phenyl}-carbamic acid isobutyl ester; {2-Chloro-4-[(5-chloro-thiophen-2-ylmethyl)-(methyl)amino]-phenyl}-carbamic acid butyl ester; {2-Chloro-4-[(5-chloro-thiophen-2-ylmethyl)-(methyl)amino]-phenyl}-carbamic acid hexyl ester; {2-Chloro-4-[(5-chloro-thiophen-2-ylmethyl)-(methyl)amino]-phenyl}-carbamic acid 4-nitrobenzyl ester; {2-Chloro-4-[(5-chloro-thiophen-2-ylmethyl)-(methyl)amino]-phenyl}-carbamic acid but-3-enyl ester; {2-Chloro-4-[(5-chloro-thiophen-2-ylmethyl)-(methyl)amino]-phenyl}-carbamic acid but-2-ynyl ester; {2-Chloro-4-[(5-chloro-thiophen-2-ylmethyl)-(methyl)amino]-phenyl}-carbamic acid 2,2-dimethylpropyl ester; {2-Chloro-4-[(5-chloro-thiophen-2-ylmethyl)-(methyl)amino]-phenyl}-carbamic acid 2-chlorobenzyl ester; {2-Chloro-4-[(5-chloro-thiophen-2-ylmethyl)-(methyl)amino]-phenyl}-carbamic acid 3-chloropropyl ester; {2-Chloro-4-[(5-chloro-thiophen-2-ylmethyl)-(methyl)amino]-phenyl}-carbamic acid 2-benzyloxyethyl ester; 3-{2-Chloro-4-[(5-chloro-thiophen-2-ylmethyl)-(methyl)amino]-phenyl}-1-methyl-1-propyl-urea; 1-{2-Chloro-4-[(5-chloro-thiophen-2-ylmethyl)-(methyl)amino]-phenyl}-3-(2-fluorophenyl)-urea; N-{2-Chloro-4-[(5-chloro-thiophen-2-ylmethyl)-(methyl)amino]-phenyl}-2,2,2-trifluoroacetamide; N-{2-Chloro-4-[(5-chloro-thiophen-2-ylmethyl)-amino]-phenyl}-2,2,2-trifluoroacetamide;
N-{5-[(5-Chloro-thiophen-2-ylmethyl)-amino]-4'-dimethylamino-biphenyl-2-yl}-2-(4-fluorophenyl)-acetamide; N-{2-Chloro-4-[(5-chloro-thiophen-2-ylmethyl)-(methyl)amino]-phenyl}-2-(4-chlorophenyl)-acetamide; [4-(3-Fluoro-4-trifluoromethyl-benzylamino)-2-methylphenyl]-carbamic acid ethyl ester; 2-(4-Fluorophenyl)-N-{2-methyl-4-[(6-p-tolyloxypyridin-3-ylmethyl)-amino]-phenyl}-acetamide; N-[2-Methyl-4-(4-trifluoromethyl-benzylamino)-phenyl]-butyramide; 2-(4-Fluorophenyl)-N-{2-methyl-4-[(6-trifluoromethylpyridin-3-ylmethyl)-amino]-phenyl}-acetamide; Pentanoic acid {4-[(5-chloro-thiophen-2-ylmethyl)-(methyl)amino]-2-methylphenyl}-amide; 3,3-Dimethyl-N-{2-methyl-4-[(6-p-tolyloxypyridin-3-ylmethyl)-amino]-phenyl}-butyramide; [2-Methyl-4-(4-trifluoromethyl-benzylamino)-phenyl]-carbamic acid ethyl ester; N-{2-Chloro-4-[(5-chloro-thiophen-2-ylmethyl)-(methyl)amino]-phenyl}-2-(4-chlorophenyl)-propionamide; [4-(4-Chloro-benzylamino)-2-methylphenyl]-carbamic acid ethyl ester; {4-[(6-Methoxy-benzo[b]thiophen-2-ylmethyl)-amino]-2-methylphenyl}-carbamic acid propyl ester; {4-[(5-Chloro-thiophen-2-ylmethyl)-amino]-2-quinolin-3-yl-phenyl}-carbamic acid ethyl ester; {4-[(5-Dimethylamino-3-methyl-benzo[b]thiophen-2-ylmethyl)-amino]-2-methylphenyl}-carbamic acid propyl ester; 3,3-Dimethyl-N-{2-methyl-4-[(6-trifluoromethylpyridin-3-ylmethyl)-amino]-phenyl}-butyramide; N-(4-{[6-(4-Cyanophenoxy)-pyridin-3-ylmethyl]-amino}-2-methylphenyl)-2-(4-fluorophenyl)-acetamide; {2-Benzyloxy-4-[(4-fluorobenzyl)-(methyl)amino]-phenyl}-thiocarbamic acid S-ethyl ester; {2-Cyclopentyloxy-4-[(4-fluorobenzyl)-(methyl)amino]-phenyl}-thiocarbamic acid S-ethyl ester; N-{4-[(6-Chloropyridin-3-ylmethyl)-amino]-2-methylphenyl}-2-(4-fluorophenyl)-acetamide; {4-[(7-Dimethylamino-benzo[b]thiophen-2-ylmethyl)-amino]-2-methylphenyl}-carbamic acid propyl ester; 1-{2-Cyclopentyloxy-4-[(4-fluorobenzyl)-(methyl)amino]-phenyl}-3-ethyl-urea; 2-Amino-4-methyl-pentanoic acid [2-methyl-4-(4-trifluoromethyl-benzylamino)-phenyl]-amide; {4-[(6-Methoxy-benzo[b]thiophen-2-ylmethyl)-amino]-2-methylphenyl}-carbamic acid ethyl ester; 2-Amino-4-methyl-pentanoic acid [2-methyl-4-(4-trifluoromethyl-benzylamino)-phenyl]-amide; 2-(4-Fluorophenyl)-N- {2-methyl-4-[(4-methyl-2-phenylpyrimidin-5-ylmethyl)-amino]-phenyl}-acetamide; 3,3-Dimethyl-N-{2-methyl-4-[(2-phenylpyrimidin-5-ylmethyl)-amino]-phenyl}-butyramide; {4-[(5-Chloro-thiophen-2-ylmethyl)-amino]-2-pyridin-3-yl-phenyl}-carbamic acid ethyl ester; 1-Amino-cyclopropanecarboxylic acid [2-methyl-4-(4-trifluoromethyl-benzylamino)-phenyl]-amide; {4-[(5-Chloro-thiophen-2-ylmethyl)-amino]-2-pyridin-4-yl-phenyl}-carbamic acid ethyl ester; N-[2-Methyl-4-(4-trifluoromethyl-benzylamino)-phenyl]-2-piperidin-1-yl-acetamide; N-(4-{[5-(4-Chlorophenoxy)-1,3-dimethyl-1H-pyrazol-4-ylmethyl]-amino}-2-methylphenyl)-2,2-dimethylpropionamide; 2,2-Dimethyl-N-{2-methyl-4-[(6-phenoxypyridin-3-ylmethyl)-amino]-phenyl}-propionamide; N-[2-Methyl-4-(4-trifluoromethyl-benzylamino)-phenyl]-2-pyrrolidin-1-yl-acetamide; [4-[(5-Chloro-thiophen-2-ylmethyl)-amino]-2-(6-methoxypyridin-3-yl)-phenyl]-carbamic acid ethyl ester; 4-[(3-Methyl-4-propoxycarbonylamino-phenylamino)-methyl]-benzoic acid methyl ester; N-[2-Methyl-4-(4-trifluoromethyl-benzylamino)-phenyl]-2-morpholin-4-yl-acetamide;
2,2-Dimethyl-N-{2-methyl-4-[(3-methyl-5-phenylisoxazol-4-ylmethyl)-amino]-phenyl}-propionamide; {4-[(5-Chloro-thiophen-2-ylmethyl)-amino]-2-iodophenyl}-carbamic acid ethyl ester; N-{4-[(5-Chloro-thiophen-2-ylmethyl)-amino]-2-iodophenyl}-2-(4-fluorophenyl)-acetamide; and {4-[(5-Chloro-thiophen-2-ylmethyl)-aimno]-2-quinolin-5-yl-phenyl}-carbamic acid ethyl ester, or a pharmaceutically acceptable salt thereof.

In another embodiment, the invention relates a compound according to formula 3 wherein said compound is selected from the group of:
{2-Amino-4-[(4-tert-butylphenylamino)-methyl]-phenyl}-carbamic acid ethyl ester;
(2-Amino-4-phenylaminomethyl-phenyl)-carbamic acid ethyl ester; [2-Amino-4-(naphthalen-2-ylaminomethyl)-phenyl]-carbamic acid ethyl ester; [2-Amino-4-(p-tolylamino-methyl)-phenyl]-carbamic acid ethyl ester; {2-Amino-4-[(4-trifluoromethylphenylamino)-methyl]-phenyl}-carbamic acid ethyl ester; {2-Amino-4-[(4-chlorophenylamino)-methyl]-phenyl}-carbamic acid ethyl ester; {2-Amino-4-[(3-fluorophenylamino)-methyl]-phenyl}-carbamic acid ethyl ester; {2-Amino-4-[(4-fluorophenylamino)-methyl]-phenyl}-carbamic acid ethyl ester; {2-Amino-4-[(2-fluorophenylamino)-methyl]-phenyl}-carbamic acid ethyl ester; [2-Amino-4-(biphenyl-4-ylaminomethyl)-phenyl]-carbamic acid ethyl ester; {2-Amino-4-[(2,4-difluorophenylamino)-methyl]-phenyl}-carbamic acid ethyl ester; {2-Amino-4-[(4-methoxyphenylamino)-methyl]-phenyl}-carbamic acid ethyl ester; {2-Amino-4-[(4-cyclohexylphenylamino)-methyl]-phenyl}-carbamic acid ethyl ester; [2-Amino-4-(indan-5-ylaminomethyl)-phenyl]-carbamic acid ethyl ester; {2-Amino-4-[(4-isopropylphenylamino)-methyl]-phenyl}-carbamic acid ethyl ester; {2-Amino-4-[(4-butylphenylamino)-methyl]-phenyl}-carbamic acid ethyl ester; {2-Amino-4-[(4-chloro-3-fluorophenylamino)methyl]phenyl}carbamic acid ethyl ester; {2-Amino-4-[(2,4-dichlorophenylamino)methyl]phenyl}carbamic acid ethyl ester; {2-Amino-4-[(2,3-dichlorophenylamino)methyl]phenyl}carbamic acid ethyl ester; {2-Amino-4-[(3,5-dichlorophenylamino)methyl]phenyl}carbamic acid ethyl ester; {2-Amino-4-[(3,4-dichlorophenylamino)methyl]phenyl}carbamic acid ethyl ester; {2-Amino-4-[(3-trifluoromethylphenylamino)methyl]phenyl}carbamic acid ethyl ester; {2-Amino-4-[(3-fluoro-4-trifluoromethylphenylamino)methyl]phenyl}carbamic acid ethyl ester;
{2-Amino-4-[(3,4-difluorophenylamino)methyl]phenyl}carbamic acid ethyl ester; {2-Amino-4-[(4-cyanophenylamino)methyl]phenyl}carbamic acid ethyl ester; {2-Amino-4-[(4-fluoro-3-trifluoromethylphenylamino)methyl]phenyl}carbamic acid ethyl ester;
{2-Amino-4-[(3-chloro-4-methylphenylamino)methyl]phenyl}carbamic acid ethyl ester; {2-Amino-4-[(3-chlorophenylamino)methyl]phenyl}carbamic acid ethyl ester;
[2-Amino-4-(m-tolylaminomethyl)phenyl]carbamic acid ethyl ester; {2-Amino-4-[1-(4-chlorophenylamino)ethyl]phenyl}carbamic acid ethyl ester; {2-Amino-4-[1-(4-trifluoromethylphenylamino)ethyl]phenyl}carbamic acid ethyl ester; N-{2-Amino-4-[(3-fluorophenylamino)methyl]phenyl}-2,2-dimethylpropionamide; {4-[(4-Chlorophenylamino)methyl]phenyl}carbamic acid ethyl ester; {4-[(4-Trifluoromethylphenylamino)methyl]phenyl}carbamic acid ethyl ester; {4-[1-(4-Chlorophenylamino)ethyl]phenyl}carbamic acid ethyl ester; {4-[(4-Fluorophenylamino)methyl]-2-methylphenyl} carbamic acid ethyl ester; {4-[(4-Chlorophenylamino)methyl]-2-methylphenyl}carbamic acid ethyl ester; {2-Methyl-4-[(4-trifluoromethylphenylamino)methyl]phenyl} carbamic acid ethyl ester; {4-[(3,4-Difluorophenylamino)methyl]-2-methylphenyl}carbamic acid ethyl ester; {4-[(3-Fluorophenylamino)methyl]-2-methylphenyl}carbamic acid ethyl ester; {2-Chloro-4-[(4-chlorophenylamino)methyl]phenyl}carbamic acid ethyl ester; {2-Chloro-4-[(4-trifluoromethyl-phenylamino)-methyl]-phenyl}-carbamic acid ethyl ester; {2-Chloro-4-[(4-fluorophenylamino)methyl]phenyl}carbamic acid ethyl ester; {2-Chloro-4-[(3-fluorophenylamino)methyl]phenyl}carbamic acid ethyl ester; {2-Chloro-4-[(3,4-dichlorophenylamino)methyl]phenyl}carbamic acid ethyl ester; {2-Chloro-4-[(4-chloro-3-fluorophenylamino)methyl]phenyl}carbamic acid ethyl ester; {4-[(4-Chlorophenylamino)methyl]-2-fluorophenyl}carbamic acid ethyl ester; {4-[(4-Chloro-3-fluorophenylamino)methyl]-2-fluorophenyl}carbamic acid ethyl ester; {2-Fluoro-4-[(4-trifluoromethylphenylamino)methyl]phenyl}carbamic acid ethyl ester;
{4'-Dimethylamino-5-[(3-fluorophenylamino)methyl]biphenyl-2-yl}carbamic acid ethyl ester; {4'-Dimethylamino-5-[(4-trifluoromethylphenylamino)methyl]biphenyl-2-yl}carbamic acid ethyl ester; {4'-Chloro-5-[(3-fluorophenylamino)methyl]biphenyl-2-yl}carbamic acid ethyl ester; {4'-Chloro-5-[(4-trifluoromethylphenylamino)methyl]biphenyl-2-yl}carbamic acid ethyl ester; N-{4-[(4-chlorophenylamino)methyl]phenyl}butyramide; N-{4-[(3,4-dichlorophenylamino)methyl]phenyl}butyramide; N-{4-[(4-chloro-3-fluorophenylamino)methyl]phenyl}butyramide; N-{4[(4-fluoro-phenylamino)methyl]-2-methylphenyl}butyramide; N-{4[(3-fluorophenylamino)methyl]-2-methylphenyl}butyramide; N-{4-[(4-chlorophenylamino)methyl]-2-methylphenyl}butyramide; N-{4-[(3,4-dichlorophenylamino)methyl]-2-methylphenyl}butyramide; N-{4-[(4-chloro-3-fluorophenylamino)methyl]-2-methylphenyl}butyramide; N-{2-chloro-4-[(4-trifluoromethylphenylamino)methyl]phenyl}butyramide; N-{2-chloro-4-[(4-fluorophenylamino)methyl]phenyl}butyramide; N-{2-chloro-4-[(3-fluorophenylamino)methyl]phenyl}butyramide; N-{2-chloro-4-[(4-chlorophenylamino)methyl]phenyl}butyramide; N-{2-chloro-4-[(3,4-dichlorophenylamino)methyl]phenyl}butyramide; N-{2-chloro-4-[(4-chloro-3-fluorophenylamino)methyl]phenyl}butyramide; N-{2-fluoro-4-[(3-fluorophenylamino)methyl]phenyl}butyramide; N-{4-[(4-chlorophenylamino)methyl]-2-fluorophenyl}butyramide; N-{2-fluoro-4-[(4-trifluoromethylphenylamino)methyl]phenyl}butyramide; N-{4-[(3,4-dichlorophenylamino)methyl]-2-fluorophenyl}butyramide; and N- {4-[(4-chloro-3-fluorophenylamino)methyl]-2-fluorophenyl}butyramide or a pharmaceutically acceptable salt thereof.

In another embodiment, the invention relates a compound according to formula 4 wherein said compound is selected from the group of:
N-[4-Chloro-1-(4-trifluoromethylbenzyl)-2,3-dihydro-1H-indol-5-yl]-3,3-dimethylbutyramide; N-[4-Chloro-1-(5-chlorothiophen-2-ylmethyl)-2,3-dihydro-1H-indol-5-yl]-3,3-dimethylbutyramide; [1-(4-Fluorobenzyl)-2,3-dihydro-1H-indol-5-yl]-carbamic acid propyl ester; N-[1-(4-Fluorobenzyl)-2,3-dihydro-1H-indol-5-yl]-C-phenyl-methanesulfonamide; 4-Fluoro-N-[1-(4-fluorobenzyl)-2,3-dihydro-1H-indol-5-yl]-benzamide; N-[1-(4-Fluorobenzyl)-2,3-dihydro-1H-indol-5-yl]-3,3-dimethylbutyramide; N-[1-(4-Fluorobenzyl)-2,3-dihydro-1H-indol-5-yl]-2-thiophen-2-ylacetamide; N-[1-(4-Fluorobenzyl)-2,3-dihydro-1H-indol-5-yl]-2-(4-fluorophenyl)-acetamide; 3-[1-(5-Chlorothiophen-2-ylmethyl)-2,3-dihydro-1H-indol-5-yl]-1,1-diisopropylurea; Morpholine-4-carboxylic acid [1-(5-chlorothiophen-2-ylmethyl)-2,3-dihydro-1H-indol-5-yl]-amide; Pyrrolidine-1-carboxylic acid [1-(5-chlorothiophen-2-ylmethyl)-2,3-dihydro-1H-indol-5-yl]-amide; [1-(5-Chlorothiophen-2-ylmethyl)-2,3-dihydro-1H-indol-5-yl]-carbamic acid 2-benzyloxyethyl ester; 3-[1-(5-Chlorothiophen-2-ylmethyl)-2,3-dihydro-1H-indol-5-yl]-1-methyl-1-propylurea; [1-(5-Chlorothiophen-2-ylmethyl)-2,3-dihydro-1H-indol-5-yl]-carbamic acid tert-butyl ester; N-[1-(5-Chlorothiophen-2-ylmethyl)-2,3-dihydro-1H-indol-5-yl]-C-phenyl-methanesulfonamide; Butane-1-sulfonic acid [1-(5-chlorothiophen-2-ylmethyl)-2,3-dihydro-1H-indol-5-yl]-amide; N-[1-(5-Chlorothiophen-2-ylmethyl)-2,3-dihydro-1H-indol-5-yl]-4-fluorobenzamide; N-[1-(5-Chlorothiophen-2-ylmethyl)-2,3-dihydro-1H-indol-5-yl]-2,2-dimethylpropionamide;
N-[1-(5-Chlorothiophen-2-ylmethyl)-2,3-dihydro-1H-indol-5-yl]-2-phenoxyacetamide; N-[1-(5-Chlorothiophen-2-ylmethyl)-2,3-dihydro-1H-indol-5-yl]-3,3-dimethylbutyramide; N-[1-(5-Chlorothiophen-2-ylmethyl)-2,3-dihydro-1H-indol-5-yl]-butyramide; Cyclopentanecarboxylic acid [1-(5-chlorothiophen-2-ylmethyl)-2,3-dihydro-1H-indol-5-yl]-amide; N-[1-(5-Chlorothiophen-2-ylmethyl)-2,3-dihydro-1H-indol-5-yl]-2-thiophen-2-ylacetamide; N-[1-(5-Chlorothiophen-2-ylmethyl)-2,3-dihydro-1H-indol-5-yl]-isonicotinamide; N-[1-(5-Chlorothiophen-2-ylmethyl)-2,3-dihydro-1H-indol-5-yl]-4-dimethylaminobenzamide; N-[1-(5-Chlorothiophen-2-ylmethyl)-2,3-dihydro-1H-indol-5-yl]-2-(4-fluorophenyl)-acetamide; N-[1-(5-Chlorothiophen-2-ylmethyl)-2,3-dihydro-1H-indol-5-yl]-6-trifluoromethylnicotinamide; 1-tert-Butyl-3-[1-(5-chlorothiophen-2-ylmethyl)-2,3-dihydro-1H-indol-5-yl]-urea; 1-[1-(5-Chlorothiophen-2-ylmethyl)-2,3-dihydro-1H-indol-5-yl]-3-ethylurea; 1-Benzyl-3-[1-(5-chlorothiophen-2-ylmethyl)-2,3-dihydro-1H-indol-5-yl]-urea; 1-[1-(5-Chlorothiophen-2-ylmethyl)-2,3-dihydro-1H-indol-5-yl]-3-phenethylurea; 1-[1-(5-Chlorothiophen-2-ylmethyl)-2,3-dihydro-1H-indol-5-yl]-3-thiophen-2-ylurea; 1-[1-(5-Chlorothiophen-2-ylmethyl)-2,3-dihydro-1H-indol-5-yl]-3-thiophen-3-ylurea; [1-(5-Chlorothiophen-2-ylmethyl)-2,3-dihydro-1H-indol-5-yl]-carbamic acid propyl ester; 2,2-Dimethyl-N-[6-nitro-1-(4-trifluoromethylbenzyl)-2,3-dihydro-1H-indol-5-yl]-propionamide; N-[1-(5-Chlorothiophen-2-ylmethyl)-6-nitro-2,3-dihydro-1H-indol-5-yl]-2,2-dimethylpropionamide; 2-(4-Fluorophenyl)-N-[6-nitro-1-(4-trifluoromethylbenzyl)-2,3-dihydro-1H-indol-5-yl]-acetamide; N-[1-(5-Chlorothiophen-2-ylmethyl)-6-nitro-2,3-dihydro-1H-indol-5-yl]-2-(4-fluorophenyl)-acetamide; N-[1-(5-Chlorothiophen-2-ylmethyl)-6-nitro-2,3-dihydro-1H-indol-5-yl]-3,3-dimethylbutyramide; N-[6-Amino-1-(5-chlorothiophen-2-ylmethyl)-2,3-dihydro-1H-indol-5-yl]-3,3-dimethylbutyramide; N-[6-Amino-1-(4-trifluoromethylbenzyl)-2,3-dihydro-1H-indol-5-yl]-2,2-dimethylpropionamide; N-[6-Amino-1-(5-chlorothiophen-2-ylmethyl)-2,3-dihydro-1H-indol-5-yl]-2,2-dimethylpropionamide;
N-[6-Amino-1-(4-trifluoromethylbenzyl)-2,3-dihydro-1H-indol-5-yl]-2-(4-fluorophenyl)-acetamide; N-[6-Amino-1-(4-trifluoromethylbenzyl)-2,3-dihydro-1H-indol-5-yl]-3,3-dimethylbutyramide; N-[6-Amino-1-(4-fluorobenzyl)-2,3-dihydro-1H-indol-5-yl]-3,3-dimethylbutyramide; N-[6-Amino-1-(3-fluoro-4-trifluoromethylbenzyl)-2,3-dihydro-1H-indol-5-yl]-3,3-dimethylbutyramide; N-[1-(5-Chlorothiophen-2-ylmethyl)-1H-indol-5-yl]-3,3-dimethylbutyramide; N-[6-Bromo-1-(4-trifluoromethylbenzyl)-2,3-dihydro-1H-indol-5-yl]-3,3-dimethylbutyramide; N-[6-Bromo-1-(5-chlorothiophen-2-ylmethyl)-2,3-dihydro-1H-indol-5-yl]-3,3-dimethylbutyramide; N-[1-(4-Chlorobenzyl)-2,3-dihydro-1H-indol-5-yl]-3,3-dimethylbutyramide; 3,3-Dimethyl-N-[1-(4-trifluoromethylbenzyl)-2,3-dihydro-1H-indol-5-yl]-butyramide; N-[1-(4-Isopropylbenzyl)-2,3-dihydro-1H-indol-5-yl]-3,3-dimethylbutyramide; N-[1-(3-Fluoro-4-trifluoromethylbenzyl)-2,3-dihydro-1H-indol-5-yl]-3,3-dimethylbutyramide; N-[1-(6-Chlorobenzo[1,3]dioxol-5-ylmethyl)-2,3-dihydro-1H-indol-5-yl]-3,3-dimethylbutyramide; N-[1-(3,5-Dimethyl-1-phenyl-1H-pyrazol-4-ylmethyl)-2,3-dihydro-1H-indol-5-yl]-3,3-dimethylbutyramide; N-[1-(2-Chloro-5-trifluoromethylbenzyl)-2,3-dihydro-1H-indol-5-yl]-3,3-dimethylbutyramide;
N-{1-[5-(4-Chlorophenoxy)-1,3-dimethyl-1H-pyrazol-4-ylmethyl]-2,3-dihydro-1H-indol-5-yl}-3,3-dimethylbutyramide; 3,3-Dimethyl-N-[1-(6-p-tolyloxy-pyridin-3-ylmethyl)-2,3-dihydro-1H-indol-5-yl]-butyramide; N-{1-[6-(4-Chlorophenylsulfanyl)-pyridin-3-ylmethyl]-2,3-dihydro-1H-indol-5-yl}-3,3-dimethylbutyramide; N-{1-[6-(4-Cyanophenoxy)-pyridin-3-ylmethyl]-2,3-dihydro-1H-indol-5-yl}-3,3-dimethylbutyrmnide; 3,3-Dimethyl-N-[1-(6-trifluoromethylpyridin-3-ylmethyl)-2,3-dihydro-1H-indol-S-yl]-butyramide; 3,3-Dimethyl-N-[1-(3-1-methyl-benzo[b]thiophen-2-ylmethyl)-2,3-dihydro-1H-indol-5-yl]-butyramide; N-[1-(6-Fluoro-4H-benzo[1,3]dioxin-8-ylmethyl)-2,3-dihydro-1H-indol-5-yl]-3,3-dimethylbutyramide; 3,3-Dimethyl-N-[1-(6-phenoxypyridin-3-ylmethyl)-2,3-dihydro-1H-indol-5-yl]-butyramide; 3,3-Dimethyl-N-[1-(3-methyl-5-phenyl-isoxazol-4-ylmethyl)-2,3-dihydro-1H-indol-5-yl]-butyramide; N-(1-Benzo[b]thiophen-2-yhnethyl-2,3-dihydro-1H-indol-5-yl)-3,3-dimethylbutyramide;
N-{1-[1-(4-Fluorophenyl)-5-methyl-1H-pyrazol-4-ylmethyl]-2,3-dihydro-1H-indol-5-yl}-3,3-dimethylbutyramide; 3,3-Dimethyl-N-[1-(5-methylthiophen-2-ylmethyl)-2,3-dihydro-1H-indol-5-yl]-butyramide; 3,3-Dimethyl-N-[1-(4-pyrrol-1-yl-benzyl)-2,3-dihydro-1H-indol-5-yl]-butyramide; N-[1-(4-Chlorobenzyl)-2,3-dihydro-1H-indol-5-yl]-2-(4-fluorophenyl)-acetamide; 2-(4-Fluorophenyl)-N-[1-(4-trifluoromethylbenzyl)-2,3-dihydro-1H-indol-5-yl]-acetamide; 2-(4-Fluorophenyl)-N-[1-(4-isopropylbenzyl)-2,3-dihydro-1H-indol-5-yl]-acetamide; 2-(4-Fluorophenyl)-N-[1-(3-fluoro-4-trifluoromethylbenzyl)-2,3-dihydro-1H-indol-5-yl]-acetamide; N-[1-(6-Chlorobenzo[1,3]dioxol-5-ylmethyl)-2,3-dihydro-1H-indol-5-yl]-2-(4-fluorophenyl)-acetamide; N-[1-(3,5-Dimethyl-1-phenyl-1H-pyrazol-4-ylmethyl)-2,3-dihydro-1H-indol-5-yl]-2-(4-fluorophenyl)-acetamide; N-[1-(2-Chloro-5-trifluoromethylbenzyl)-2,3-dihydro-1H-indol-5-yl]-2-(4-fluorophenyl)-acetamide; N-{1-[5-(4-Chlorophenoxy)-1,3-dimethyl-1H-pyrazol-4-ylmethyl]-2,3-dihydro-1H-indol-5-yl}-2-(4-fluorophenyl)-acetamide; N-{1-[6-(4-Cyanophenoxy)-pyridin-3-ylmethyl]-2,3-dihydro-1H-indol-5-yl}-2-(4-fluorophenyl)-acetamide; 2-(4-Fluorophenyl)-N-[1-(3-methyl-benzo[b]thiophen-2-ylmethyl)-2,3-dihydro-1H-indol-5-yl]-acetamide; N-[1-(6-Fluoro-4H-benzo[1,3]dioxin-8-ylmethyl)-2,3-dihydro-1H-indol-5-yl]-2-(4-fluorophenyl)-acetamide; 2-(4-Fluorophenyl)-N-[1-(6-phenoxypyridin-3-ylmethyl)-2,3-dihydro-1H-indol-5-yl]-acetamide; N-(1-Benzo[b]thiophen-2-ylmethyl-2,3-dihydro-1H-indol-5-yl)-2-(4-fluorophenyl)-acetamide; 2-(4-Fluorophenyl)-N-{1-[1-(4-fluorophenyl)-5-methyl-1H-pyrazol-4-ylmethyl]-2,3-dihydro-1H-indol-5-yl}-acetamide; 2-(4-Fluorophenyl)-N-[1-(5-methylthiophen-2-ylmethyl)-2,3-dihydro-1H-indol-5-yl]-acetamide; and 2-(4-Fluorophenyl)-N-[1-(4-pyrrol-1-yl-benzyl)-2,3-dihydro-1H-indol-5-yl]-acetamide, or a pharmaceutically acceptable salt thereof.

In another embodiment, the invention relates a compound according to formula 5 wherein said compound is selected from the group of:
N-(2-Bromo-4-morpholin-4-yl-6-trifluoromethyl-phenyl)-2-(4-fluoro-phenyl)-acetamide; 2-Cyclopentyl-N-(2-bromo-6-trifluoromethyl-4-morpholin-4-yl-phenyl)-acetamide; N-(2-Bromo-4-morpholin-4-yl-6-trifluoromethyl-phenyl)-3-cyclopentyl-propionamide; N-(2-Chloro-6-cyano-4-morpholin-4-yl-phenyl)-3-cyclohexyl-propionamide; 2-Cyclopentyl-N-(2,6-dimethyl-4-thiomorpholin-4-yl-phenyl)-acetamide; 2-Cyclopentyl-N-[2,6-dimethyl-4-(2-phenyl-morpholin-4-yl)-phenyl]-acetamide; 2-Cyclopentyl-N-[2,6-dimethyl-4-(2-phenyl-thiomorpholin-4-yl)-phenyl]-acetamide; 2-Cyclopentyl-N-[2,6-dimethyl-4-(3-pyridin-3-yl-thiomorpholin-4-yl)-phenyl]-acetamide; 2-Cyclopentyl-N-{2,6-dimethyl-4-[2-(4-trifluoromethyl-phenyl)-thiomorpholin-4-yl]-phenyl}-acetamide; N-{4-[2-(2-Chloro-phenyl)-thiomorpholin-4-yl]-2,6-dimethyl-phenyl}-2-cyclopentyl-acetamide; 2-Bicyclo[2.2.1]hept-2-yl-N-(2,6-dimethyl-4-morpholin-4-yl-phenyl)-acetamide; 2-Cyclohexyl-N-(2,6-dimethyl-4-morpholin-4-yl-phenyl)-acetamide; 3-(3,4-Difluoro-phenyl)-N-(2,6-dimethyl-4-morpholin-4-yl-phenyl)-propionamide; 2-Cyclopentyl-N-(2,6-dimethyl-4-morpholin-4-yl-phenyl)-acetamide; (2,6-Dimethyl-4-morpholin-4-yl-phenyl)-carbamic acid butyl ester; 2-(4-Chloro-phenyl)-N-(2,6-dimethyl-4-morpholin-4-yl-phenyl)-acetamide; 2,3-Dihydro-benzofuran-2-carboxylic acid (2,6-dimethyl-4-morpholin-4-yl-phenyl)-amide; 3-Cyclohexyl-N-(2,6-dimethyl-4-morpholin-4-yl-phenyl)-propionamide; 3-Cyclopentyl-N-(2,6-dimethyl-4-morpholin-4-yl-phenyl)-propionamide;N-(2,6-Dimethyl-4-morpholin-4-yl-phenyl)-2-(4-fluoro-phenyl)-acetamide; N-(2,6-Dimethyl-4-morpholin-4-yl-phenyl)-2-thiophen-2-yl-acetamide; N-(2,6-Dimethyl-4-morpholin-4-yl-phenyl)-3,3-dimethyl-butyramide; Hexanoic acid (2,6-dimethyl-4-morpholin-4-yl-phenyl)-amide; 2-Cycloheptyl-N-(2,6-dimethyl-4-morpholin-4-yl-phenyl)-acetamide; (2,6-Dimethyl-4-morpholin-4-yl-phenyl)-carbamic acid benzyl ester; (2,6-Dimethyl-4-morpholin-4-yl-phenyl)-carbamic acid 2-chloro-benzyl ester; 3,5,5-Trimethyl-hexanoic acid (2,6-dimethyl-4-morpholin-4-yl-phenyl)-amide; Octanoic acid (2,6-dimethyl-4-morpholin-4-yl-phenyl)-amide; Heptanoic acid (2,6-dimethyl-4-morpholin-4-yl-phenyl)-amide; N-(2,6-Dimethyl-4-morpholin-4-yl-phenyl)-2-phenyl-acetamide; 2-(3,4-Dichloro-phenyl)-N-(2,6-dimethyl-4-morpholin-4-yl-phenyl)-acetamide; 2-(4-Allyloxy-3-chloro-phenyl)-N-(2,6-dimethyl-4-morpholin-4-yl-phenyl)-acetamide; N-(2,6-Dimethyl-4-morpholin-4-yl-phenyl)-2-(3-trifluoromethylphenyl)-acetamide; N-(2,6-Dimethyl-4-morpholin-4-yl-phenyl)-2-naphthalen-2-yl-acetamide; 3-(3-Chloro-phenyl)-N-(2,6-dimethyl-4-morpholin-4-yl-phenyl)-propionamide; N-(2,6-Dimethyl-4-morpholin-4-yl-phenyl)-2-(3,4-dimethyl-phenyl)-acetamide; 2-(3-Bromo-phenyl)-N-(2,6-dimethyl-4-morpholin-4-yl-phenyl)-acetamide; 2-(3-Chloro-phenyl)-N-(2,6-dimethyl-4-morpholin-4-yl-phenyl)-acetamide; N-(2,6-Dimethyl-4-morpholin-4-yl-phenyl)-2-p-tolyl-acetamide; N-(2,6-Dimethyl-4-morpholin-4-yl-phenyl)-2-m-tolyl-acetamide; 2-(3,4-Difluoro-phenyl)-N-(2,6-dimethyl-4-morpholin-4-yl-phenyl)-acetamide; N-(2,6-Dimethyl-4-morpholin-4-yl-phenyl)-2-(3-fluoro-phenyl)-acetamide; N-(2-Bromo-4-morpholin-4-yl-6-trifluoromethyl-phenyl)-3-cyclohexyl-propionamide; N-(2-Bromo-4-morpholin-4-yl-6-trifluoromethyl-phenyl)-2-(3-fluoro-phenyl)-acetamide; N-(2-Bromo-4-morpholin-4-yl-6-trifluoromethyl-phenyl)-propionamide; N-(2-Bromo-4-morpholin-4-yl-6-trifluoromethyl-phenyl)-butyramide; N-(2-Chloro-4-morpholin-4-yl-6-trifluoromethyl-phenyl)-2-(3-fluoro-phenyl)-acetamide; N-(2-Chloro-4-morpholin-4-yl-6-trifluoromethyl-phenyl)-2-cyclopentyl-acetamide; 2-Cyclopentyl-N-{2,6-dimethyl-4-[2-(4-trifluoromethyl-phenyl)-morpholin-4-yl]-phenyl}-acetamide; N-{4-[2-(2-Chloro-phenyl)-morpholin-4-yl]-2,6-dimethyl-phenyl}-2-cyclopentyl-acetamide; 2-Cyclopentyl-N-{4-[2-(4-fluoro-phenyl)-morpholin-4-yl]-2,6-dimethylphenyl}-acetamide; 2-(2-Chloro-phenyl)-N-(2,6-dimethyl-4-morpholin-4-yl-phenyl)-acetamide; Pentanoic acid (2,6-dimethyl-4-morpholin-4-yl-phenyl)-amide; 4-Methyl-pentanoic acid (2,6-dimethyl-4-morpholin-4-yl-phenyl)-amide; 2-Cyclopent-2-enyl-N-(2,6-dimethyl-4-morpholin-4-yl-phenyl)-acetamide; 5-Methyl-hexanoic acid (2,6-dimethyl-4-morpholin-4-yl-phenyl)-amide; 3-Methyl-pentanoic acid (2,6-dimethyl-4-morpholin-4-yl-phenyl)-amide; Hex-5-enoic acid (2,6-dimethyl-4-morpholin-4-yl-phenyl)-amide; 3-Ethyl-pentanoic acid (2,6-dimethyl-4-morpholin-4-yl-phenyl)-amide; 2-Cyclopentyl-N-(4-morpholin-4-yl-2-pyridin-3-yl-6-trifluoromethyl-phenyl)-acetamide; 2-Cyclopentyl-N-(5-morpholin-4-yl-3-trifluoromethyl-biphenyl-2-yl)-acetamide; 2-Cyclopentyl-N-(4'-fluoro-5-morpholin-4-yl-3-trifluoromethyl-biphenyl-2-yl)-acetamide; 2-Cyclopentyl-N-(4'-methyl-5-morpholin-4-yl-3-trifluoromethyl-biphenyl-2-yl)-acetamide; 2-Cyclopentyl-N-(3'-methyl-5-morpholin-4-yl-3-trifluoromethyl-biphenyl-2-yl)-acetamide; 2-Cyclopentyl-N-(3',4'-difluoro-5-morpholin-4-yl-3-trifluoromethyl-biphenyl-2-yl)-acetamide; 2-(4-Fluoro-phenyl)-N-(4-morpholin-4-yl-2-pyridin-3-yl-6-trifluoromethyl-phenyl)-acetamide; 2-Cyclopentyl-N-(2,6-diethyl-4-morpholin-4-yl-phenyl)-acetamide; 2-Cyclopentyl-N-(2,6-diisopropyl-4-morpholin-4-yl-phenyl)-acetamide; 2-Cyclopentyl-N-(2,6-difluoro-4-morpholin-4-yl-phenyl)-acetamide; Hexanoic acid (2,6-difluoro-4-morpholin-4-yl-phenyl)-amide; N-(2,6-Difluoro-4-morpholin-4-yl-phenyl)-3,3-dimethyl-butyramide;
N-(2,6-Difluoro-4-morpholin-4-yl-phenyl)-2-(3-fluoro-phenyl)-acetamide; 2-Cyclopent-2-enyl-N-(2,6-difluoro-4-morpholin-4-yl-phenyl)-acetamide; 2-Bicyclo[2.2.1]hept-2-yl-N-(2,6-difluoro-4-morpholin-4-yl-phenyl)-acetamide; 2-Bicyclo[2.2.1]hept-2-yl-N-(2-methyl-4-morpholin-4-yl-6-trifluoromethyl-phenyl)-acetamide; 5-Methyl-pentanoic acid (2-methyl-4-morpholin-4-yl-6-trifluoromethylphenyl)-amide; 5-Methyl-hexanoic acid (2-methyl-4-morpholin-4-yl-6-trifluoromethyl-phenyl)-amide; 2-Cyclopent-2-enyl-N-(2-methyl-4-morpholin-4-yl-6-trifluoromethyl-phenyl)-acetamide; 2-Cyclopentyl-N-(2-methyl-4-morpholin-4-yl-6-trifluoromethyl-phenyl)-acetamide; Hexanoic acid (2-methyl-4-morpholin-4-yl-6-trifluoromethyl-phenyl)-amide; 3,3-Dimethyl-N-(2-methyl-4-morpholin-4-yl-6-trifluoromethyl-phenyl)-butyramide; 2-(3,4-Difluoro-phenyl)-N-(2-methyl-4-morpholin-4-yl-6-trifluoromethyl-phenyl)-acetamide; Hexanoic acid (2-methoxy-6-methyl-4-morpholin-4-yl-phenyl)-amide; 2-Cyclopentyl-N-(2-methoxy-6-methyl-4-morpholin-4-yl-phenyl)-acetamide; N-(2-Methoxy-6-methyl-4-morpholin-4-yl-phenyl)-3,3-dimethyl-butyramide; 2-(3,4-Difluoro-phenyl)-N-(2-methoxy-6-methyl-4-morpholin-4-yl-phenyl)-acetamide; 2-Cyclopent-2-enyl-N-(2-methoxy-6-methyl-4-morpholin-4-yl-phenyl)-acetamide; 2-(3-Fluoro-phenyl)-N-(2-methoxy-6-methyl-4-morpholin-4-yl-phenyl)-acetamide; 2-Bicyclo[2.2.1]hept-2-yl-N-(2-methoxy-6-methyl-4-morpholin-4-yl-phenyl)-acetamide; 4-Methyl-pentanoic acid (2-methoxy-6-methyl-4-morpholin-4-yl-phenyl)-amide; 5-Methyl-hexanoic acid (2-methoxy-6-methyl-4-morpholin-4-yl-phenyl)-amide; N-(2-Chloro-6-methyl-4-morpholin-4-yl-phenyl)-2-(3-fluoro-phenyl)-acetamide; and N-(2-Chloro-6-methyl-4-morpholin-4-yl-phenyl)-2-cyclopentyl-acetamide, or a pharmaceutically acceptable salt thereof.

In another embodiment, the invention relates a compound according to formula 6 wherein said compound is selected from the group of:
Hexanoic acid (4-bromo-2,6-dimethyl-phenyl)-amide, N-(4-Bromo-2,6-dimethylphenyl)-2-(4-fluoro-phenyl)-acetamide, N-(2-Bromo-4,6-dimethyl-phenyl)-2-(4-fluoro-phenyl)-acetamide, N-(2-Bromo-4,6-dimethyl-phenyl)-3,3-dimethylbutyramide, N-(2-Bromo-4,6-dimethyl-phenyl)-2-cyclopentyl-acetamide, N-(2-Bromo-4,6-dichloro-phenyl)-3,3-dimethyl-butyramide, N-(2-Bromo-4,6-dichlorophenyl)-2-(4-fluoro-phenyl)-acetamide,
N-(2-Bromo-4,6-dichloro-phenyl)-2-cyclopentyl-acetamide, Heptanoic acid (4-bromo-2,6-dimethyl-phenyl)-amide, Cyclohexanecarboxylic acid (4-bromo-2,6-dimethyl-phenyl)-amide,
N-(4-Bromo-2,6-dimethyl-phenyl)-2-thiophen-2-yl-acetamide, 2-Phenyl-cyclopropanecarboxylic acid (4-bromo-2,6-dimethyl-phenyl)-amide, N-(4-Bromo-2,6-dimethyl-phenyl)-2-(4-chloro-phenyl)-acetamide, Pentanoic acid (4-bromo-2,6-dimethyl-phenyl)-amide, Octanoic acid (4-bromo-2,6-dimethyl-phenyl)-amide, N-(4-Bromo-2,6-dimethyl-phenyl)-2-cyclopentyl-acetamide, 2-Bicyclo[2.2.1]hept-2-yl-N-(2,4-difluoro-6-morpholin-4-yl-phenyl)-acetamide, (S)-2-Amino-N-{2,6-dimethyl-4-[methyl-(4-trifluoromethyl-benzyl)-amino]-phenyl}-3-methyl-butyramide, (S)-2-Amino-4-methyl-pentanoic acid {2,6-dimethyl-4-[methyl-(4-trifluoromethyl-benzyl)-amino]-phenyl}-amide, (4-Bromo-2,6-dimethyl-phenyl)-carbamic acid ethyl ester, (4-Bromo-2,6-dimethyl-phenyl)-carbamic acid propyl ester, N-(2-Amino-4-bromo-6-methyl-phenyl)-3,3-dimethyl-butyramide, 2-Cyclopentyl-N-{2,6-dimethyl-4-[2-(4-trifluoromethyl-phenyl)-pyrrolidin-1-yl]-phenyl}-acetamide, N-(4-Azepan-1-yl-2,6-dimethyl-phenyl)-2-cyclopentyl-acetamide,
2-Cyclopentyl-N-(2,6-dimethyl-4-pyrrol-1-yl-phenyl)-acetamide, N-(3'-Amino-3,5-dimethyl-biphenyl-2-yl)-2-(4-fluoro-phenyl)-acetamide, N-(4'-Dimethylamino-3,5-dimethyl-biphenyl-2-yl)-2-(4-fluoro-phenyl)-acetamide, N-(2,4-Dimethyl-6-quinolin-3-yl-phenyl)-2-(4-fluoro-phenyl)-acetamide, 2-(4-Fluoro-phenyl)-N-(4'-hydroxy-3'-methoxy-3,5-dimethyl-biphenyl-2-yl)-acetamide, 2-(4-Fluoro-phenyl)-N-(3'-hydroxy-3,5-dimethyl-biphenyl-2-yl)-acetamide, 2-(4-Fluoro-phenyl)-N-(2'-methanesulfonylamino-3,5-dimethyl-biphenyl-2-yl)-acetamide, N-(4'-Isopropyl-3,5-dimethyl-biphenyl-2-yl)-3,3-dimethyl-butyramide, 2-Cyclopentyl-N-(3,5-dimethylbiphenyl-2-yl)-acetamide, N-(4'-Fluoro-3,5-dimethyl-biphenyl-2-yl)-2-(4-fluorophenyl)-acetamide, N-(3,5-Dimethyl-3',5'-bis-trifluoromethyl-biphenyl-2-yl)-2-(4-fluoro-phenyl)-acetamide, N-(3'-Acetylamino-3,5-dimethyl-biphenyl-2-yl)-2-(4-fluoro-phenyl)-acetamide, 2-(4-Fluoro-phenyl)-N-(2'-methoxy-3,5-dimethylbiphenyl-2-yl)-acetamide, N-(3,5-Dimethyl-4'-vinyl-biphenyl-2-yl)-2-(4-fluorophenyl)-acetamide, N-(3'-Cyano-3,5-dimethyl-biphenyl-2-yl)-2-(4-fluoro-phenyl)-acetamide, N-(3,5-Dimethyl-3'-trifluoromethoxy-biphenyl-2-yl)-2-(4-fluoro-phenyl)-acetamide, N-[2-(2,3-Dihydro-benzo[1,4]dioxin-6-yl)-4,6-dimethyl-phenyl]-2-(4-fluoro-phenyl)-acetamide, N-[2,4-Dimethyl-6-(2,2,5-trimethyl-2,3-dihydro-benzofuran-7-yl)-phenyl]-2-(4-fluoro-phenyl)-acetamide, N-[2,6-Dimethyl-4-(4-trifluoromethyl-benzylamino)-phenyl]-acetamide, N-{2,6-Dimethyl-4-[methyl-(4-trifluoromethyl-benzyl)-amino]-phenyl}-acetamide, {4-[(5-Chloro-thiophen-2-ylmethyl)-amino]-2,6-dimethyl-phenyl}-carbamic acid propyl ester, [4-(4-Fluoro-benzylamino)-2,6-dimethyl-phenyl]-carbamic acid propyl ester, [2,6-Dimethyl-4-(4-trifluoromethyl-benzylamino)-phenyl]-carbamic acid propyl ester, [4-(3-Fluoro-4-trifluoromethyl-benzylamino)-2,6-dimethyl-phenyl]-carbamic acid propyl ester, {2,6-Dimethyl-4-[(4-methyl-2-phenyl-pyrimidin-5-ylmethyl)-amino]-phenyl}-carbamic acid propyl ester, {2,6-Dimethyl-4-[(6-p-tolyloxy-pyridin-3-ylmethyl)-amino]-phenyl}-carbamic acid propyl ester, {4-[(6-Methoxy-pyridin-3-ylmethyl)-amino]-2,6-dimethyl-phenyl}-carbamic acid propyl ester, {4-[(3-Fluoro-4-trifluoromethylbenzyl)-methyl-amino]-2,6-dimethyl-phenyl}-carbamic acid propyl ester, 2-Cyclopentyl-N-[2,6-dimethyl-4-(4-trifluoromethyl-benzylamino)-phenyl]-acetamide, 2-Cyclopentyl-N- {2,6-dimethyl-4-[methyl-(4-trifluoromethyl-benzyl)-amino]-phenyl}-acetamide, 2-Cyclopentyl-N-{2,6-dimethyl-4-[(6-trifluoromethyl-pyridin-3-ylmethyl)-amino]-phenyl}-acetamide, N- {2,6-Dimethyl-4-[(6-trifluoromethyl-pyridin-3-ylmethyl)-amino]-phenyl}-3,3-dimethyl-butyramide, N-{2-Bromo-4-[(5-chloro-thiophen-2-ylmethyl)-amino]-6-trifluoromethyl-phenyl}-3-cyclohexylpropionamide, {4-[(3-Fluoro-phenylamino)-methyl]-2,6-dimethyl-phenyl}-carbamic acid ethyl ester, {2,6-Dimethyl-4-[(4-trifluoromethyl-phenylamino)-methyl]-phenyl}-carbamic acid ethyl ester, 2-Cyclopentyl-N-{4-[(3-fluoro-phenylamino)-methyl]-2,6-dimethyl-phenyl}-acetamide, N-{4-[(3-Chloro-phenylamino)-methyl]-2,6-dimethylphenyl}-2-cyclopentyl-acetamide, 2-Cyclopentyl-N-{4-[(3-methoxy-phenylamino)-methyl]-2,6-dimethyl-phenyl}-acetamide, N-{4-[(4-Chloro-phenylamino)-methyl]-2,6-dimethyl-phenyl}-2-cyclopentyl-acetamide, 2-Cyclopentyl-N-{4-[(3,4-difluoro-phenylamino)-methyl]-2,6-dimethyl-phenyl}-acetamide, 2-Cyclopentyl-N-{2,6-dimethyl-4-[(4-trifluoromethyl-phenylamino)-methyl]-phenyl}-acetamide, 2-Cyclopentyl-N-[2,6-dimethyl-4-(p-tolylamino-methyl)-phenyl]-acetamide, 2-Cyclopentyl-N-{2,6-dimethyl-4-[(3-trifluoromethyl-phenylamino)-methyl]-phenyl}-acetamide, 2-Cyclopentyl-N-{4-[(3,5-difluoro-phenylamino)-methyl]-2,6-dimethylphenyl}-acetamide, {4-[(4-Fluoro-phenylamino)-methyl]-2,6-dimethyl-phenyl}-carbamic acid propyl ester, {4-[(4-Chloro-phenylamino)-methyl]-2,6-dimethylphenyl}-carbamic acid propyl ester, {2,6-Dimethyl-4-[(4-trifluoromethyl-phenylamino)-methyl]-phenyl}-carbamic acid propyl ester, {4-[(3,5-Difluoro-phenylamino)-methyl]-2,6-dimethyl-phenyl}-carbamic acid propyl ester, {4-[(3-Fluoro-phenylamino)-methyl]-2,6-dimethyl-phenyl}-carbamic acid propyl ester, N-(4-Bromo-2-methyl-6-morpholin-4-yl-phenyl)-3,3-dimethyl-butyramide, {4-[(4-Methoxyphenylamino)-methyl]-2,6-dimethylphenyl}-carbamic acid propyl ester, (R)-2-Amino-4-methylpentanoic acid [2,6-dimethyl-4-(4-trifluoromethylbenzylamino)-phenyl]-amide, Pentanoic acid {4-[(4-chlorophenylamino)-methyl]-2,6-dimethylphenyl}-amide, 2-(4-Chlorophenyl)-N-{4-[(4-chlorophenylamino)-methyl]-2,6-dimethylphenyl}-acetamide, {2,6-Dimethyl-4-[(4-trifluoromethylphenylamino)-methyl]-phenyl}-carbamic acid 2-methoxyethyl ester, N-{4-[(5-Chloro-pyridin-2-ylamino)-methyl]-2,6-dimethylphenyl}-2-cyclopentylacetamide, 2-Cyclopentyl-N-{4-[(2,6-dichloro-pyridin-4-ylamino)-methyl]-2,6-dimethylphenyl}-acetamide, N-{2-Chloro-6-methyl-4-[(6-trifluoromethyl-pyridin-3-ylmethyl)-amino]-phenyl}-2-(3-fluoro-phenyl)-acetamide, N-[2-Chloro-6-trifluoromethyl-4-(4-trifluoromethylbenzylamino)-phenyl]-2-cyclopentylacetamide, [2-Amino-6-methyl-4-(4-trifluoromethylbenzylamino)-phenyl]-carbamic acid ethyl ester, 3,3-Dimethyl-N-[2-methyl-6-morpholin-4-yl-4-(4-trifluoromethylbenzylamino)-phenyl]-butyramide, 2-Cyclopentyl-N-{2,6-dichloro-4-[(4-fluoro-phenylamino)-methyl]-phenyl}-acetamide, 2-Cyclopentyl-N-{2,6-dichloro-4-[(5-trifluoromethylpyridin-2-ylamino)-methyl]-phenyl}-acetamide, or a pharmaceutically acceptable salt thereof.

In another embodiment, the invention relates a compound according to formula 7 wherein said compound is selected from the group of:
(2,4-Dimethyl-6-morpholin-4-yl-pyridin-3-yl)-carbamic acid benzyl ester; (2,4-Dimethyl-6-morpholin-4-yl-pyridin-3-yl)-carbamic acid 2-chloro-benzyl ester; 2-(4-Chloro-phenyl)--(2,4-dimethyl-6-morpholin-4-yl-pyridin-3-yl)-acetamide; 2-Phenyl-cyclopropanecarboxylic acid (2,4-dimethyl-6-morpholin-4-yl-pyridin-3-yl)-amide; N-(2,4-Dimethyl-6-morpholin-4-yl-pyridin-3-yl)-2-thiophen-2-yl-acetamide; 3-Cyclohexyl-*N*-(2,4-dimethyl-6-morpholin-4-yl-pyridin-3-yl)-propionamide; (2,4-Dimethyl-6-morpholin-4-yl-pyridin-3-yl)-carbamic acid isobutyl ester; 3-(3-Chlorophenyl)-*N*-(2,4-dimethyl-6-morpholin-4-yl-pyridin-3-yl)-propionamide; *N*-(2,4-Dimethyl-6-morpholin-4-yl-pyridin-3-yl)-2-(3,5-dimethyl-phenyl)-acetamide; *N*-(2,4-Dimethyl-6-morpholin-4-yl-pyridin-3-yl)-3-p-tolyl-propionamide; 2-(3-Chlorophenyl)-*N*-(2,4-dimethyl-6-morpholin-4-yl-pyridin-3-yl)-acetamide; 2-(3,4-Dichlorophenyl)-*N*-(2,4-dimethyl-6-morpholin-4-yl-pyridin-3-yl)-acetamide; *N*-(2,4-Dimethyl-6-morpholin-4-yl-pyridin-3-yl)-2-thiophen-3-yl-acetamide; *N*-(2,4-Dimethyl-6-morpholin-4-yl-pyridin-3-yl)-2-p-tolyl-acetamide; 2-(3-Bromo-phenyl)-*N*-(2,4-dimethyl-6-morpholin-4-yl-pyridin-3-yl)-acetamide; *N*-(2,4-Dimethyl-6-morpholin-4-yl-pyridin-3-yl)-2-(3-trifluoromethyl-phenyl)-acetamide; *N*-(2,4-Dimethyl-6-morpholin-4-yl-pyridin-3-yl)-2-phenyl-acetamide; 3,5,5-Trimethyl-hexanoic acid (2,4-dimethyl-6-morpholin-4-yl-pyridin-3-yl)-amide; Octanoic acid (2,4-dimethyl-6-morpholin-4-yl-pyridin-3-yl)-amide; *N*-(2,4-Dimethyl-6-morpholin-4-yl-pyridin-3-yl)-2-naphthalen-2-yl-acetamide; Heptanoic acid (2,4-dimethyl-6-morpholin-4-yl-pyridin-3-yl)-amide; *N*-(2,4-Dimethyl-6-morpholin-4-yl-pyridin-3-yl)-2-(3,4-dimethyl-phenyl)-acetamide; 2-Cyclohex-1-enyl-*N*-(2,4-dimethyl-6-morpholin-4-yl-pyridin-3-yl)-acetamide; *N*-(2,4-Dimethyl-6-morpholin-4-yl-pyridin-3-yl)-2-(4-methoxy-3-methyl-phenyl)-acetamide; *N*-(2,4-Dimethyl-6-morpholin-4-yl-pyridin-3-yl)-2-(4-methoxy-phenyl)-acetamide; *N*-(2,4-Dimethyl-6-morpholin-4-yl-pyridin-3-yl)-3-(4-methoxy-phenyl)-propionamide; *N*-(2,4-Dimethyl-6-morpholin-4-yl-pyridin-3-yl)-2-m-tolyl-acetamide; *N*-(2,4-Dimethyl-6-morpholin-4-yl-pyridin-3-yl)-2-(4-fluoro-phenyl)-acetamide; *N*-(2,4-Dimethyl-6-morpholin-4-yl-pyridin-3-yl)-3,3-dimethyl-butyramide; *N*-(2,4-Dimethyl-6-morpholin-4-yl-pyridin-3-yl)-2-(3-fluorophenyl)-acetamide; 2-Bicyclo[2.2.1]hept-2-yl-*N*-(2,4-dimethyl-6-morpholin-4-yl-pyridin-3-yl)-acetamide; 2-(3,4-Difluoro-phenyl)-*N*-(2,4-dimethyl-6-morpholin-4-yl-pyridin-3-yl)-acetamide; 4-Methyl-pentanoic acid (2,4-dimethyl-6-morpholin-4-yl-pyridin-3-yl)-amide; 2-Cyclopent-2-enyl-*N*-(2,4-dimethyl-6-morpholin-4-yl-pyridin-3-yl)-acetamide; 2-Cyclohexyl-*N*-(2,4-dimethyl-6-morpholin-4-yl-pyridin-3-yl)-acetamide; 5-Methyl-hexanoic acid (2,4-dimethyl-6-morpholin-4-yl-pyridin-3-yl)-amide; 2-Cyclopentyl-*N*-(2,4-dimethyl-6-morpholin-4-yl-pyridin-3-yl)-acetamide; 3-Cyclopentyl-*N*-(2,4-dimethyl-6-morpholin-4-yl-pyridin-3-yl)-propionamide; and
Hexanoic acid (2,4-dimethyl-6-morpholin-4-yl-pyridin-3-yl)-amide; N-(4-Chloro-2-methoxy-6-morpholin-4-yl-pyridin-3-yl)-2-cyclopentylacetamide; N-(2-Chloro-4-methoxy-6-morpholin-4-yl-pyridin-3-yl)-2-cyclopentylacetamide; N-(2-Chloro-4-methoxy-6-morpholin-4-yl-pyridin-3-yl)-3,3-dimethylbutyramide; N-(4-Chloro-2-methoxy-6-morpholin-4-yl-pyridin-3-yl)-3,3-dimethylbutyramide; N-(4-Chloro-2-methoxy-6-morpholin-4-yl-pyridin-3-yl)-propionamide or a pharmaceutically acceptable salt thereof.

In another embodiment, the invention relates a compound according to formula 8 wherein said compound is selected from the group of:
N-[4-Amino-6-methyl-2-(4-trifluoromethylbenzylamino)-pyrimidin-5-yl]-2-cyclopentylacetamide, N-[4-Amino-6-methyl-2-(4-trifluoromethylbenzylamino)-pyrimidin-5-yl]-3,3-dimethylbutyramide, N-[4-Amino-6-methyl-2-(4-trifluoromethylbenzylamino)-pyrimidin-5-yl]-2-(4-fluorophenyl)-acetamide,
Hexanoic acid [4-amino-6-methyl-2-(4-trifluoromethylbenzylamino)-pyrimidin-5-yl]-amide, N-[4-Amino-6-methyl-2-(4-trifluoromethylbenzylamino)-pyrimidin-5-yl]-2-(3-chlorophenyl)-acetamide, 2-Cyclopentyl-N-(4,6-dimethyl-2-morpholin-4-yl-pyrimidin-5-yl)-acetamide, N-(4,6-Dimethyl-2-morpholin-4-yl-pyrimidin-5-yl)-3,3-dimethylbutyramide, N-(4,6-Dimethyl-2-morpholin-4-ylpyrimidin-5-yl)-2-(4-fluorophenyl)-acetamide, 2-(3,4-Difluorophenyl)-N-(4,6-dimethyl-2-morpholin-4-ylpyrimidin-5-yl)-acetamide, N-(4,6-Dimethyl-2-morpholin-4-ylpyrimidin-5-yl)-2-(3-fluorophenyl)-acetamide and Hexanoic acid (4,6-dimethyl-2-morpholin-4-ylpyrimidin-5-yl)-amide, or a pharmaceutically acceptable salt thereof.

In another embodiment, the present invention relates to novel compounds, which are openers of the KCNQ potassium channels.

In another embodiment of the present invention, the following definitions are applied for formula 1:
The term heteroatom refers to a nitrogen, oxygen or sulphur atom.
Halogen means fluoro, chloro, bromo or iodo.
The expression C₁₋₆-alk(en/yn)yl means a C₁₋₆-alkyl, C₂₋₆-alkenyl or a C₂₋₆-alkynyl group.
The term C₁₋₆-alkyl refers to a branched or unbranched alkyl group having from one to six carbon atoms inclusive, including but not limited to methyl, ethyl, 1-propyl, 2-propyl, 1-butyl, 2-butyl, 2-methyl-2-propyl, 2-2-dimethyl-1-propyl and 2-methyl-1-propyl.
Similarly, C₂₋₆-alkenyl and C₂₋₆-alkynyl, respectively, designate such groups having from two to six carbon atoms, including one double bond and one triple bond respectively, including but not limited to ethenyl, propenyl, butenyl, ethynyl, propynyl and butynyl.
The expression C₃₋₈-cycloalk(en)yl means a C₃₋₈-cycloalkyl- or cycloalkenyl group.
The term C₃₋₈-cycloalkyl designates a monocyclic or bicyclic carbocycle having three to eight C-atoms, including, but not limited to, cyclopropyl, cyclopentyl, cyclohexyl, etc.
The term C₃₋₈-cycloalkenyl designates a monocyclic or bicyclic carbocycle having three to eight C-atoms and including one double bond.

When two substituents together with the nitrogen atom to which they are attached form a 4-8 membered saturated or unsaturated ring which optionally contains 1, 2 or 3 further heteroatoms, then a monocyclic ring system is formed by 4 to 8 atoms selected from the nitrogen atom, 1-7 carbonatoms and 0-3 heteroatoms selected from N, S or O. Examples of such ring systems are azetidine, beta-lactame, pyrrolidine, piperidine, piperazine, morpholine, pyrrole, oxazolidine, thiazolidine, imidazolidine, tetrazole and pyrazole.

The term aryl refers to aromatic systems such as pyridine, thiazole, oxazole, phenyl, naphtyl, thiophene and furan, which are optionally substituted with one or more substituents independently being hydroxy, halogen, C₁₋₆-alk(en/yn)yl, C₃₋₈-cycloalk(en)yl, C₃₋₈-cycloalk(en)yl-C₁₋₆-alk(en/yn)yl, halo-C₁₋₆-alk(en/yn)yl, C₁₋₆-alkoxy, C₃₋₈-alkoxy, acyl, nitro or cyano, -CO-NH-C₁₋₆-alk(en/yn)yl, -CO-N(C₁₋₆-alk(en/yn)yl)₂, -NH-C₁₋₆-alk(en/yn)yl, -N(C₁₋₆-alk(en/yn)yl)₂, -S- C₁₋₆-alk(en/yn)yl, -SO₂-C₁₋₆-alk(en/yn)yl, -SO₂O-C₁₋₆-alk(en/yn)yl, -NH₂, -SO₂N(C₁₋₆-alk(en/yn)yl)₂ or - SO₂NH-C₁₋₆-alk(en/yn)yl; or
two adjacent substituents may together with the aromatic group to which they are attached form a 4-8 membered ring, which optionally contains one or two heteroatoms.

When two adjacent substituents together with the aromatic group to which they are attached form a 4-8 membered ring, which optionally contains one or two heteroatoms, then a ring system is formed by 4-8 atoms selected from 3-8 carbonatoms and 0-2 heteroatoms selected from N, S, or O. Such two adjacent substituents may together form:
- (CH₂)_{n"}-CH₂-, -CH=CH-(CH₂)_{m"}-, -CH₂-CH=CH-(CH₂)_{p"}, -CH=CH-CH=CH-,
- (CH₂)_{n"}-O-, -O-(CH₂)_{m"}-O-, -CH₂-O-(CH₂)_{p"}-O-, -CH₂-O-CH₂-O-CH₂-,
- (CH₂)_{n"}-S-, -S-(CH₂)_{m"}-S-, -CH₂-S-(CH₂)_{p"}-S-, -CH₂-S-CH₂-S-CH₂-,
- (CH₂)_{n"}-NH-, -NH-(CH₂)_{m"}-NH-, -CH₂-NH-(CH₂)_{p"}-NH-, -CH=CH-NH-,
- O-(CH₂)_{m"}-NH-, -CH₂-O-(CH₂)_{p"}-NH- or -O-(CH₂)_{p"}-NH-CH₂-, -S-(CH₂)_{m"}-NH-,
- N=CH-NH-, -N=CH-O- or -N=CH-S-, wherein m" is 1, 2 or 3, n" is 2, 3 or 4 and p" is 1 or 2.

As used herein, the term acyl refers to formyl, C₁₋₆-alk(en/yn)ylcarbonyl, C₃₋₈-cycloalk(en)ylcarbonyl, arylcarbonyl, aryl-C₁₋₆-alk(en/yn)ylcarbonyl or a C₃₋₈-cycloalk(en)yl-C₁₋₆-alk(en/yn)yl-carbonyl group, wherein C₁₋₆-alk(en/yn)yl, C₃₋₈-cycloalk(en)yl and aryl are as defined above.

The term halo-C₁₋₆-alk(en/yn)yl designates C₁₋₆-alk(en/yn)yl being substituted with one or more halogen atoms, including but not limited to trifluormethyl. Similarly, halo-C₃₋₈-cycloalk(en)yl designates C₃₋₈-cycloalk(en)yl being substituted with one or more halogen atoms and halo-C₃₋₈-cycloalk(en)yl-C₁₋₆-alk(en/yn)yl, designates C₃₋₈-cycloalk(en)yl-C₁₋₆-alk(en/yn)yl being substituted with one or more halogen atoms.

In the term C₃₋₈-cycloalk(en)yl-C₁₋₆-alk(en/yn)yl, C₃₋₈-cycloalk(en)yl and C₁₋₆-alk(en/yn)yl are as defined above.

Furthermore, terms such as hydroxy-C₁₋₆-alk(en/yn)yl, hydroxy-C₃₋₈-cycloalk(en)yl, aryl-C₁₋₆-alk(en/yn)yl, C₁₋₆-alk(en/yn)yloxy, C₃₋₈-cycloalk(en)yloxy, C₁₋₆-alk(en/yn)ylcarbonyl, C₃₋₈-cycloalk(en)ylcarbonyl, arylcarbonyl, aryl-C₁₋₆-alk(en/yn)ylcarbonyl, C₃₋₈-cycloalk(en)yl-C₁₋₆-alk(en/yn)ylcarbonyl, aryl-C₃₋₈-cycloalk(en)yl, hydroxy-C₃₋₈-cycloalk(en)yl-C₁₋₆-alk(en/yn)yl, hydroxy-C₁₋₆-alk(en/yn)yl-C₃₋₈-cycloalk(en)yl, cyano-C₁₋₆-alk(en/yn)yl, cyano-C₃₋₈-cycloalk(en)yl, cyano-C₃₋₈-cycloalk(en)yl-C₁₋₆-alk(en/yn)yl, NR¹⁰R^{10'}-C₁₋₆-alk(en/yn)yl and NR¹⁰R^{10'}-C₃₋₈-cycloalk(en)yl etc. designate groups in which the C₁₋₆-alk(en/yn)yl, C₃₋₈-cycloalk(en)yl and aryl are as defined above.

In another embodiment of the present invention, the following definitions are applied for formula 2:
The term heteroatom refers to a nitrogen, oxygen or sulphur atom.

Halogen means fluoro, chloro, bromo or iodo.

The expressions C₁₋₆-alk(en/yn)yl and C₁₋₆-alk(an/en/yn)yl mean a C₁₋₆-alkyl, C₂₋₆-alkenyl or a C₂₋₆-alkynyl group.

The term C₁₋₆-alkyl refers to a branched or un-branched alkyl group having from one to six carbon atoms inclusive, including but not limited to methyl, ethyl, 1-propyl, 2-propyl, 1-butyl, 2-butyl, 2-methyl-2-propyl and 2-methyl-1-propyl.

Similarly, C₂₋₆-alkenyl and C₂₋₆-alkynyl, respectively, designate such groups having from two to six carbon atoms, including one double bond and one triple bond respectively, including but not limited to ethenyl, propenyl, butenyl, ethynyl, propynyl and butynyl.

The expression C₁₋₃-alk(en/yn)yl means a C₁₋₃-alkyl, C₂₋₃-alkenyl or a C₂₋₃-alkynyl group.

The term C₁₋₃-alkyl refers to a branched or un-branched alkyl group having from one to three carbon atoms inclusive, including but not limited to methyl, ethyl, 1-propyl and 2-propyl.

Similarly, C₂₋₃-alkenyl and C₂₋₃-alkynyl, respectively, designate such groups having from two to three carbon atoms, including one double bond and one triple bond respectively, including but not limited to ethenyl, propenyl, ethynyl and propynyl.

The expressions C₃₋₈-cycloalk(en)yl and C₃₋₈-cycloalk(an/en)yl mean a C₃₋₈-cycloalkyl- or cycloalkenyl group.

The term C₃₋₈-cycloalkyl designates a monocyclic or bicyclic carbocycle having three to eight C-atoms, including but not limited to cyclopropyl, cyclopentyl, cyclohexyl, etc.

The expressions C₃₋₆-cycloalk(en)yl and C₃₋₆-cycloalk(an/en)yl mean a C₃₋₆-cycloalkyl- or cycloalkenyl group.

The term C₃₋₆-cycloalkyl designates a monocyclic or bicyclic carbocycle having three to six C-atoms, including but not limited to cyclopropyl, cyclopentyl, cyclohexyl, etc.

The term C₃₋₈-cycloalkenyl designates a monocyclic or bicyclic carbocycle having three to eight C-atoms and including one double bond.

The term heterocycloalk(en)yl designates monocyclic or bicyclic ring systems wherein the ring is formed by 5 to 8 atoms being selected from the group consisting of carbonatoms and heteroatoms; with the proviso that one or two of the ring forming atoms are independently selected heteroatoms. The term heterocycloalk(en)yl may thus designate a monocyclic or bicyclic ring system wherein the ring is formed by 5 to 8 atoms selected from 3-7 carbonatoms and 1 or 2 heteroatoms selected from N, S, or O. Examples of such ring systems are morpholine, pyrrolidine, piperidine and piperazine.

The term halo-C₁₋₆-alk(en/yn)yl designates C₁₋₆-alk(en/yn)yl being substituted with one or more halogen atoms, including but not limited to trifluoromethyl. Similarly, halo-C₃₋₈-cycloalk(en)yl designates C₃₋₈-cycloalk(en)yl being substituted with one or more halogen atoms and halo-heterocycloalk(en)yl designates heterocycloalk(en)yl being substituted with one or more halogen atoms.

The term N**R**¹⁰**R**^{10'}-C₁₋₆-alk(en/yn)yl designates C₁₋₆-alk(en/yn)yl being substituted with N**R**¹⁰**R**^{10'}; N**R**¹²**R**^{12'}-C₁₋₆-alk(en/yn)yl designates C₁₋₆-alk(en/yn)yl being substituted with N**R**¹²**R**^{12'}; and N**R**⁷**R**^{7'}-C₁₋₆-alk(en/yn)yl designates C₁₋₆-alk(en/yn)yl being substituted with N**R**⁷**R**^{7'}. 2-amino-4-methyl-pentane is an example of such group, the example is not intended to be construed as limiting.

The term N**R**¹⁰**R**^{10'}-C₃₋₈-cycloalk(en)yl designates C₃₋₈-cycloalk(en)yl being substituted with N**R**¹⁰**R**^{10'}; N**R**¹²**R**^{12'}-C₃₋₈-cycloalk(en)yl designates C₃₋₈-cycloalk(en)yl being substituted with N**R**¹²**R**^{12'}; and N**R**⁷**R**^{7'}-C₃₋₈-cycloalk(en)yl designates C₃₋₈-cycloalk(en)yl being substituted with N**R**⁷**R**^{7'}. 1-amino-cyclopropane is an example of such group, the example is not intended to be construed as limiting.

The term N**R**¹⁰**R**^{10'}-C₃₋₈-cycloalk(en)yl-C₁₋₆-alk(en/yn)yl designates C₃₋₈-cycloalk(en)yl-C₁₋₆-alk(en/yn)yl being substituted with N**R**¹⁰**R**^{10'}; N**R**¹²**R**^{12'}-C₃₋₈-cycloalk(en)yl-C₁₋₆-alk(en/yn)yl designates C₃₋₈-cycloalk(en)yl-C₁₋₆-alk(en/yn)yl being substituted with N**R**¹²**R**^{12'}; and N**R**⁷**R**^{7'}-C₃₋₈-cycloalk(en)yl-C₁₋₆-alk(en/yn)yl designates C₃₋₈-cycloalk(en)yl-C₁₋₆-alk(en/yn)yl being substituted with N**R**⁷**R**^{7'}.

When any of N**R**¹²**R**^{12'}-C₁₋₆-alk(en/yn)yl, N**R**¹²**R**^{12'}-C₃₋₈-cycloalk(en)yl, N**R**¹²**R**^{12'}-C₃₋₈-cycloalk(en)yl-C₁₋₆-alk(en/yn)yl is optionally substituted, then any of C₁₋₆-alk(en/yn)yl, C₃₋₈-cycloalk(en)yl, C₃₋₈-cycloalk(en)yl-C₁₋₆-alk(en/yn)yl is optionally substituted with one or more substituents independently being C₁₋₆-alk(en/yn)yl, C₃₋₈-cycloalk(en)yl, C₃₋₈-cycloalk(en)yl-C₁₋₆-alk(en/yn)yl or Ar.

As used herein, the term acyl refers to formyl, C₁₋₆-alk(en/yn)ylcarbonyl, C₃₋₈-cycloalk(en)ylcarbonyl, Ar-carbonyl, Ar-C₁₋₆-alk(en/yn)ylcarbonyl or a C₃₋₈-cycloalk(en)yl-C₁₋₆-alk(en/yn)yl-carbonyl group, wherein C₁₋₆-alk(en/yn)yl, C₃₋₈-cycloalk(en)yl and Ar are as defmed above.

When two substituents together with a nitrogen atom form a 5-8 membered saturated or unsaturated ring which optionally contains one further heteroatom, then a monocyclic ring system is formed by 5 to 8 atoms, one or two of said atoms are heteroatoms selected from N, S, or O. Examples of such ring systems are pyrrolidine, piperidine, piperazine, morpholine, pyrrole, oxazolidine, thiazolidine, imidazolidine, azetidine, beta-lactame, tetrazole and pyrazole.

When two adjacent substituents together with an aromatic group to which they are attached form a 5-8 membered ring, which optionally contains one or two heteroatoms, then a ring is formed by 5-8 atoms selected from 3-8 carbonatoms and 0-2 heteroatoms selected from N, S, or O and. Such two adjacent substituents may together form:
- (CH₂)_{n"}-CH₂-, -CH=CH-(CH₂)_{m"}-, -CH₂-CH=CH-(CH₂)_{p"}, -CH=CH-CH=CH-,
- (CH₂)_{n"}-O-, -O-(CH₂)_{m"}-O-, -CH₂-O-(CH₂)_{p"}-O-, -CH₂-O-CH₂-O-CH₂-,
- (CH₂)_{n"}-S-, -S-(CH₂)_{m"}-S-, -CH₂-S-(CH₂)_{p"}-S-, -CH₂-S-CH₂-S-CH₂-,
- (CH₂)_{n"}-NH- , -NH-(CH₂)_{m"}-NH-, -CH₂-NH-(CH₂)_{p"}-NH-, -CH=CH-NH-,
- O-(CH₂)_{m"}-NH-, -CH₂-O-(CH₂)_{p"}-NH- or -O-(CH₂)_{p"}-NH-CH₂-, -S-(CH₂)_{m"}-NH-, - N=CH-NH-, -N=CH-O- or -N=CH-S-, wherein m" is 1, 2 or 3, n" is 2, 3 or 4 and p" is 1 or 2.

The term Ar refers to optionally substituted aromatic systems of 5-10 carbon atoms, wherein 0, 1, 2, 3 or 4 carbon atoms may be replaced by heteroatoms independently selected from N, S, or O. Examples of such Ar groups are optionally substituted phenyl, optionally substituted naphtyl, optionally substituted quinoline, optionally substituted indol, optionally substituted pyridine, optionally substituted pyrimidine, optionally substituted thiophene, optionally substituted furan, optionally substituted thiazole and optionally substituted oxazole. Such optionally substituted Ar groups may be substituted with one or more substituents independently being hydroxy, halogen, C₁₋₆-alk(en/yn)yl, C₃₋₈-cycloalk(en)yl, C₃₋₈-cycloalk(en)yl-C₁₋₆-alk(en/yn)yl, halo-C₁₋₆-alk(en/yn)yl, C₁₋₆-alk(en/yn)yloxy, C₃₋₈-alk(en/yn)yloxy, acyl, nitro, cyano, -CO-NH-C₁₋₆-alk(en/yn)yl, -CO-N(C₁₋₆-alk(en/yn)yl)₂, -NH₂, -NH-C₁₋₆-alk(en/yn)yl, - N(C₁₋₆-alk(en/yn)yl)2, S- C₁₋₆-alk(en/yn)yl, -SO₂N(C₁₋₆-alk(en/yn)yl)₂ and -SO₂NH-C₁₋₆-alk(en/yn)yl, SO₂-C₁₋₆-alk(en/yn)yl and SO₂O-C₁₋₆-alk(en/yn)yl; or two adjacent substituents may together with the aromatic group form a 5-8 membered ring, which optionally contains one or two heteroatoms and which may be saturated or unsaturated.

The terms C₃₋₈-cycloalk(en)yl-C₁₋₆-alk(en/yn)yl, C₁₋₆-alk(en/yn)yl-C₃₋₈-cycloalk(en)yl, Ci-6-alk(en/yn)yl-heterocycloalk(en)yl, Ar, Ar-C₁₋₆-alk(en/yn)yl, Ar-C₃₋₈-cycloalk(en)yl, Ar-heterocycloalk(en)yl, Ar-C₃₋₈-cycloalk(en)yl-C₁₋₆-alk(en/yn)yl, ArC₁₋₆-alk(en/yn)yl-C₃₋₈-cycloalk(en)yl, Ar-C₁₋₆-alk(en/yn)yl-heterocycloalk(en)yl, C₁₋₆-alk(en/yn)yloxy, C₂₋₆-alkenyloxy, C₂₋₆-alkynyloxy, C₃₋₈-cycloalk(en)yloxy, C₁₋₆-alk(en/yn)yloxy-C₁₋₆-alk(en/yn)yl, C₃₋₈-cycloalk(en)yloxy-C₁₋₆-alk(en/yn)yl, C₁₋₆-alk(en/yn)yloxy-C₃₋₈-cycloalk(en)yl, C₁₋₆-alk(en/yn)yloxy-heterocycloalk(en)yl, Ar-oxy-C₁₋₆-alk(en/yn)yl, Ar-C₁₋₆-alk(en/yn)yloxy-C₁₋₆-alk(en/yn)yl, C₁₋₆-alk(en/yn)ylcarbonyl, C₃₋₈-alk(en/yn)ylcarbonyl, Ar-carbonyl, Ar-C₁₋₆-alk(en/yn)ylcarbonyl, C₃₋₈-cycloalk(en)yl-C₁₋₆-alk(en/yn)ylcarbonyl, -CO-C₁₋₆-alk(en/yn)yl, S-C₁₋₆-alk(en/yn)yl, SO₂-C₁₋₆-alk(en/yn)yl and SO₂O-C₁₋₆-alk(en/yn)yl, C₁₋₆-alk(en/yn)yloxy-carbonyl-C₁₋₆-alk(en/yn)yl, C₃₋₈-cycloalk(en)yloxy-carbonyl-C₁₋₆-alk(en/yn)yl, C₃₋₈-cycloalk(en)yl-C₁₋₆-alk(en/yn)yloxy-carbonyl-C₁₋₆-alk(en/yn)yl, acyl, acyl-C₁₋₆-alk(en/yn)yl, acyl-C₃₋₈-cycloalk(en)yl, acyl-heterocycloalk(en)yl, acyl-C₃₋₈-cycloalk(en)yl-C₁₋₆-alk(en/yn)yl, acyl-C₁₋₆-alk(en/yn)yl-C₃₋₈-cycloalk(en)yl, acyl-C₁₋₆-alk(en/yn)yl-heterocycloalk(en)yl, hydroxy-C₁₋₆-alk(en/yn)yl, hydroxy-C₃₋₈-cycloalk(en)yl, hydroxy-heterocycloalk(en)yl, hydroxy-C₃₋₈-cycloalk(en)yl-C₁₋₆-alk(en/yn)yl, hydroxy-C₁₋₆-alk(en/yn)yl-C₃₋₈-cycloalk(en)yl, hydroxy-C₁₋₆-alk(en/yn)yl-heterocycloalk(en)yl, halo-C₁₋₆-alk(en/yn)yl, halo-C₃₋₈-cycloalk(en)yl, halo-heterocycloalk(en)yl, halo-C₃₋₈-cycloalk(en)yl-C₁₋₆-alk(en/yn)yl, halo-C₁₋₆-alk(en/yn)yl-C₃₋₈-cycloalk(en)yl, halo-C₁₋₆-alk(en/yn)yl-heterocycloalk(en)yl, halo-C₁₋₆-alk(en/yn)yl-Ar, halo-C₃₋₈-cycloalk(en)yl-Ar, halo-C₃₋₈-cycloalk(en)yl-C₁₋₆-alk(en/yn)yl-Ar, halo-C₁₋₆-alk(en/yn)yl-C₃₋₈-cycloalk(en)yl-Ar, halo-heterocycloalk(en)yl-Ar, cyano-C₁₋₆-alk(en/yn)yl, cyano-C₃₋₈-cycloalk(en)yl, cyano-heterocycloalk(en)yl, cyano-C₃₋₈-cycloalk(en)yl-C₁₋₆-alk(en/yn)yl, cyano-C₁₋₆-alk(en/yn)yl-C₃₋₈-cycloalk(en)yl, cyano-C₁₋₆-alk(en/yn)yl-heterocycloalk(en)yl etc. designate such groups in which the C₁₋₆-alk(en/yn)yl, C₂₋₆-alkenyl, C₂₋₆-alkynyl, C₃₋₈-cycloalk(en)yl, heterocycloalk(en)yl, Ar, cyano, halo-C₁₋₆-alk(en/yn)yl, halo-C₃₋₈-cycloalk(en)yl, halo-heterocycloalk(en)yl and acyl are as defmed above.

In another embodiment of the present invention, the following definitions are applied for formula 3:
The term heteroatom refers to a nitrogen, oxygen or sulphur atom.

Halogen means fluoro, chloro, bromo or iodo.

The expressions C₁₋₆-alk(en/yn)yl and C₁₋₆-alk(an/en/yn)yl mean a C₁₋₆-alkyl, C₂₋₆-alkenyl or a C₂₋₆-alkynyl group.

The term C₁₋₆-alkyl refers to a branched or unbranched alkyl group having from one to six carbon atoms inclusive, including but not limited to methyl, ethyl, 1-propyl, 2-propyl, 1-butyl, 2-butyl, 2-methyl-2-propyl, 2-2-dimethyl-1-propyl and 2-methyl-1-propyl.

Similarly, C₂₋₆-alkenyl and C₂₋₆-alkynyl, respectively, designate such groups having from two to six carbon atoms, including one double bond and one triple bond respectively, including but not limited to ethenyl, propenyl, butenyl, ethynyl, propynyl and butynyl.

The expressions C₁₋₄-alkyl and C₁₋₄-alkanyl refer to a branched or unbranched alkyl group having from one to four carbon atoms inclusive, including but not limited to methyl, ethyl, 1-propyl, 2-propyl, 1-butyl, 2-butyl, 2-methyl-2-propyl and 2-methyl-1-propyl.

The expression C₁₋₃-alk(en/yn)yl means a C₁₋₃-alkyl, C₂₋₃-alkenyl or a C₂₋₃-alkynyl group.

The term C₁₋₃-alkyl refers to a branched or unbranched alkyl group having from one to three carbon atoms inclusive, including but not limited to methyl, ethyl, 1-propyl and 2-propyl.

Similarly, C₂₋₃-alkenyl and C₂₋₃-alkynyl, respectively, designate such groups having from two to three carbon atoms, including one double bond and one triple bond respectively, including but not limited to ethenyl, propenyl, ethynyl and propynyl.

The expressions C₃₋₈-cycloalk(en)yl and C₃₋₈-cycloalk(an/en)yl mean a C₃₋₈-cycloalkyl- or cycloalkenyl group.

The term C₃₋₈-cycloalkyl designates a monocyclic or bicyclic carbocycle having three to eight C-atoms, including but not limited to cyclopropyl, cyclopentyl, cyclohexyl, etc.

The expressions C₃₋₆-cycloalk(en)yl and C₃₋₆-cycloalk(an/en)yl mean a C₃₋₆-cycloalkyl- or cycloalkenyl group.

The term C₃₋₆-cycloalkyl designates a monocyclic or bicyclic carbocycle having three to six C-atoms, including but not limited to cyclopropyl, cyclopentyl, cyclohexyl, etc.

The term C₃₋₈-cycloalkenyl, designates a monocyclic or bicyclic carbocycle having three to eight C-atoms and including one double bond.

The expression C₅₋₈-cycloalk(en)yl means a C₅₋₈-cycloalkyl- or cycloalkenyl group.

The term C₅₋₈-cycloalkyl designates a monocyclic or bicyclic carbocycle having five to eight C-atoms, including but not limited to cyclopentyl, cyclohexyl, etc.

The term C₅₋₈-cycloalkenyl designates a monocyclic or bicyclic carbocycle having five to eight C-atoms and including one or two double bonds.

In the term C₃₋₈-cycloalk(en)yl-C₁₋₆-alk(en/yn)yl, C₃₋₈-cycloalk(en)yl and C₁₋₆-alk(en/yn)yl are as defined above.

The term Ar refers to optionally substituted aromatic systems of 5-10 carbon atoms, wherein 0, 1, 2, 3 or 4 carbon atoms may be replaced with independently selected heteroatoms. Examples of such Ar groups are optionally substituted phenyl, optionally substituted naphtyl, optionally substituted thiophene, optionally substituted furan, optionally substituted thiazole, optionally substituted pyridine, optionally substituted pyrimidine, optionally substituted pyrrole and optionally substituted oxazole. Ar may be substituted with one or more substituents independently being hydroxy, halogen, C₁₋₆-alk(en/yn)yl, C₃₋₈-cycloalk(en)yl, C₃₋₈-cycloalk(en)yl-C₁₋₆-alk(en/yn)yl, halo-C₁₋₆-alk(en/yn)yl, C₁₋₆-alk(an/en/yn)yloxy, C₃₋₈-alk(an/en/yn)yloxy, acyl, cyano, -CO-NH-C₁₋₆-alk(en/yn)yl, -CO-N(C₁₋₆-alk(en/yn)yl)₂, NH-C₁₋₆-alk(en/yn)yl, -N(C₁₋₆-alk(en/yn)yl)₂, -NH₂, -S-C₁₋₆-alk(en/yn)yl, -SO₂-C₁₋₆-alk(en/yn)yl, -SO₂N(C₁₋₆-alk(en/yn)yl)₂, -SO₂NH-C₁₋₆-alk(en/yn)yl and -SO₂O-C₁₋₆-alk(en/yn)yl; or two substituents may together form a 5-8 membered saturated or unsaturated ring which optionally contains one or two heteroatoms. Such two ringforming substituents may be adjacent and may together form:
- (CH₂)_{n**}-CH₂-, -CH=CH-(CH₂)_{m**}-, -CH₂-CH=CH-(CH₂)_{p**},
- (CH₂)_{n**}O-, -O-(CH₂)_{m**}-O-, -CH₂-O-(CH₂)_{p**}-O-, -CH₂-O-CH₂-O-CH₂-,
- (CH₂)_{n**}-S-, -S-(CH₂)_{m**}-S-, -CH₂-S-(CH₂)_{p**}-S-, -CH₂-S-CH₂-S-CH₂-,
- (CH₂)_{n**}-NH- , -NH-(CH₂)_{m**}-NH-, -CH₂-NH-(CH₂)_{p**}-NH-, - CH=CH-NH-,
- O-(CH₂)_{m**}-NH-, -CH₂-O-(CH₂)_{p**}-NH- or -O-(CH₂)_{p**}-NH-CH₂-,
- S-(CH₂)_{m**}-NH-, -N=CH-NH-, -N=CH-O- or -N=CH-S-, wherein m** is 1, 2 or 3, n** is 2, 3 or 4 and p** is 1 or 2.

As used herein, the term acyl refers to formyl, C₁₋₆-alk(en/yn)ylcarbonyl, C₃₋₈-cycloalk(en)ylcarbonyl, Ar-carbonyl, Ar-C₁₋₆-alk(en/yn)ylcarbonyl or a C₃₋₈-cycloalk(en)yl-C₁₋₆-alk(en/yn)yl-carbonyl group, wherein C₁₋₆-alk(en/yn)yl, C₃₋₈-cycloalk(en)yl and Ar are as defined above.

The term halo-C₁₋₆-alk(en/yn)yl designates C₁₋₆-alk(en/yn)yl being substituted with one or more halogen atoms, including but not limited to trifluoromethyl. Similarly, halo-C₃₋₈-cycloalk(en)yl designates C₃₋₈-cycloalk(en)yl being substituted with one or more halogen atoms and halo-C₃₋₈-cycloalk(en)yl-C₁₋₆-alk(en/yn)yl, designates C₃₋₈-cycloalk(en)yl-C₁₋₆-alk(en/yn)yl being substituted with one or more halogen atoms.

The terms hydroxy-C₁₋₆-alk(en/yn)yl, hydroxy-C₃₋₈-alk(en/yn)yl, hydroxy-C₃₋₈-cycloalk(en)yl, Ar-C₁₋₆-alk(en/yn)yl, Ar-C₃₋₈-cycloalk(en)yl, C₁₋₆-alk(an/en/yn)yloxy, C₁₋₄-alkanyloxy, C₂₋₆-alkenyloxy, C₂₋₆-alkynyloxy, C₃₋₈-alk(an/en/yn)yloxy, C₁₋₆-alk(en/yn)ylcarbonyl, C₃₋₈-alk(en/yn)ylcarbonyl, Ar-carbonyl, Ar-C₁₋₆-alk(en/yn)ylcarbonyl, C₃₋₈-cycloalk(en)yl-C₁₋₆-alk(en/yn)ylcarbonyl etc. designate such groups in which the C₁₋₆-alk(en/yn)yl, C₂₋₆-alkenyl, C₂₋₆-alkynyl, C₃₋₈-cycloalk(en)yl and Ar are as defined above.

The term "two substituents together form a 5-8 membered saturated or unsaturated ring, which optionally contains one or two heteroatoms," refers to aliphatic or aromatic carbocyclic or heterocyclic systems wherein the ring is formed by 5 to 8 atoms which may be substituted by one or more substituents independently being C₁₋₆-alk(en/yn)yl, C₃₋₈-alk(en/yn)yl, C₃₋₈-cycloalk(en)yl-C₁₋₆-alk(en/yn)yl, halogen, halo-C₁₋₆-alk(en/yn)yl, halo-C₃₋₈-alk(en/yn)yl or C₃₋₈-cycloalk(en)yl-C₁₋₆-alk(en/yn)yl. The ring forming atoms are selected from 3-8 carbon atoms and 0-2 heteroatoms selected from N, S, or O. When the two ring forming substituents are attached to the same nitrogen atom, then said nitrogen atom becomes one of the atoms forming the ring. When two ring forming substituents are attached to an aliphatic or aromatic carbocyclic or heterocyclic group, then the two ringforming substituents are conveniently attached adjacent to each other and the ring formed by the two substituents is fused to the aliphatic or aromatic carbocyclic or heterocyclic group. Two ring forming substituents may together be represented by:
- (CH₂)_{n"}-CH₂-, -CH=CH-(CH₂)_{m"}-, -CH₂-CH=CH-(CH₂)_{p"},
- CH=CH-CH=CH-, -(CH₂)_{n"}-O-, -O-(CH₂)_{m"}-O-, -CH₂-O-(CH₂)_{p"}-O-,
- CH₂-O-CH₂-O-CH₂-, -(CH₂)_{n"}-S-, -S-(CH₂)_{m"}-S-, -CH₂-S-(CH₂)_{p"}-S-,
- CH₂-S-CH₂-S-CH₂-, -(CH₂)_{n"}-NH-, -NH-(CH₂)_{m"}-NH-, -CH₂-NH-(CH₂)_{p"}-NH-,
- CH=CH-NH-, -O-(CH₂)_{m"}-NH-, -CH₂-O-(CH₂)_{p"}-NH- or -O-(CH₂)_{p"}-NH-CH₂-,
- S-(CH₂)_{m"}-NH-, -N=CH-NH-, -N=CH-O- or -N=CH-S-, wherein m" is 1, 2 or 3, n" is 2, 3 or 4 and p" is 1 or 2.

In another embodiment of the present invention, the following definitions are applied for formula 4:
The term heteroatom refers to a nitrogen, oxygen or sulphur atom.

Halogen means fluoro, chloro, bromo or iodo.

The expressions C₁₋₆-alk(en/yn)yl and C₁₋₆-alk(an/en/yn)yl mean a C₁₋₆-alkyl, C₂₋₆-alkenyl or a C₂₋₆-alkynyl group. The term C₁₋₆-alkyl refers to a branched or un-branched alkyl group having from one to six carbon atoms inclusive, including but not limited to methyl, ethyl, 1-propyl, 2-propyl, 1-butyl, 2-butyl, 2-methyl-2-propyl and 2-methyl-1-propyl. Similarly, C₂₋₆-alkenyl and C₂₋₆-alkynyl, respectively, designate such groups having from two to six carbon atoms, including one double bond and one triple bond respectively, including but not limited to ethenyl, propenyl, butenyl, ethynyl, propynyl and butynyl.

The expression C₁₋₃-alk(en/yn)yl means a C₁₋₃-alkyl, C₂₋₃-alkenyl or a C₂₋₃-alkynyl group. The term C₁₋₃-alkyl refers to a branched or un-branched alkyl group having from one to three carbon atoms inclusive, including but not limited to methyl, ethyl, 1-propyl and 2-propyl. Similarly, C₂₋₃-alkenyl and C₂₋₃-alkynyl, respectively, designate such groups having from two to three carbon atoms, including one double bond and one triple bond respectively, including but not limited to ethenyl, 1-propenyl, 2-propenyl, 3-propenyl, ethynyl, 1-propynyl and 3-propynyl.

The expressions C₃₋₈-cycloalk(en)yl and C₃₋₈-cycloalk(an/en)yl mean a C₃₋₈-cycloalkyl- or cycloalkenyl group. The term C₃₋₈-cycloalkyl designates a monocyclic or bicyclic carbocycle having three to eight C-atoms, including but not limited to cyclopropyl, cyclobutyl, cyclopentyl, cyclohexyl, etc. The term C₃₋₈-cycloalkenyl designates a monocyclic or bicyclic carbocycle having three to eight C-atoms and including one double bond.

The expressions C₃₋₆-cycloalk(en)yl and C₃₋₆-cycloalk(an/en)yl mean a C₃₋₆-cycloalkyl- or cycloalkenyl group. The term C₃₋₆-cycloalkyl designates a monocyclic or bicyclic carbocycle having three to six C-atoms, including but not limited to cyclopropyl, cyclobutyl, cyclopentyl, cyclohexyl, etc.

The term heterocycloalk(en)yl designates a monocyclic or bicyclic ring system wherein the ring is formed by 4 to 8 atoms selected from 2-7 carbonatoms and 1 or 2 heteroatoms selected from N, S, or O.

When two substituents together with a carbon atom to which they are attached form a 3-8 membered saturated or unsaturated ring which optionally contains 1 or 2 heteroatoms, then a monocyclic ring system is formed by 3 to 8 atoms selected from 1-8 carbonatoms and 0-2 heteroatoms selected from N, S, or O. Examples of such ring systems are cyclopropyl, cyclobutyl, cyclopentyl, cyclohexyl.

The term halo-C₁₋₆-alk(en/yn)yl designates C₁₋₆-alk(en/yn)yl being substituted with one or more halogen atoms, including but not limited to trifluoromethyl. Similarly, halo-C₃₋₈-cycloalk(en)yl designates C₃₋₈-cycloalk(en)yl being substituted with one or more halogen atoms and halo-heterocycloalk(en)yl designates heterocycloalk(en)yl being substituted with one or more halogen atoms.

The term N**R**¹²**R**^{12'}-C₁₋₆-alk(en/yn)yl designates C₁₋₆-alk(en/yn)yl being substituted with N**R**¹²**R**^{12'}. The term N**R**¹²**R**^{12'}-C₃₋₈-cycloalk(en)yl designates C₃₋₈-cycloalk(en)yl being substituted with N**R**¹²**R**^{12'}. The term N**R**¹²**R**^{12'}-C₃₋₈-cycloalk(en)yl-C₁₋₆-alk(en/yn)yl designates C₃₋₈-cycloalk(en)yl-C₁₋₆-alk(en/yn)yl being substituted with N**R**¹²**R**^{12'}. When any of N**R**¹²**R**^{12'}-C₁₋₆-alk(en/yn)yl, N**R**¹²**R**^{12'}-C₃₋₈-cycloalk(en)yl and N**R**¹²**R**^{12'}-C₃₋₈-cycloalk(en)yl-C₁₋₆-alk(en/yn)yl is optionally substituted, then any of C₁₋₆-alk(en/yn)yl, C₃₋₈-cycloalk(en)yl, C₃₋₈-cycloalk(en)yl-C₁₋₆-alk(en/yn)yl is optionally substituted with one or more substituents independently being C₁₋₆-alk(en/yn)yl, C₃₋₈-cycloalk(en)yl, C₃₋₈-cycloalk(en)yl-C₁₋₆-alk(en/yn)yl or Ar.

As used herein, the term acyl refers to formyl, C₁₋₆-alk(en/yn)ylcarbonyl, C₃₋₈-cycloalk(en)ylcarbonyl, Ar-carbonyl, Ar-C₁₋₆-alk(en/yn)ylcarbonyl or a C₃₋₈-cycloalk(en)yl-C₁₋₆-alk(en/yn)yl-carbonyl group, wherein C₁₋₆-alk(en/yn)yl, C₃₋₈-cycloalk(en)yl and Ar are as defined above.

When two substituents together with a nitrogen atom to which they are attached form a 4-8 membered saturated or unsaturated ring which optionally contains 1, 2 or 3 further heteroatoms, then a monocyclic ring system is formed by 4 to 8 atoms selected from the nitrogen atom, 1-7 carbonatoms and 0-3 further heteroatoms selected from N, S, or O. Examples of such ring systems are azetidine, beta-lactame, pyrrolidine, piperidine, piperazine, morpholine, pyrrole, oxazolidine, thiazolidine, imidazolidine, azetidine, beta-lactame, tetrazole and pyrazole.

When two adjacent substituents together with the aromatic group to which they are attached form a 4-8 membered ring, which optionally contains one or two heteroatoms, then a ring system is formed by 4-8 atoms selected from 3-8 carbonatoms and 0-2 heteroatoms selected from N, S, or O. Such two adjacent substituents may together form:
- (CH₂)_{n"}-CH₂-, -CH=CH-(CH₂)_{m"}-, -CH₂-CH=CH-(CH₂)_{p"}-, -CH=CH-CH=CH-,
- (CH₂)_{n"}-O-, -O-(CH₂)_{m"}-O-, -CH₂-O-(CH₂)_{p"}-O-, -CH₂-O-CH₂-O-CH₂-,
- (CH₂)_{n"}-S-, -S-(CH₂)_{m"}-S-, -CH₂-S-(CH₂)_{p"}-S-, -CH₂-S-CH₂-S-CH₂-,
- (CH₂)_{n"}-NH-, -NH-(CH₂)_{m"}-NH-, -CH₂-NH-(CH₂)p_{"}-NH-, -CH=CH-NH-,
- O-(CH₂)_{m"}-NH-, -CH₂-O-(CH₂)_{p"}-NH- or -O-(CH₂)_{p"}-NH-CH₂-, -S-(CH₂)_{m"}-NH-, - N=CH-NH-, -N=CH-O- or -N=CH-S-, wherein m" is 1, 2 or 3, n" is 2, 3 or 4 and p" is 1 or 2.

The term Ar refers to optionally substituted aromatic systems of 5-10 carbon atoms, wherein 0, 1, 2, 3 or 4 carbon atoms may be replaced by heteroatoms independently selected from N, S, or O. Examples of such Ar groups are optionally substituted phenyl, optionally substituted naphtyl, optionally substituted pyridine, optionally substituted pyrrole, optionally substituted pyrimidine, optionally substituted quinoline, optionally substituted indole, optionally substituted thiophene, optionally substituted furan, optionally substituted thiazole and optionally substituted oxazole. Ar may be substituted with one or more substituents independently being hydroxy, halogen, C₁₋₆-alk(en/yn)yl, C₃₋₈-cycloalk(en)yl, C₃₋₈-cycloalk(en)yl-C₁₋₆-alk(en/yn)yl, halo-C₁₋₆-alk(en/yn)yl, C₁₋₆-alk(en/yn)yloxy, C₃₋₈-alk(en/yn)yloxy, acyl, nitro or cyano, -CO-NH-C₁₋₆-alk(en/yn)yl, -CO-N(C₁₋₆-alk(en/yl)yl)₂, -NH₂, -NH-C₁₋₆-alk(en/yn)yl, -N(C₁₋₆-alk(en/yn)yl)₂, -S- C₁₋₆-alk(en/yn)yl, -SO₂-C₁₋₆-alk(en/yn)yl, - SO₂N(C₁₋₆-alk(en/yn)yl)₂ and -SO₂NH-C₁₋₆-alk(en/yn)yl; or two adjacent substituents may together with the aromatic group to which they are attached form a 4-8 membered ring, which optionally contains one or two heteroatoms and which may be saturated or unsaturated.

The terms C₃₋₈-cycloalk(en)yl-C₁₋₆-alk(en/yn)yl, C₁₋₆-alk(en/yn)yl-C₃₋₈-cycloalk(en)yl, C₁₋₆-alk(en/yn)yl-heterocycloalk(en)yl, Ar, Ar-C₁₋₆-alk(en/yn)yl, Ar-C₃₋₈-cycloalk(en)yl, Ar-heterocycloalk(en)yl, Ar-C₃₋₈-cycloalk(en)yl-C₁₋₆-alk(en/yn)yl, ArC₁₋₆-alk(en/yn)yl-C₃₋₈-cycloalk(en)yl, Ar-C₁₋₆-alk(en/yn)yl-heterocycloalk(en)yl, C₁₋₆-alk(en/yn)yloxy, C₂₋₆-alkenyloxy, C₂₋₆-alkynyloxy, C₃₋₈-cycloalk(en)yloxy, C₁₋₆-alk(en/yn)yloxy-C₁₋₆-alk(en/yn)yl, C₃₋₈-cycloalk(en)yloxy-C₁₋₆-alk(en/yn)yl, C₁₋₆-alk(en/yn)yloxy-C₃₋₈-cycloalk(en)yl, C₁₋₆-alk(en/yn)yloxy-heterocycloalk(en)yl, Ar-oxy-C₁₋₆-alk(en/yn)yl, Ar-C₁₋₆-alk(en/yn)yloxy-C₁₋₆-alk(en/yn)yl, C₁₋₆-alk(en/yn)ylcarbonyl, C₃₋₈-alk(en/yn)ylcarbonyl, Ar-carbonyl, Ar-C₁₋₆-alk(en/yn)ylcarbonyl, C₃₋₈-cycloalk(en)yl-C₁₋₆-alk(en/yn)ylcarbonyl, -CO-C₁₋₆-alk(en/yn)yl, -S-C₁₋₆-alk(en/yn)yl, -SO₂-C₁₋₆-alk(en/yn)yl and -SO₂O-C₁₋₆-alk(en/yn)yl, C₁₋₆-alk(en/yn)yloxy-carbonyl-C₁₋₆-alk(en/yn)yl, C₃₋₈-cycloalk(en)yloxy-carbonyl-C₁₋₆-alk(en/yn)yl, C₃₋₈-cycloalk(en)yl-C₁₋₆-alk(en/yn)yloxy-carbonyl-C₁₋₆-alk(en/yn)yl, acyl, acyl-C₁₋₆-alk(en/yn)yl, acyl-C₃₋₈-cycloalk(en)yl, acyl-heterocycloalk(en)yl, acyl-C₃₋₈-cycloalk(en)yl-C₁₋₆-alk(en/yn)yl, acyl-C₁₋₆-alk(en/yn)yl-C₃₋₈-cycloalk(en)yl, acyl-C₁₋₆-alk(en/yn)yl-heterocycloalk(en)yl, hydroxy-C₁₋₆-alk(en/yn)yl, hydroxy-C₃₋₈-cycloalk(en)yl, hydroxy-heterocycloalk(en)yl, hydroxy-C₃₋₈-cycloalk(en)yl-C₁₋₆-alk(en/yn)yl, hydroxy-C₁₋₆-alk(en/yn)yl-C₃₋₈-cycloalk(en)yl, hydroxy-C₁₋₆-alk(en/yn)yl-heterocycloalk(en)yl, halo-C₁₋₆-alk(en/yn)yl, halo-C₃₋₈-cycloalk(en)yl, halo-heterocycloalk(en)yl, halo-C₃₋₈-cycloalk(en)yl-C₁₋₆-alk(en/yn)yl, halo-C₁₋₆-alk(en/yn)yl-C₃₋₈-cycloalk(en)yl, halo-C₁₋₆-alk(en/yn)yl-heterocycloalk(en)yl, halo-C₁₋₆-alk(en/yn)yl-Ar, halo-C₃₋₈-cycloalk(en)yl-Ar, halo-C₃₋₈-cycloalk(en)yl-C₁₋₆-alk(en/yn)yl-Ar, halo-C₁₋₆-alk(en/yn)yl-C₃₋₈-cycloalk(en)yl-Ar, halo-heterocycloalk(en)yl-Ar, cyano-C₁₋₆-alk(en/yn)yl, cyano-C₃₋₈-cycloalk(en)yl, cyano-heterocycloalk(en)yl, cyano-C₃₋₈-cycloalk(en)yl-C₁₋₆-alk(en/yn)yl, cyano-C₁₋₆-alk(en/yn)yl-C₃₋₈-cycloalk(en)yl, cyano-C₁₋₆-alk(en/yn)yl-heterocycloalk(en)yl etc. designate such groups in which the C₁₋₆-alk(en/yn)yl, C₂₋₆-alkenyl, C₂₋₆-alkynyl, C₃₋₈-cycloalk(en)yl, heterocycloalk(en)yl, Ar, cyano, halo-C₁₋₆-alk(en/yn)yl, halo-C₃₋₈-cycloalk(en)yl, halo-heterocycloalk(en)yl and acyl are as defmed above.

In another embodiment of the present invention, the following definitions are applied for formula 5:
The term heteroatom refers to a nitrogen, oxygen or sulphur atom.

Halogen means fluoro, chloro, bromo or iodo.

The expression C₁₋₆-alk(en/yn)yl means a C₁₋₆-alkyl, C₂₋₆-alkenyl or a C₂₋₆-alkynyl group. The term C₁₋₆-alkyl refers to a branched or un-branched alkyl group having from one to six carbon atoms inclusive, including but not limited to methyl, ethyl, 1-propyl, 2-propyl, 1-butyl, 2-butyl, 2-methyl-2-propyl and 2-methyl-1-propyl. Similarly, C₂₋₆-alkenyl and C₂₋₆-alkynyl, respectively, designate such groups having from two to six carbon atoms, including one double bond and one triple bond respectively, including but not limited to ethenyl, propenyl, butenyl, ethynyl, propynyl and butynyl.

The expression C₁₋₁₀-alk(en/yn)yl means a C₁₋₁₀-alkyl, C₂₋₁₀-alkenyl or a C₂₋₁₀-alkynyl group. The term C₁₋₁₀-alkyl refers to a branched or un-branched alkyl group having from one to six carbon atoms inclusive, including but not limited to methyl, ethyl, 1-propyl, 2-propyl, 1-butyl, 2-butyl, 1-pentyl, 1-hexyl, 1-heptyl, 1-octyl, 1-nonyl, 1-decyl, 2-methyl-2-propyl and 2-methyl-1-propyl. Similarly, C₂₋₁₀-alkenyl and C₂₋₁₀-alkynyl, respectively, designate such groups having from two to six carbon atoms, including one double bond and one triple bond respectively, including but not limited to ethenyl, propenyl, butenyl, pentenyl, hexenyl, heptenyl, octenyl, nonenyl, decenyl, ethynyl; propynyl, butynyl, pentynyl, hexynyl, heptynyl, octynyl, nonynyl, and decynyl.

The expression C₃₋₈-cycloalk(en)yl means a C₃₋₈-cycloalkyl- or cycloalkenyl group. The term C₃₋₈-cycloalkyl designates a monocyclic or bicyclic carbocycle having three to eight C-atoms, including but not limited to cyclopropyl, cyclopentyl, cyclohexyl, cycloheptyl, cyclooctyl, [1.1.1]bicyclopentyl, bicyclo[2.2.1]heptyl, [2.2.2]bicyclooctyl and [3.3.0]bicyclooctyl, etc. The term C₃₋₈-cycloalkenyl designates a monocyclic or bicyclic carbocycle having three to eight C-atoms and including one double bond.

The term halo-C₁₋₆-alk(en/yn)yl designates C₁₋₆-alk(en/yn)yl being substituted with one or more halogen atoms, including but not limited to trifluoromethyl.

Similarly, halo-C₃₋₈-cycloalk(en)yl designates C₃₋₈-cycloalk(en)yl being substituted with one or more halogen atoms.

In the expression halo-C₃₋₈-cycloalk(en)yl-C₁₋₆-alk(en/yn)yl the terms C₁₋₆-alk(en/yn)yl and halo-C₃₋₈-cycloalk(en)yl are as defined above.

When two adjacent substituents together with the aromatic group to which they are attached form a 4-8 membered ring, which optionally contains one, two or three heteroatoms, then a ring system is formed by 4-8 atoms selected from 4-8 carbonatoms and 0-3 heteroatoms selected from N, S, or O. Such two adjacent substituents may together form:
- (CH₂)ₐ-CH₂-, -CH=CH-(CH₂)_{b}-, -CH₂-CH=CH-(CH₂)_{c}, -CH=CH-CH=CH-,
- (CH₂)ₐ-O-, -O-(CH₂)_{b}-O-, -CH₂-O-(CH₂)_{c}-O-, -CH₂-O-CH₂-O-CH₂-,
- (CH₂)ₐ-S-, -S-(CH₂)_{b}-S-, -CH₂-S-(CH₂)_{c}-S-, -CH₂-S-CH₂-S-CH₂-,
- (CH₂)ₐ-NH-, -NH-(CH₂)_{b}-NH-, -CH₂-NH-(CH₂)_{c}-NH-, -CH=CH-NH-,
- O-(CH₂)_{b}-NH-, -CH₂-O-(CH₂)_{c}-NH- or -O-(CH₂)_{c}-NH-CH₂-, -S-(CH₂)_{b}-NH-,
- N=CH-NH-, -N=CH-O- or -N=CH-S- or -N=N-NH-,
   wherein b is 1, 2 or 3, a is 2, 3 or 4 and c is 1 or 2.

The term Ar refers to optionally substituted aromatic systems of 5-10 carbon atoms, wherein 0, 1, 2, 3 or 4 carbon atoms may be replaced by heteroatoms independently selected from N, S, or O. Examples of such Ar groups are optionally substituted phenyl, optionally substituted naphthyl, optionally substituted pyridine, optionally substituted thiophene, optionally substituted furan, optionally substituted thiazole, optionally substituted quinoline, optionally substituted indole, optionally substituted 2,3-dihydro-benzofuran, optionally substituted pyrimidine, optionally substituted pyrrole and optionally substituted oxazole. Ar may be substituted with one or more substituents independently being hydroxy, halogen, C₁₋₆-alk(en/yn)yl, C₃₋₈-cycloalk(en)yl, C₃₋₈-cycloalk(en)yl-C₁₋₆-alk(en/yn)yl, halo-C₁₋₆-alk(en/yn)yl, C₁₋₆-alk(en/yn)yloxy, C₃₋₈-alk(en/yn)yloxy, acyl, nitro or cyano, -CO-NH-C₁₋₆-alk(en/yn)yl, -CO-N(C₁₋₆-alk(en/yn)yl)₂, -NH₂, -NH-C₁₋₆-alk(en/yn)yl, -N(C₁₋₆-alk(en/yn)yl)₂, -S-C₁₋₆-alk(en/yn)yl, -SO₂-C₁₋₆-alk(en/yn)yl, -SO₂N(C₁₋₆-alk(en/yn)yl)₂ and -SO₂NH-C₁₋₆-alk(en/yn)yl; or two adjacent substituents may together with the aromatic group to which they are attached form a 4-8 membered ring, which optionally contains one, two or three heteroatoms. When Ar is substituted with CO-NH-C₁₋₆-alk(en/yn)yl or CO-N(C₁₋₆-alk(en/yn)yl)₂, then the carbon atom of the CO group is attached to Ar. When Ar is substituted with NH₂, NH-C₁₋₆-alk(en/yn)yl or N(C₁₋₆-alk(en/yn)yl)₂, then the nitrogen atom is attached to Ar. When Ar is substituted with -S-C₁₋₆-alk(en/yn)yl, -SO₂-C₁₋₆-alk(en/yn)yl, -SO₂N(C₁₋₆-alk(en/yn)yl)₂ or -SO₂NH-C₁₋₆-alk(en/yn)yl then the sulphur atom is attached to Ar.

The term acyl refers to formyl, C₁₋₆-alk(en/yn)ylcarbonyl,
C₃₋₈-cycloalk(en)ylcarbonyl, Ar-carbonyl, Ar-C₁₋₆-alk(en/yn)ylcarbonyl or a C₃₋₈-cycloalk(en)yl-C₁₋₆-alk(en/yn)yl-carbonyl group, wherein C₁₋₆-alk(en/yn)yl, C₃₋₈-cycloalk(en)yl and Ar are as defined above.

The terms C₃₋₈-cycloalk(en)yl-C₁₋₆-alk(en/yn)yl, Ar-C₁₋₆-alk(en/yn)yl, C₁₋₆-alk(en/yn)yloxy and C₃₋₈-cycloalk(en)yloxy; designate such groups in which the C₁₋₆-alk(en/yn)yl, C₃₋₈-cycloalk(en)yl and Ar are as defined above.

Similarly, C₃₋₈-cycloalk(en)yl-C₁₋₆-alk(en/yn)yloxy designate such groups in which C₃₋₈-cycloalk(en)yl and C₁₋₆-alk(en/yn)yloxy are as defined above.

The expressions Ar-C₃₋₈-cycloalk(en)yl and Ar-C₃₋₈-cycloalk(en)yl-C₁₋₆-alk(en/yn)yl designate such groups in which the C₁₋₆-alk(en/yn)yl, C₃₋₈-cycloalk(en)yl and Ar are as defined above.

In another embodiment of the present invention, the following definitions are applied for formula 6:
The term heteroatom refers to a nitrogen, oxygen or sulphur atom.

Halogen means fluoro, chloro, bromo or iodo.

Amino means NH₂.

The expression "C₁₋₆-alk(en/yn)yl" means C₁₋₆-alkyl, C₂₋₆-alkenyl or C₂₋₆-alkynyl. The term "C₁₋₆-alkyl" refers to a branched or unbranched alkyl group having from one to six carbon atoms inclusive, including but not limited to methyl, ethyl, prop-1-yl, prop-2-yl, 2-methyl-prop-1-yl, 2-methyl-prop-2-yl, 2,2-dimethyl-prop-1-yl, but-1-yl, but-2-yl, 3-methyl-but-1-yl, 3-methyl-but-2-yl, pent-1-yl, pent-2-yl, pent-3-yl, hex-1-yl, hex-2-yl and hex-3-yl. The term "C₂₋₆-alkenyl" designates such groups having from two to six carbon atoms and one double bond, including but not limited to ethenyl, propenyl, and butenyl. The term "C₂₋₆-alkynyl" designates such groups having from two to six carbon atoms and one triple bond, including but not limited to ethynyl, propynyl and butynyl.

The expression "C₁₋₈-alk(en/yn)yl" means C₁₋₈-alkyl, C₂₋₈-alkenyl or C₂₋₈-alkynyl. The term "C₁₋₈-alkyl" refers to a branched or unbranched alkyl group having from one to eight carbon atoms inclusive, including but not limited to methyl, ethyl, prop-1-yl, prop-2-yl, 2-methyl-prop-1-yl, 2-methyl-prop-2-yl, 2,2-dimethyl-prop-1-yl, but-1-yl, but-2-yl, 3-methyl-but-1-yl, 3-methyl-but-2-yl, pent-1-yl, pent-2-yl, pent-3-yl, hex-1-yl, hex-2-yl and hex-3-yl, 1-heptyl, 2-heptyl, 3-heptyl and 4-heptyl. The term "C₂₋₈-alkenyl" designates such groups having from two to eight carbon atoms and one double bond, including but not limited to ethenyl, propenyl, and butenyl. The term "C₂₋₈-alkynyl" designates such groups having from two to eight carbon atoms and one triple bond, including but not limited to ethynyl, propynyl and butynyl.

The expression "C₃₋₈-cycloalk(en)yl" means C₃₋₈-cycloalkyl or C₃₋₈-cycloalkenyl. The term "C₃₋₈-cycloalkyl" designates a monocyclic or bicyclic carbocycle having three to eight C-atoms, including but not limited to cyclopropyl, cyclopentyl, cyclohexyl, bicycloheptyl such as 2-bicyclo[2.2.1]heptyl. The term "C₃₋₈-cycloalkenyl" designates a monocyclic or bicyclic carbocycle having three to eight C-atoms and one double bond, including but not limited to cyclopropenyl, cyclopentenyl and cyclohexenyl.

The term "C₃₋₈-heterocycloalk(en)yl" means C₃₋₈-heterocycloalkyl or C₃₋₈-heterocycloalkenyl. The term "C₃₋₈-heterocycloalkyl" designates a monocyclic or bicyclic ring system wherein the ring is formed by 3 to 8 atoms selected from 2-7 carbon atoms and 1 or 2 heteroatoms independently selected from N, S, or O. Examples of C₃₋₈-heterocycloalkyles are pyrrolidine, azepan, morpholine and piperidine. The term "C₃₋₈-heterocycloalkenyl" designates a monocyclic or bicyclic ring system with one double bond, wherein the ring is formed by 3 to 8 atoms selected from 2-7 carbon atoms and 1 or 2 heteroatoms independently selected from N, S, or O.

The term Aryl refers to monocyclic or bicyclic aromatic systems of 5-10 carbon atoms, including but not limited to phenyl and naphthyl. Any Aryl which is mentioned either alone or as a part of a larger substituent is optionally substituted and may thus be substituted with one or more substituents such as with 0, 1, 2, 3 or 4 substituents. Any Aryl which is mentioned either alone or as a part of a larger substituent may thus be substituted with one or more substituents independently selected from the group consisting of amino, halogen, cyano, C₁₋₆-alk(en/yn)yl, C₃₋₈-cycloalk(en)yl, C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, C₃₋₈-heterocycloalk(en)yl, C₁₋₆-alkyl-C₃₋₈-heterocycloalk(en)yl, hydroxy, C₁₋₆-alk(en/yn)yloxy, C₃₋₈-cycloalk(en)yloxy, C₃₋₈-cycloalk(en)yl-C₁₋₆-alk(en/yn)yloxy, halo-C₁₋₆-alk(en/yn)yloxy, halo-C₃₋₈-cycloalk(en)yloxy, halo-C₃₋₈-cycloalk(en)yl-C₁₋₆-alk(en/yn)yloxy, C₁₋₆-alk(en/yn)ylamino, di-(C₁₋₆-alk(en/yn)yl)amino, C₁₋₆-alk(en/yn)yl-CO-NH- and C₁₋₆-alk(en/yn)yl-sulfonamide; or two adjacent substituents may together with the Aryl group to which they are attached form a 4-8 membered ring, which optionally contains one or two heteroatoms and which is optionally substituted with one or more C₁₋₆-alk(en/yn)yl groups. When two adjacent substituents together with the Aryl group to which they are attached form a 4-8 membered ring, which optionally contains one or two heteroatoms, then a ring system is formed by 4-8 atoms selected from 3-8 carbon atoms and 0-2 heteroatoms independently selected from N, S, or O. Such two adjacent substituents may together form: -(CH₂)ₙ-O-, -O-(CH₂),-O-, -CH₂-O-(CH₂)p-O-, -CH₂-O-CH₂-O-CH₂-, -O-C(CH₃)₂-(CH₂)ₘ-, -(CH₂)ₙ-S-, -S-(CH₂)ₘ-S-, -CH₂-S-(CH₂)ₚ-S- or -CH₂-S-CH₂-S-CH₂-, -S-C(CH₃)₂-(CH₂)ₘ-; wherein m is 1, 2 or 3, n is 2, 3 or 4 and p is 1 or 2.

The term "Heteroaryl" refers to monocyclic or bicyclic heteroaromatic systems of 5-10 atoms selected from 1, 2, 3, 4, 5, 6, 7, 8 or 9 carbon atoms and 1, 2, 3 or 4 heteroatoms independently selected from N, S, or O, including but not limited to pyridine, pyrrole, pyrimidine, quinoline, indole, thiophene, furan, imidazoles such as 3H-imidazol and 1H-imidazol, triazoles such as [1,2,3]triazole and [1,2,4]triazole, tetrazoles such as 2H-tetrazole and oxazole. Any Heteroaryl which is mentioned either alone or as a part of a larger substituent is optionally substituted and may thus be substituted with one or more substituents such as with 0, 1, 2, 3 or 4 substituents. Any Heteroaryl which is mentioned either alone or as a part of a larger substituent may thus be substituted with one or more substituents independently selected from the group consisting of halogen, cyano, amino, halo-C₁₋₆-alk(en/yn)yl, halo-C₃₋₈-cycloalk(en)yl, halo-C₃₋₈-cycloalk(en)yl-C₁₋₆-alk(en/yn)yl, C₁₋₆-alk(en/yn)yl, C₃₋₈-cycloalk(en)yl, C₃₋₈-cycloalk(en)yl-C₁₋₆-alk(en/yn)yl, Aryl, Aryl-C₁₋₆-alk(en/yn)yl, C₁₋₆-alk(en/yn)yloxy, C₃₋₈-cycloalk(en)yloxy, C₃₋₈-cycloalk(en)yl-C₁₋₆-alk(en/yn)yloxy, C₁₋₆-alk(en/yn)yl-phenoxy, C₃₋₈-cycloalk(en)yl-phenoxy, C₃₋₈-cycloalk(en)yl-C₁₋₆-alk(en/yn)yl-phenoxy, amino-phenoxy, halo-phenoxy, cyano-phenoxy, halo-C₁₋₆-alk(en/yn)yl-phenoxy, halo-C₃₋₈-cycloalk(en)yl-phenoxy, halo-C₃₋₈-cycloalk(en)yl-C₁₋₆-alk(en/yn)yl-phenoxy, C₃₋₈-heterocycloalk(en)yl-phenoxy, C₁₋₆-alkyl-C₃₋₈-heterocycloalk(en)yl-phenoxy, hydroxy-phenoxy, C₁₋₆-alk(en/yn)yloxy-phenoxy, C₃₋₈-cycloalk(en)yloxy-phenoxy, C₃₋₈-cycloalk(en)yl-C₁₋₆-alk(en/yn)yloxy-phenoxy, halo-C₁₋₆-alk(en/yn)yloxy-phenoxy, halo-C₃₋₈-cycloalk(en)yloxy-phenoxy, halo-C₃₋₈-cycloalk(en)yl-C₁₋₆-alk(en/yn)yloxy-phenoxy, C₁₋₆-alk(en/yn)ylamino-phenoxy, di-(C₁₋₆-alk(en/yn)yl)amino-phenoxy, C₁₋₆-alk(en/yn)yl-CO-NH-phenoxy and C₁₋₆-alk(en/yn)yl-sulfonamide-phenoxy.

The term "halo-C₁₋₆-alk(en/yn)yl" designates C₁₋₆-alk(en/yn)yl being substituted with one or more halogen atoms, including but not limited to trifluoromethyl and 3,3,3-trifluoro-1-propyl. Similarly, halo-C₃₋₈-cycloalk(en)yl designates C₃₋₈-cycloalk(en)yl being substituted with one or more halogen atoms and "halo-phenoxy" designates phenoxy being substituted with one or more halogen atoms.

The term "amino-C₁₋₆-alk(en/yn)yl" designates C₁₋₆-alk(en/yn)yl being substituted with one amino group, including but not limited to 1-amino-2-methyl-prop-1-yl and 1-amino-3-methyl-but-1-yl. Similarly, amino-C₃₋₈-cycloalk(en)yl designates C₃₋₈-cycloalk(en)yl being substituted with one amino group and amino-C₃₋₈-cycloalk(en)yl-C₁₋₆-alk(en/yn)yl designates C₃₋₈-cycloalk(en)yl-C₁₋₆-alk(en/yn)ylbeing wherein C₃₋₈-cycloalk(en)yl is substituted with one amino group.

In the expressions C₃₋₈-cycloalk(en)yl-C₁₋₆-alk(en/yn)yl, C₃₋₈-heterocycloalk(en)yl-C₁₋₆-alk(en/yn)yl, C₁₋₆-alk(en/yn)yl-C₃₋₈-heterocycloalk(en)yl-C₁₋₆-alk(en/yn)yl, Aryl-C₁₋₆-alk(en/yn)yl, Aryl-C₃₋₈-cycloalk(en)yl, Aryl-C₃₋₈-cycloalk(en)yl-C₁₋₆-alk(en/yn)yl, Aryl-C₃₋₈-heterocycloalk(en)yl, Heteroaryl-C₁₋₆-alk(en/yn)yl, Heteroaryl-C₃₋₈-cycloalk(en)yl, Heteroaryl-C₃₋₈-cycloalk(en)yl-C₁₋₆-alk(en/yn)yl, halo-C₃₋₈-cycloalk(en)yl-C₁₋₆-alk(en/yn)yl, halo-C₁₋₆-alk(en/yn)yl-C₃₋₈-heterocycloalk(en)yl-C₁₋₆-alk(en/yn)yl, C₁₋₆-alk(en/yn)yloxy, C₃₋₈-cycloalk(en)yloxy, C₃₋₈-cycloalk(en)yl-C₁₋₆-alk(en/yn)yloxy, C₃₋₈-heterocycloalk(en)yloxy, C₁₋₆-alk(en/yn)yloxy-C₁₋₆-alk(en/yn)yl, C₃₋₈-cycloalk(en)yloxy-C₁₋₆-alk(en/yn)yl, C₃₋₈-cycloalk(en)yl-C₁₋₆-alk(en/yn)yloxy-C₁₋₆-alk(en/yn)yl, halo-C₁₋₆-alk(en/yn)yloxy, halo-C₃₋₈-cycloalk(en)yloxy, halo-C₃₋₈-cycloalk(en)yl-C₁₋₆-alk(en/yn)yloxy, amino-C₁₋₆-alk(en/yn)yl, amino-C₃₋₈-cycloalk(en)yl, amino-C₃₋₈-cycloalk(en)yl-C₁₋₆-alk(en/yn)yl, R⁷NH-C₁₋₆-alk(en/yn)yl, C₁₋₆-alk(en/yn)ylamino, di-(C₁₋₆-alk(en/yn)yl)amino, C₁₋₆-alk(en/yn)yl-CO-NH-, C₁₋₆-alk(en/yn)yl-sulfonamide C₁₋₆-alk(en/yn)yl-phenoxy, C₃₋₈-cycloalk(en)yl-phenoxy, C₃₋₈-cycloalk(en)yl-C₁₋₆-alk(en/yn)yl-phenoxy, halo-phenoxy, halo-C₁₋₆-alk(en/yn)yl-phenoxy, halo-C₃₋₈-cycloalk(en)yl-phenoxy, halo-C₃₋₈-cycloalk(en)yl-C₁₋₆-alk(en/yn)yl-phenoxy, C₃₋₈-heterocycloalk(en)yl-phenoxy, C₁₋₆-alkyl-C₃₋₈-heterocycloalk(en)yl-phenoxy, C₁₋₆-alk(en/yn)yloxy-phenoxy, C₃₋₈-cycloalk(en)yloxy-phenoxy, C₃₋₈-cycloalk(en)yl-C₁₋₆-alk(en/yn)yloxy-phenoxy, halo-C₁₋₆-alk(en/yn)yloxy-phenoxy, halo-C₃₋₈-cycloalk(en)yloxy-phenoxy, halo-C₃₋₈-cycloalk(en)yl-C₁₋₆-alk(en/yn)yloxy-phenoxy, C₁₋₆-alk(en/yn)ylamino-phenoxy, di-(C₁₋₆-alk(en/yn)yl)amino-phenoxy, C₁₋₆-alk(en/yn)yl-CO-NH-phenoxy and C₁₋₆-alk(en/yn)yl-sulfonamide-phenoxy the terms "C₁₋₆-alk(en/yn)yl", "C₃₋₈-cycloalk(en)yl", "C₃₋₈-heterocycloalk(en)yl", "Aryl", "Heteroaryl", "halo-C₁₋₆-alk(en/yn)yl", "halo-C₃₋₈-cycloalk(en)yl", "halo-phenoxy", "amino-C₁₋₆-alk(en/yn)yl", "amino-C₃₋₈-cycloalk(en)yl" and "amino-C₃₋₈-cycloalk(en)yl-C₁₋₆-alk(en/yn)yl" are as defined above.

Any C₁₋₆-alk(en/yn)yl which is mentioned either alone or as a part of a larger substituent independently contains 1, 2, 3, 4, 5 or 6 carbon atoms.

Any C₁₋₈-alk(en/yn)yl which is mentioned either alone or as a part of a larger substituent independently contains 1, 2, 3, 4, 5, 6, 7 or 8 carbon atoms.

Any C₃₋₈-cycloalk(en)yl which is mentioned either alone or as a part of a larger substituent independently contains 3, 4, 5, 6, 7 or 8 carbon atoms.

Any C₃₋₈-heterocycloalk(en)yl which is mentioned either alone or as a part of a larger substituent independently contains 2, 3, 4, 5, 6 or 7 carbon atoms and 1 or 2 heteroatoms.

Any Aryl which is mentioned either alone or as a part of a larger substituent independently contains 5, 6, 7, 8, 9 or 10 carbon atoms.

Any Heteroaryl which is mentioned either alone or as a part of a larger substituent independently contains 5, 6, 7, 8, 9 or 10 atoms selected from 1, 2, 3, 4, 5, 6, 7, 8 or 9 carbon atoms and 1, 2, 3 or 4 heteroatoms.

In another embodiment of the present invention, the following definitions are applied for formula 7:
The term "heteroatom" refers to a nitrogen, oxygen or sulphur atom.

"Halogen" means fluoro, chloro, bromo or iodo. "Halo" means halogen.

"Cyano" designates
C≡N
which is attached to the remainder of the molecule via the carbon atom.

The expression "C₁₋₆-alk(en/yn)yl" means C₁₋₆-alkyl, C₂₋₆-alkenyl or C₂₋₆-alkynyl. The term "C₁₋₆-alkyl" refers to a branched or unbranched alkyl group having from one to six carbon atoms, including but not limited to methyl, ethyl, prop-1-yl, prop-2-yl, 2-methyl-prop-1-yl, 2-methyl-prop-2-yl, 2,2-dimethyl-prop-1-yl, but-1-yl, but-2-yl, 3-methyl-but-1-yl, 3-methyl-but-2-yl, pent-1-yl, pent-2-yl, pent-3-yl, hex-1-yl, hex-2-yl and hex-3-yl.

The term "C₂₋₆-alkenyl" refers to a branched or unbranched alkenyl group having from two to six carbon atoms and one double bond, including but not limited to ethenyl, propenyl, and butenyl.

The term "C₂₋₆-alkynyl" refers to a branched or unbranched alkynyl group having from two to six carbon atoms and one triple bond, including but not limited to ethynyl, propynyl and butynyl.

The expression "C₁₋₈-alk(en/yn)yl" means C₁₋₈-alkyl, C₂₋₈-alkenyl or C₂₋₈-alkynyl. The term "C₁₋₈-alkyl" refers to a branched or unbranched alkyl group having from one to eight carbon atoms, including but not limited to methyl, ethyl, prop-1-yl, prop-2-yl, 2-methyl-prop-1-yl, 2-methyl-prop-2-yl, 2,2-dimethyl-prop-1-yl, but-1-yl, but-2-yl, 3-methyl-but-1-yl, 3-methyl-but-2-yl, pent-1-yl, pent-2-yl, pent-3-yl, hex-1-yl, hex-2-yl, hex-3-yl, 2-methyl-4,4-dimethyl-pent-1-yl and hept-1-yl.

The term "C₂₋₈-alkenyl" refers to a branched or unbranched alkenyl group having from two to eight carbon atoms and one double bond, including but not limited to ethenyl, propenyl, and butenyl.

The term "C₂₋₈-alkynyl" refers to a branched or unbranched alkynyl group having from two to eight carbon atoms and one triple bond, including but not limited to ethynyl, propynyl and butynyl.

The expression "C₃₋₈-cycloalk(en)yl" means C₃₋₈-cycloalkyl or C₃₋₈-cycloalkenyl. The term "C₃₋₈-cycloalkyl" designates a monocyclic or bicyclic carbocycle having three to eight carbon atoms, including but not limited to cyclopropyl, cyclopentyl, cyclohexyl, bicycloheptyl such as 2-bicyclo[2.2.1]heptyl.

The term "C₃₋₈-cycloalkenyl" designates a monocyclic or bicyclic carbocycle having three to eight carbon atoms and one double bond, including but not limited to cyclopentenyl and cyclohexenyl.

The term "C₃₋₈-heterocycloalk(en)yl" means C₃₋₈-heterocycloalkyl or C₃₋₈-heterocycloalkenyl.

The term "C₃-₈-heterocycloalkyl" designates a monocyclic or bicyclic ring system wherein the ring is formed by 3 to 8 atoms selected from 2-7 carbon atoms and 1 or 2 heteroatoms independently selected from nitrogen, oxygen and sulphur atoms. Examples of C₃₋₈-heterocycloalkyls are pyrrolidine, azepan, morpholine, piperidine, piperazine and tetrahydrofuran.

The term "C₃₋₈-heterocycloalkenyl" designates a monocyclic or bicyclic ring system with one double bond, wherein the ring is formed by 3 to 8 atoms selected from 2-7 carbon atoms and 1 or 2 heteroatoms independently selected from nitrogen, oxygen and sulphur atoms. Examples of C₃₋₈-heterocycloalkenyls are dihydropyrrole, dihydrofuran and dihydrothiophene.

When C₃₋₈-heterocycloalk(en)yl comprises nitrogen then C₃₋₈-heterocycloalk(en)yl is attached to the remainder of the molecule via a carbon atom or nitrogen atom of the heterocyclic ring.

When C₃₋₈-heterocycloalk(en)yl does not comprise nitrogen then C₃₋₈-heterocycloalk(en)yl is attached to the remainder of the molecule via a carbon atom of the heterocyclic ring.

The term "halo-C₁₋₆-alk(en/yn)yl" designates C₁₋₆-alk(en/yn)yl being substituted with halogen, including but not limited to trifluoromethyl.

Similarly, "halo-C₃₋₈-cycloalk(en)yl" designates C₃₋₈-cycloalk(en)yl being substituted with halogen, including but not limited to chlorocyclopropane and chlorocyclohexane. Similarly, "halo-C₃₋₈-cycloalk(en)yl-C₁₋₆-alk(en/yn)yl" designates halo-C₃₋₈-cycloalk(en)yl being attached to the remainder of the molecule via C₁₋₆-alk(en/yn)yl.

The term "C₁₋₆-alk(en/yn)yloxy" designates C₁₋₆-alk(en/yn)yl being attached to the remainder of the molecule via an oxygen atom.

Similarly, "C₃₋₈-cycloalk(en)yloxy" designates C₃₋₈-cycloalk(en)yl being attached to the remainder of the molecule via an oxygen atom.

In the expressions "C₃₋₈-cycloalk(en)yl-C₁₋₆-alk(en/yn)yl", "Aryl-C₁₋₆-alk(en/yn)yl", "Aryl-C₃₋₈-cycloalk(en)yl", "Aryl-C₃₋₈-cycloalk(en)yl-C₁₋₆-alk(en/yn)yl", "C₃₋₈-heterocycloalk(en)yl-C₁₋₆-alk(en/yn)yl", "C₁₋₆-alk(en/yn)yl-C₃₋₈-heterocycloalk(en)yl-C₁₋₆-alk(en/yn)yl", "Heteroaryl-C₁₋₆-alk(en/yn)yl", "Heteroaryl-C₃₋₈-cycloalk(en)yl", "Heteroaryl-C₃₋₈-cycloalk(en)yl-C₁₋₆-alk(en/yn)yl", "NR⁴R⁵-C₁₋₆-alk(en/yn)yl", "NR⁴R⁵-C₃₋₈-cycloalk(en)yl", "NR⁴R⁵-C₃₋₈-cycloalk(en)yl-C₁₋₆-alk(en/yn)yl", "C₃₋₈-cycloalk(en)yl-C₁₋₆-alk(en/yn)yloxy", "C₁₋₆-alk(en/yn)yloxy-C₁₋₆-alk(en/yn)yl", "C₃₋₈-cycloalk(en)yloxy-C₁₋₆-alk(en/yn)yl" and "C₃₋₈-cycloalk(en)yl-C₁₋₆-alk(en/yn)yloxy-C₁₋₆-alk(en/yn)yl" the terms "C₁₋₆-alk(en/yn)yl", "C₃₋₈-cycloalk(en)yl", "Aryl", "C₃₋₈-heterocycloalk(en)yl", "Heteroaryl", "C₁₋₆-alk(en/yn)yloxy" and "C₃₋₈-cycloalk(en)yloxy" are as defined above.

The term "Heteroaryl" refers to monocyclic or bicyclic heteroaromatic systems being selected from the group consisting of pyridine, thiophene, furan, pyrrole, pyrazole, triazole, tetrazole, oxazole, imidazole, thiazole, benzofuran, benzothiophene and indole.

The term Aryl designates monocyclic or bicyclic aromatic systems being selected from the group consisting of phenyl and naphthyl.

The term "optionally substituted Aryl-C₁₋₆-alk(en/yn)yl" designates Aryl-C₁₋₆-alk(en/yn)yl wherein the Aryl moiety is optionally substituted, such as with 1, 2 or 3 substituents independently selected from the group consisting of halogen, cyano, C₁₋₆-alk(en/yn)yl, C₃₋₈-cycloalk(en)yl, C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, C₁₋₆-alk(en/yn)yloxy, C₃₋₈-cycloalk(en)yloxy and C₃₋₈-cycloalk(en)yl-C₁₋₆-alk(en/yn)yloxy.

Similarly, "optionally substituted Aryl-C₃₋₈-cycloalk(en)yl" designates Aryl-C₃₋₈-cycloalk(en)yl wherein the Aryl moiety is optionally substituted, such as with 1, 2 or 3 substituents independently selected from the group consisting of halogen, cyano, C₁₋₆-alk(en/yn)yl, C₃₋₈-cycloalk(en)yl, C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, C₁₋₆-alk(en/yn)yloxy, C₃₋₈-cycloalk(en)yloxy and C₃₋₈-cycloalk(en)yl-C₁₋₆-alk(en/yn)yloxy.

Similarly, "optionally substituted Aryl-C₃₋₈-cycloalk(en)yl-C₁₋₆-alk(en/yn)yl" designates Aryl-C₃₋₈-cycloalk(en)yl-C₁₋₆-alk(en/yn)yl wherein the Aryl moiety is optionally substituted, such as with 1, 2 or 3 substituents independently selected from the group consisting of halogen, cyano, C₁₋₆-alk(en/yn)yl, C₃₋₈-cycloalk(en)yl, C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, C₁₋₆-alk(en/yn)yloxy, C₃₋₈-cycloalk(en)yloxy and C₃₋₈-cycloalk(en)yl-C₁₋₆-alk(en/yn)yloxy.

In another embodiment of the present invention, the following definitions are applied for formula 8:
The term "heteroatom" refers to a nitrogen, oxygen or sulphur atom.

"Halogen" means fluoro, chloro, bromo or iodo. "Halo" means halogen.

"Cyano" designates
C≡N
which is attached to the remainder of the molecule via the carbon atom.

"Amino" designates NH₂, which is attached to the remainder of the molecule via the nitrogen atom.

The expression "C₁₋₆-alk(en/yn)yl" means C₁₋₆-alkyl, C₂₋₆-alkenyl or C₂₋₆-alkynyl. The term "C₁₋₆-alkyl" refers to a branched or unbranched alkyl group having from one to six carbon atoms, including but not limited to methyl, ethyl, prop-1-yl, prop-2-yl, 2-methyl-prop-1-yl, 2-methyl-prop-2-yl, 2,2-dimethyl-prop-1-yl, but-1-yl, but-2-yl, 3-methyl-but-1-yl, 3-methyl-but-2-yl, pent-1-yl, pent-2-yl, pent-3-yl, hex-1-yl, hex-2-yl and hex-3-yl.

The term "C₂₋₆-alkenyl" refers to a branched or unbranched alkenyl group having from two to six carbon atoms and one double bond, including but not limited to ethenyl, propenyl, and butenyl.

The term "C₂₋₆-alkynyl" refers to a branched or unbranched alkynyl group having from two to six carbon atoms and one triple bond, including but not limited to ethynyl, propynyl and butynyl.

The expression "C₁₋₁₀-alk(en/yn)yl" means C₁₋₁₀-alkyl, C₂₋₁₀-alkenyl or C₂₋₁₀-alkynyl. The term "C₁₋₁₀-alkyl" refers to a branched or unbranched alkyl group having from one to ten carbon atoms, including but not limited to methyl, ethyl, prop-1-yl, prop-2-yl, 2-methyl-prop-1-yl, 2-methyl-prop-2-yl, 2,2-dimethyl-prop-1-yl, but-1-yl, but-2-yl, 3-methyl-but-1-yl, 3-methyl-but-2-yl, pent-1-yl, pent-2-yl, pent-3-yl, hex-1-yl, hex-2-yl, hex-3-yl, 2-methyl-4,4-dimethyl-pent-1-yl and hept-1-yl.

The term "C₂₋₁₀-alkenyl" refers to a branched or unbranched alkenyl group having from two to ten carbon atoms and one double bond, including but not limited to ethenyl, propenyl, and butenyl.

The term "C₂₋₁₀-alkynyl" refers to a branched or unbranched alkynyl group having from two to ten carbon atoms and one triple bond, including but not limited to ethynyl, propynyl and butynyl.

The expression "C₃₋₈-cycloalk(en)yl" means C₃₋₈-cycloalkyl or C₃₋₈-cycloalkenyl. The term "C₃₋₈-cycloalkyl" designates a monocyclic or bicyclic carbocycle having three to eight carbon atoms, including but not limited to cyclopropyl, cyclopentyl, cyclohexyl, bicycloheptyl such as 2-bicyclo[2.2.1]heptyl.

The term "C₃₋₈-cycloalkenyl" designates a monocyclic or bicyclic carbocycle having three to eight carbon atoms and one double bond, including but not limited to cyclopentenyl and cyclohexenyl.

The term "halo-C₁₋₆-alk(en/yn)yl" designates C₁₋₆-alk(en/yn)yl being substituted with halogen, including but not limited to trifluoromethyl.

The term "halo-C₁₋₆-alk(en/yn)yloxy" designates C₁₋₆-alk(en/yn)yloxy being substituted with halogen, including but not limited to trifluoromethyloxy.

Similarly, "halo-C₃₋₈-cycloalk(en)yl" designates C₃₋₈-cycloalk(en)yl being substituted with halogen, including but not limited to chlorocyclopropane and chlorocyclohexane. Similarly, "halo-C₃₋₈-cycloalk(en)yloxy" designates C₃₋₈-cycloalk(en)yloxy being substituted with halogen, including but not limited to chlorocyclopropyloxy and chlorocyclohexyloxy.

Similarly, "halo-C₃₋₈-cycloalk(en)yl-C₁₋₆-alk(en/yn)yloxy" designates halo-C₃₋₈-cycloalk(en)yl being attached to the remainder of the molecule via C₁₋₆-alk(en/yn)yloxy.

The term "C₁₋₆-alk(en/yn)yloxy" designates C₁₋₆-alk(en/yn)yl being attached to the remainder of the molecule via an oxygen atom.

Similarly, "C₃₋₈-cycloalk(en)yloxy" designates C₃₋₈-cycloalk(en)yl being attached to the remainder of the molecule via an oxygen atom.

The term "aryl" designates monocyclic or bicyclic aromatic systems being selected from the group consisting of phenyl, naphthyl, thiophen, furan, benzothiophen and benzofuran.

The term "optionally substituted aryl-C₁₋₆-alk(en/yn)yl" designates aryl-C₁₋₆-alk(en/yn)yl wherein the aryl moiety is optionally substituted, such as with 1, 2 or 3 substituents independently selected from the group consisting of halogen, cyano, C₁₋₆-alk(en/yn)yl, C₃₋₈-cycloalk(en)yl, C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, C₁₋₆-alk(en/yn)yloxy, C₃₋₈-cycloalk(en)yloxy and C₃₋₈-cycloalk(en)yl-C₁₋₆-alk(en/yn)yloxy.

Similarly, "optionally substituted aryl" designates aryl wherein the aryl is optionally substituted, such as with 1, 2 or 3 substituents independently selected from the group consisting of halogen, cyano, C₁₋₆-alk(en/yn)yl, C₃₋₈-cycloalk(en)yl, C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, C₁₋₆-alk(en/yn)yloxy, C₃₋₈-cycloalk(en)yloxy and C₃₋₈-cycloalk(en)yl-C₁₋₆-alk(en/yn)yloxy.

In the expressions "C₃₋₈-cycloalk(en)yl-C₁₋₆-alk(en/yn)yl", "aryl-C₁₋₆-alk(en/yn)yl" and "C₃₋₈-cycloalk(en)yl-C₁₋₆-alk(en/yn)yloxy" the terms "C₁₋₆-alk(en/yn)yl", "C₃₋₈-cycloalk(en)yl", "aryl" and "C₁₋₆-alk(en/yn)yloxy" are as defined above.

The present invention covers any combination of the mentioned embodiments as weel as any combination of the mentioned embodiments together with any aspect of the invention.

The salts of the invention are preferably pharmaceutically acceptable salts. Such salts include pharmaceutical acceptable acid addition salts, pharmaceutically acceptable metal salts, ammonium and alkylated ammonium salts.

The pharmaceutically acceptable salts of the invention are preferably acid addition salts. The acid addition salts of the invention are preferably pharmaceutically acceptable salts of the compounds of the invention formed with non-toxic acids.Acid addition salts include salts of inorganic acids as well as organic acids. Representative examples of suitable inorganic acids include hydrochloric, hydrobromic, hydroiodic, sulfuric, sulfamic, phosphoric and nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, ethanesulfonic, tartaric, ascorbic, pamoic, gluconic, citraconic, aspartic, stearic, palmitic, EDTA, glycolic, p-aminobenzoic, glutamic, bis-methylenesalicylic, methanesulfonic, ethanedisulfonic, itaconic, benzenesulfonic, p-toluenesulfonic acids, theophylline acetic acids, as well as the 8-halotheophyllines, for example 8-bromotheophylline and the like. Further examples of pharmaceutical acceptable inorganic or organic acid addition salts include the pharmaceutically acceptable salts listed in J. Pharm. Sci. 1977,66,2, which is incorporated herein by reference.

Examples of metal salts include lithium, sodium, potassium, magnesium salts and the like.

Examples of ammonium and alkylated ammonium salts include ammonium, methyl-, dimethyl-, trimethyl-, ethyl-, hydroxyethyl-, diethyl-, n-butyl-, sec-butyl-, tert-butyl-, tetramethylammonium salts and the like.

Also intended as pharmaceutically acceptable acid addition salts are the hydrates, which the present compounds are able to form.

The compounds of the present invention may have one or more asymmetric centres and it is intended that any optical isomers, as separated, pure or partially purified optical isomers or racemic mixtures thereof are included within the scope of the invention.

Furthermore, when a double bond or a fully or partially saturated ring system is present in the molecule geometric isomers may be formed. It is intended that any geometric isomers, as separated, pure or partially purified geometric isomers or mixtures thereof are included within the scope of the invention. Likewise, molecules having a bond with restricted rotation may form geometric isomers. These are also intended to be included within the scope of the present invention.

Furthermore, some of the compounds of the present invention may exist in different tautomeric forms and it is intended that any tautomeric forms that the compounds are able to form are included within the scope of the present invention.

The compounds of this invention may exist in unsolvated as well as in solvated forms with solvents such as water, ethanol and the like. In general, the solvated forms are considered equivalent to the unsolvated forms for the purposes of this invention. Racemic forms can be resolved into the optical antipodes by known methods, for example, by separation of diastereomeric salts thereof with an optically active acid, and liberating the optically active amine compound by treatment with a base. Another method for resolving racemates into the optical antipodes is based upon chromatography on an optically active matrix. Racemic compounds of the present invention can also be resolved into their optical antipodes, e.g. by fractional crystallization of d- or 1- (tartrates, mandelates or camphorsulphonate) salts. The compounds of the present invention may also be resolved by the formation of diastereomeric derivatives.

Additional methods for the resolution of optical isomers, known to those skilled in the art, may be used. Such methods include those discussed by J. Jaques, A. Collet and S. Wilen in "Enantiomers, Racemates, and Resolutions", John Wiley and Sons, New York (1981).

Optically active compounds can also be prepared from optically active starting materials.

The invention also encompasses prodrugs of the present compounds, which on administration undergo chemical conversion by metabolic processes before becoming pharmacologically active substances.

The present invention also relates to a pharmaceutical composition. The compounds of the invention or salts thereof may be administered alone or in combination with pharmaceutically acceptable carriers or diluents, in either single or multiple doses. The pharmaceutical compositions according to the invention may be formulated with pharmaceutically acceptable carriers or diluents as well as any other known adjuvants and excipients in accordance with conventional techniques such as those disclosed in Remington: The Science and Practice of Pharmacy, 19 Edition, Gennaro, Ed., Mack Publishing Co., Easton, PA, 1995.

The pharmaceutical compositions may be specifically formulated for administration by any suitable route such as the oral, rectal, nasal, pulmonary, topical (including buccal and sublingual), transdermal, intracisternal, intraperitoneal, vaginal and parenteral (including subcutaneous, intramuscular, intrathecal, intravenous and intradermal) route, the oral route being preferred. It will be appreciated that the preferred route will depend on the general condition and age of the subject to be treated, the nature of the disorder or disease to be treated and the active ingredient chosen.

The pharmaceutical compositions formed by combining the compound of the invention and the pharmaceutical acceptable carriers are then readily administered in a variety of dosage forms suitable for the disclosed routes of administration. The formulations may conveniently be presented in unit dosage form by methods known in the art of pharmacy.

The compounds of this invention are generally utilized as the free substance or as a pharmaceutically acceptable salt thereof. One example is an acid addition salt of a compound having the utility of a free base. When a compound of the invention contains a free base such salts are prepared in a conventional manner by treating a solution or suspension of a free base of the invention with a chemical equivalent of a pharmaceutically acceptable acid. Representative examples are mentioned above.

Pharmaceutical compositions for oral administration may be solid or liquid. Solid dosage forms for oral administration include e.g. capsules, tablets, dragees, pills, lozenges, powders, granules and tablette e.g. placed in a hard gelatine capsule in powder or pellet form or e.g. in the form of a troche or lozenge. Where appropriate, pharmaceutical compositions for oral administration may be prepared with coatings such as enteric coatings or they can be formulated so as to provide controlled release of the active ingredient such as sustained or prolonged release according to methods well known in the art. Liquid dosage forms for oral administration include e.g. solutions, emulsions, suspensions, syrups and elixirs.

Formulations of the present invention suitable for oral administration may be presented as discrete units such as capsules or tablets, each containing a predetermined amount of the active ingredient, and which may include a suitable excipient. Furthermore, the orally available formulations may be in the form of a powder or granules, a solution or suspension in an aqueous or non-aqueous liquid, or an oil-in-water or water-in-oil liquid emulsion.

Suitable pharmaceutical carriers include inert solid diluents or fillers, sterile aqueous solution and various organic solvents. Examples of solid carriers are lactose, terra alba, sucrose, cyclodextrin, talc, gelatine, agar, pectin, acacia, magnesium stearate, stearic acid, lower alkyl ethers of cellulose, corn starch, potato starch, gums and the like. Examples of liquid carriers are syrup, peanut oil, olive oil, phospho lipids, fatty acids, fatty acid amines, polyoxyethylene and water.

The carrier or diluent may include any sustained release material known in the art, such as glyceryl monostearate or glyceryl distearate, alone or mixed with a wax.

Any adjuvants or additives usually used for such purposes such as colourings, flavourings, preservatives etc. may be used provided that they are compatible with the active ingredients.

The amount of solid carrier may vary but will usually be from about 25 mg to about 1 g.

If a liquid carrier is used, the preparation may be in the form of a syrup, emulsion, soft gelatine capsule or sterile injectable liquid such as an aqueous or non-aqueous liquid suspension or solution.

Tablets may be prepared by mixing the active ingredient with ordinary adjuvants or diluents and subsequently compressing the mixture in a conventional tabletting machine.

Pharmaceutical compositions for parenteral administration include sterile aqueous and nonaqueous injectable solutions, dispersions, suspensions or emulsions as well as sterile powders to be reconstituted in sterile injectable solutions or dispersions prior to use. Depot injectable formulations are also contemplated as being within the scope of the present invention.

For parenteral administration, solutions of the compound of the invention in sterile aqueous solution, aqueous propylene glycol, aqueous vitamin E or sesame or peanut oil may be employed. Such aqueous solutions should be suitably buffered if necessary and the liquid diluent first rendered isotonic with sufficient saline or glucose. The aqueous solutions are particularly suitable for intravenous, intramuscular, subcutaneous and intraperitoneal administration. The sterile aqueous media employed are all readily available by standard techniques known to those skilled in the art.

Solutions for injections may be prepared by dissolving the active ingredient and possible additives in a part of the solvent for injection, preferably sterile water, adjusting the solution to the desired volume, sterilising the solution and filling it in suitable ampoules or vials. Any suitable additive conventionally used in the art may be added, such as tonicity agents, preservatives, antioxidants, etc.

Other suitable administration forms include suppositories, sprays, ointments, cremes, gels, inhalants, dermal patches, implants, etc.

A typical oral dosage is in the range of from about 0.001 to about 100 mg/kg body weight per day, preferably from about 0.01 to about 50 mg/kg body weight per day, and more preferred from about 0.05 to about 10 mg/kg body weight per day administered in one or more dosages such as 1 to 3 dosages. The exact dosage will depend upon the frequency and mode of administration, the sex, age, weight and general condition of the subject treated, the nature and severity of the disorder or disease treated and any concomitant diseases to be treated and other factors evident to those skilled in the art.

The formulations may conveniently be presented in unit dosage form by methods known to those skilled in the art. A typical unit dosage form for oral administration one or more times per day such as 1 to 3 times per day may contain from 0.01 to about 1000 mg, such as about 0.01 to 100 mg, preferably from about 0.05 to about 500 mg, and more preferred from about 0.5 mg to about 200 mg.

For parenteral routes such as intravenous, intrathecal, intramuscular and similar administration, typically doses are in the order of about half the dose employed for oral administration.

Typical examples of recipes for the formulation of the invention are as follows:
1) Tablets containing 5.0 mg of a compound of the invention calculated as the free base:
   Compound of the invention 5.0 mg
   Lactose 60 mg
   Maize starch 30 mg
   Hydroxypropylcellulose 2.4 mg
   Microcrystalline cellulose 19.2 mg
   Croscarmellose Sodium Type A 2.4 mg Magnesium stearate 0.84 mg
2) Tablets containing 0.5 mg of a compound of the invention calculated as the free base:
   Compound of the invention 0.5 mg
      Lactose 46.9 mg
      Maize starch 23.5 mg
      Povidone 1.8 mg
      Microcrystalline cellulose 14.4 mg
      Croscarmellose Sodium Type A 1.8 mg
      Magnesium stearate 0.63 mg
3) Syrup containing per millilitre:
   Compound of the invention 25 mg
   Sorbitol 500 mg
   Hydroxypropylcellulose 15 mg
   Glycerol 50 mg
   Methyl-paraben 1 mg
   Propyl-paraben 0.1 mg
   Ethanol 0.005 mL
   Flavour 0.05 mg
   Saccharin sodium 0.5 mg
   Water ad 1 mL
4) Solution for injection containing per millilitre:
   Compound of the invention 0.5 mg
   Sorbitol 5.1 mg
   Acetic Acid 0.05 mg
   Saccharin sodium 0.5 mg
   Water ad 1mL

### Experimental section.

In the following, seven examples of scientific evidence in support of the present invention will be presented. All findings listed are unprecedented, that is, have not found written material showing similar results.

### Example 1. Electrophysiology, rat.

Reports have suggested that inhibition of the number of spontaneously active dopaminergic neurones in the ventral tegmental area (VTA), i.e. the mesolimbic system, in rats may account for an antipsychotic potential of a compound (Chiodo and Bunney 1983, J. Neurosci., 5, 2539-2544.). In the mesolimbic system, all clinically used neuroleptics initially increase the firing rate of dopaminergic neurons (Tung et al., 1991, J. Neural Transm. Gen Sect., 84(1-2), 53-64, ). After chronic administration, such neuroleptics eventually (after 3-4 weeks of treatment) decrease the firing rate to below pre-treatment levels (Skarsfeldt 1992, Synapse, 10, 25-33; White and Wang 1983, Science, 221, 1054-1057). This inhibitory effect on dopaminergic neurones, which is believed to be mediated by a depolarisation blockade, is thought to be of therapeutic significance to the antipsychotic effect of neuroleptics (Grace and Bunney 1986, J. Pharmacol. Exp. Ther. 238, 1092-1100). By inference, a compound that causes an acute decrease in spontaneous firing rate of mesolimbic dopaminergic neurones could be anticipated to possess a fast-onset antipsychotic potential. The presence of KCNQ subunits on DA neurons in the VTA in rodents is well-documented but their functionality is unknown (Saganich et al. 2001, J. Neurosci. 21(13)4609-4624; Cooper et al. 2001, J. Neurosci., 21(24)9529-9540). Consequently, it was studied *in vivo* whether KCNQ openers could acutely inhibit spontaneous activity of DA neurons in the VTA.

Subjects. Male Wistar rats (Harlan, The Netherlands) weighing 270-340 g were used. The animals were housed under a 12-hr light/dark cycle under controlled conditions for regular in-door temperature (21±2°C) and humidity (55±5%) with food and tap water available ad libitum.

Experimental procedure. The rats were anaesthetised with an intraperitoneal injection of chloral hydrate (400 mg/kg). A femoral vein catheter was then inserted for supplementary anaesthetic injections (100 mg/kg) and drug administration. Animals were then mounted in a stereotaxic frame, the skull was exposed, and a hole (0.5 x 0.5 cm) was drilled above the ventral tegmental area. Extracellular single-cell recordings were performed using electrodes pulled from glass capillaries and filled with 2% Pontamine Sky Blue in 2 M NaCl. The tip of the electrode was broken under microscopic control, yielding an impedance of 2.0 - 8.0 MΩ at 135 Hz. The electrode was then lowered into the brain, using a hydraulic microdrive, aimed at the following coordinates: 5.5 - 5.0 mm posterior to Bregma; 0.5 - 0.9 mm lateral to the midline. Extracellular action potentials were amplified, discriminated and monitored on an oscilloscope and an audiomonitor. Discriminated spikes were collected and analysed using Spike 2 software (Cambridge Electronic Design Ltd., Cambridge, UK) on a PC-based system connected to a CED 1401 interface unit (Cambridge Electronic Design Ltd.). Presumed dopaminergic neurons were typically found 7.0 - 8.5 mm beneath the brain surface and were characterised by (1) a slow and irregular firing pattern (0.5 - 10 Hz), and (2) triphasic action potentials with a predominant positive component, a negative component followed by a minor positive component, with an overall duration > 2.5 ms (Bunney et al. 1973, J. Pharmacol. Exp. Ther., 185, 560-571.). Administration of compounds. Once a stable basal firing rate was obtained, cumulated doses of N-(2-amino-4-(4-fluorobenzylamino)-phenyl) carbamic acid ethyl ester; dose range 0.3-6.0 mg/kg; volume range 0.15-1.0 ml/kg), 2-Cyclopentyl-N-(2,6-dimethyl-4-morpholin-4-yl-phenyl)-acetamide (dose range 0.03-0.3 mg/kg; volume range 0.1-1.0 ml/kg) or N-(2,6-Dimethyl-4-morpholin-4-yl-phenyl)-3,3-dimethyl-butyramide (dose range 0.03-0.5 mg/kg; volume range 0.12-1.0 ml/kg) were administered i.v., each injection being separated by at least 3 min. These i.v. doses match the s.c. dose range of 0-10 mg/kg.

Statistical analysis. Drug effects were assessed by statistical comparison of the mean firing rate calculated from the 2 - 3 min period immediately before the first drug administration (baseline) to the mean firing rate calculated from at least 60 s at the maximal drug effect. Data were analysed statistically by a one-way ANOVA followed by Student-Newman-Keuls posthoc test. A p-value less than 0.05 was considered significant.

Results. As can be seen from Table 1, N-(2-amino-4-(4-fluorobenzylamino)-phenyl) carbamic acid ethyl ester, 2-Cyclopentyl-N-(2,6-dimethyl-4-morpholin-4-yl-phenyl)-acetamide, and N-(2,6-Dimethyl-4-morpholin-4-yl-phenyl)-3,3-dimethyl-butyramide all significantly, and dose-dependently inhibited the spontaneous DA cell firing in the VTA of anaesthetised rats following acute administration of compound. This data support a potential for these compounds to posses a fast-onset antipsychotic potential. Table 1. Effects of compounds on spontaneous DA cell firing in the VTA of anaesthetised rats.

| Cumulated dose (mg/kg) | N-(2-amino-4-(4-fluorobenzylamino)-phenyl) carbamic acid ethyl ester | 2-Cyclopentyl-N-(2,6-dimethyl-4-morpholin-4-yl-phenyl)-acetamide | N-(2,6-Dimethyl-4-morpholin-4-yl-phenyl)-3,3-dimethyl-butyramide |
|---|---|---|---|
| 0 (Vehicle) | 97.5 ± 1.2 (6) | 97.8 ± 0.7 (8) | 97.5 ± 0.8 (10) |
| 0.03 | - | 95.1 ± 1.9 (4) | 89.8 ± 4.5 (5) |
| 0.1 | - | 88.2 ±2.8 (5) * | 81.0 ± 4.0 (5) ** |
| 0.25 | - | - | 74.1 ± 6.3 (4) *** |
| 0.3 | 95.4 ± 5.0 (4) | 74.6 ± 3.6 (4) *** | - |
| 0.5 | - | - | 68.1 ± 6.0 (4) *** |
| 0.6 | - | 64.1 ± 7.1 (2) *** | - |
| 0.9 | - | 55.8 ± 6.5 (2) *** | - |
| 1.0 | 90.5 ± 4.6 (5) | - | 57.1 ± 7.9 (3) *** |
| 2.0 | 81.1 ± 5.1 (5) * | - | - |
| 4.0 | 67.1 ± 3.7 (4) *** | - | - |
| 6.0 | 60.6 ± 0.7 (2) *** | - | - |

Mean ± standard error of the mean. Spontaneous DA cell firing rates expressed as a percentage of baseline firing rate; n is indicated in brackets; * p<0.05, ** p<0.01, *** p<0.001 compared to baseline (pre-drug administration activity).

### Example 2. Amphetamine challenge, rat.

D-amphetamine administration to rodents stimulates an increase in locomotor activity via mesolimbic dopamine receptors in the nucleus accumbens. While psychostimulant psychosis may not model all forms of schizophrenia, it may have applicability to paranoid schizophrenia and non-schizophrenic psychotic disorders (Krystal et al. pp. 214-224 in Neurobiology of Mental Illness ISBN 0-19-511265-2). It is believed that inhibition of the amphetamine-induced increase in locomotor activity is a reliable method for the evaluation of compounds with an antipsychotic potential (Ögren et al., European J. Pharmacol. 1984, 102, 459-464). In the following experiment, it was tested if the inhibition of spontaneous DA neurons in the mesolimbic circuit that was assessed above, could be translated into behavioral antipsychotic endpoint.

Subjects. Male Wistar rats (Taconic, Denmark) weighing 170-240 g are used. The animals were housed under a 12-hr light/dark cycle under controlled conditions for regular in-door temperature (21±2°C) and humidity (55±5%) with food and tap water available ad libitum. Eight rats were used at each dose level and in the parallel control group receiving the vehicle to the test compound plus d-amphetamine and the group receiving vehicle injections only.

Experimental procedure. The experiment is made in normal light conditions in an undisturbed room. The test substance is injected 30 min before s.c. before the injection of d-amphetamine sulphate (0.5 mg/kg). Immediately after injection of d-amphetamine, the rats are placed individually in the test cages that are placed in a U-frame, equipped with 4 infrared light sources and photocells. The light beams cross the cage 4 cm above the cage floor. Recording of a motility count requires interruption of adjacent light beams, thus avoiding counts induced by stationary movements of the rat. Motility (counts) is recorded for a period of 2 hours. The mean motility induced by vehicle (saline) treatment in the absence of d-amphetamine is used as baseline. The 100 per cent effect of d-amphetamine is accordingly calculated to be total motility counts minus baseline. The response in groups receiving test compound is thus determined by the total motility counts minus baseline, expressed in per cent of the similar result recorded in the parallel amphetamine control group. The per cent responses are converted to per cent inhibition from which ED50 values are calculated by means of log-probit analyses. In a parallel set of data, the potential sedative properties (motility inhibition) of the test compounds are evaluated using essentially the same procedure with the exception of not administering d-amphetamine-sulphate at the initiation of locomotor assessment. Results. As can be seen from Table 2, N-(2-amino-4-(4-fluorobenzylamino)-phenyl) carbamic acid ethyl ester, N-(2,6-Dimethyl-4-morpholin-4-yl-phenyl)-3,3-dimethylbutyramide, 2-Cyclopentyl-N-(2,6-dimethyl-4-morpholin-4-yl-phenyl)-acetamide all produced an inhibition of the d-amphetamine induced hyperactivity in rats. The potency with which their effects were exerted was stronger than their potency to inhibit locomotor activity, that is, the inhibition of amphetamine-induced hyperactivity could not be explained by sedative properties of the compounds. Rather, their efficacy would reflect an antipsychotic potential of N-(2-amino-4-(4-fluorobenzylamino)-phenyl) carbamic acid ethyl ester, N-(2,6-Dimethyl-4-morpholin-4-yl-phenyl)-3,3-dimethylbutyramide and 2-Cyclopentyl-N-(2,6-dimethyl-4-morpholin-4-yl-phenyl)-acetamide. Since lithium is well accepted as efficacious for the treatment of acute mania and the prophylaxis of bipolar disorders (Goldberg 2000, J. Clin. Psychiatry 61 (Suppl. 13), 12-18), while olanzapine is accepted for its efficacy for the treatment of schizophrenia, and both lithium and olanzapine were efficacious in this model, these data support a potential for N-(2-amino-4-(4-fluorobenzylamino)-phenyl) carbamic acid ethyl ester, N-(2,6-Dimethyl-4-morpholin-4-yl-phenyl)-3,3-dimethyl-butyramide, 2-Cyclopentyl-N-(2,6-dimethyl-4-morpholin-4-yl-phenyl)-acetamide to treat mania and bipolar disorder as well as schizophrenia.

**Table 2. Effects of compounds on amphetamine-induced hyperactivity in the rat.**

| Compound | Amphetamine antagonism ED50 (mg/kg) ± std.dev. | Motility inhibition ED50 (mg/kg) ± std.dev. |
|---|---|---|
| N-(2-amino-4-(4-fluorobenzylamino)-phenyl) carbamic acid ethyl ester | 2,3 (1,2) | >8,1 |
| N-(2,6-Dimethyl-4-morpholin-4-yl-phenyl)-3,3-dimethyl-butyramide | 2,1 (1,5) | 7,6 (4,8) |
| 2-Cyclopentyl-N-(2,6-dimethyl-4-morpholin-4-yl-phenyl)-acetamide | 2,6 (2,3) | 5,3 (2,6) |
| Lithium-chloride | 12 (1,7) | >40 |
| Olanzapine | 0,21 (1,7) | 0,72 (2,4) |

### Example 3. Microdialysis, rat.

It is well-known that psychostimulants increase locomotor activity via an increase in extracellular DA levels in the nucleus accumbens, which is the terminal area of the mesolimbic DA projections (Guix et al., 1992, Neurosci. Lett., 138(1), 137-140; Moghaddam et al., 1989, Synapse, 4(2), 156-161). It is also known, that the antagonistic effect of antipsychotics on stimulant-induced hyperlocomotion is related to the effect of antipsychotics to inhibit the stimulated DA levels in the nucleus accumbens (Broderick et al., 2004, Prog. Neuropsychopharmacology and Biol. Psych., 28, 157-171). Thus, the nucleus accumbens is an accepted neuroanatomical site for testing reversal of positive symptoms of psychosis. Consequently, the following experiments were conducted to investigate the effect of N-(2-amino-4-(4-fluorobenzylamino)-phenyl) carbamic acid ethyl ester, 2-Cyclopentyl-N-(2,6-dimethyl-4-morpholin-4-yl-phenyl)-acetamide, N-(2,6-Dimethyl-4-morpholin-4-yl-phenyl)-3,3-dimethyl-butyramide on baseline and amphetamine-evoked levels of DA in the nucleus accumbens of freely moving rats. The experiments were conducted such that the data may be associated with the behavioural data obtained above. Subjects. Male Sprague-Dawley rats (Charles River), initially weighing 275-300 g, were used. The animals were housed under a 12-hr light/dark cycle under controlled conditions for regular in-door temperature (21±2°C) and humidity (55±5%) with food and tap water available ad libitum.

Surgery. Animals were anaesthetized with hypnorm/dormicum (2 ml/kg s.c.) and intracerebral guide cannulas (CMA/12) were stereotaxically implanted, positioning the dialysis probe tip in the nucleus accumbens (co-ordinates: 1.7 mm anterior to bregma, -1,2 mm lateral to bregma, 8.0 mm ventral to the dura). Anchor screws and acrylic cement was applied for fixation of the guide cannula. The body temperature of the animals was maintained at 37°C by means of a rectal probe and a heating plate. The rats were allowed to recover from surgery for 2 days, housed singly in cages. Experimental procedure. On the day of the experiment, a microdialysis probe (CMA/12, 0,5 mm diameter, 2 mm length) was inserted through the guide cannula of the conscious animal. The probes were connected to a microinjection pump via a dual channel swivel which allowed the animals unrestricted movements. Perfusion of the microdialysis probe with filtered Ringer solution (145 mM NaCl, 3 mM KCl, 1 mM MgCl₂, 1,2 mM CaCl₂) was maintained for the duration of the experiment at a constant flow rate of 1 µL/min. After 180 min of stabilisation, the experiments were initiated. Dialysates were collected every 20 min. After the experiments the rats were sacrificed by decapitation, their brains removed, frozen and sliced for probe placement verification.

Administration of compounds. 2-Cyclopentyl-N-(2,6-dimethyl-4-morpholin-4-yl-phenyl)-acetamide (5 mg/kg), N-(2,6-Dimethyl-4-morpholin-4-yl-phenyl)-3,3-dimethyl-butyramide (5 mg/kg), retigabine (8,1 mg/kg) or vehicle (10% 2-hydroxy-propyl-beta-cyclodextrin, isotonic, pH 5-7) was administered subcutaneously in a volume of 2.5 ml/kg. Thirty min after the first administration dex-amphetamine sulphate (0.5 mg/kg s.c.) was administered.

Analysis of dialysate. The concentration of dopamine (DA) in the dialysates was assessed by means of HPLC with electrochemical detection. The dialysate constitutents were separated by reverse phase liquid chromatography (ODS 150 x 3 mm, 3 µM). Mobile phase consisted of 90 mM NaH₂PO₄, 50 mM sodium citrate, 367 mg/l sodium 1-octanesulfonic acid, 50 µM EDTA and 8% acetonitrile (pH 4.0) at a flow rate of 0.5 ml/min. Electrochemical detection of DA was accomplished using a coulometric detector; potential set at E1 =-75 mV and E2 = 300 mV (guard cell at 350 mV) (Coulochem II, ESA). The dialysate levels of DA in the three dialyse samples preceding the administration of compound were averaged and used as baseline level of DA (100%)

Statistical analysis. The dialysate levels of DA in the three dialyse samples preceding the administration of compound were averaged and used as baseline level of DA (100%)

Data were analysed using repeated measure analyses of variance followed by post hoc tests (Tukey test), when appropriate. *P < 0.05 were considered significant.

Results. As can be seen in Table 3, N-(2-amino-4-(4-fluorobenzylamino)-phenyl) carbamic acid ethyl ester (P < 0.001), 2-Cyclopentyl-N-(2,6-dimethyl-4-morpholin-4-yl-phenyl)-acetamide (P < 0.05) and N-(2,6-Dimethyl-4-morpholin-4-yl-phenyl)-3,3-dimethyl-butyramide (P = 0.002) significantly dampened the amphetamine-induced increase in extracellular levels of DA in the nucleus accumbens of freely moving rats. Neither 2-Cyclopentyl-N-(2,6-dimethyl-4-morpholin-4-yl-phenyl)-acetamide, N-(2,6-Dimethyl-4-morpholin-4-yl-phenyl)-3,3-dimethyl-butyramide nor retigabine significantly affected the basal extracellular DA level in this region (data not shown). These data suggest that the antagonistic effect of N-(2-amino-4-(4-fluorobenzylamino)-phenyl) carbamic acid ethyl ester, 2-Cyclopentyl-N-(2,6-dimethyl-4-morpholin-4-yl-phenyl)-acetamide, and N-(2,6-Dimethyl-4-morpholin-4-yl-phenyl)-3,3-dimethyl-butyramide on amphetamine-induced activity in rats seen above, i.e. antipsychotic activity, is indeed associated with a dampening of provoked DA levels in the nucleus accumbens which further strengthens the antipsychotic potential of these compounds. The observation that merely provoked levels of DA were affected, but not basal levels of DA, suggests a low risk of causing anhedonia, a trait that is transiently, but frequently, observed with clinically used antipsychotics (Guys: I have heard this mentioned at several lectures but am searching for a reference!).

**Table 3. Effects of compounds on the amphetamine-evoked increase in DA levels in the nucleus accumbens of freely moving rats.**

| Time (min) | Amphetamine + vehicle % of baseline | Amphetamine + 2-Cyclopentyl-N-(2,6-dimethyl-4-morpholin-4-yl-phenyl)-acetamide (5 mg/kg) % of baseline | Amphetamine + N-(2,6-Dimethyl-4-morpholin-4-yl-phenyl)-3,3-dimethyl-butyramide (5 mg/kg) % of baseline | Amphetamine + N-(2-amino-4-(4-fluorobenzylam ino)-phenyl) carbamic acid ethyl ester (8,1 mg/kg) % of baseline |
|---|---|---|---|---|
| -40 | 91 ± 6 | 95 ± 6 | 108 ± 5 | 105 ± 3 |
| -20 | 96 ± 5 | 106 ± 5 | 100 ± 3 | 99 ± 6 |
| 0 | 112 ± 7 | 99 ± 4 | 91 ± 4 | 96 ± 6 |
| 20 | 168 ± 19 | 125 ± 9 | 112 ± 12 | 160 ± 11 |
| 40 | 338 ± 27 | 264 ± 39 | 227 ± 46 | 241 ± 33 |
| 60 | 375 ± 46 | 265 ± 17* | 262 ± 53* | 221 ± 26* |
| 80 | 319 ± 59 | 231 ± 22 | 195 ± 38* | 217 ± 14* |
| 100 | 232 ± 48 | 167 ± 25 | 172 ± 24 | 173 ± 9 |
| 120 | 162 ± 37 | 109 ± 11 | 166 ± 32 | 139 ± 9 |
| 140 | 129 ± 27 | 93 ± 15 | 129 ± 35 | 120 ± 9 |

Normalised DA levels in the nucleus accumbens of freely moving rats are shown. * P < 0.05 compared to amphetamine-vehicle group, same time.

### Example 4. Amphetamine sensitisation, mouse.

Clinical data imply that amphetamine-naïve schizophrenic and bipolar patients display an exaggerated response to a first dose of amphetamine implying that these patients may show a dopaminergic sensitisation (Strakowski et al. 1996, Biol. Psychiatry 40, 872-880, Lieberman et al. 1987, Psychopharmacology, 91, 415-433, Strakowski et al., 2001, CNS Drugs 15, 701-708). This phenomenon is modelled in rodents when repeated intermittent administration of amphetamine leads to a progressive increase in the behavioral response to an amphetamine challenge, a phenomenon known as behavioral sensitisation (Robinson and Berridge, Brain Research Rev. 1993, 18(3):247-91). The mesolimbic dopamine pathway is believed to be the major neural circuit involved in this behavioral sensitisation (Robinson and Becker, Brain Research 1986, 396(2):157-98). Inhibition of the behavioral response to an acute amphetamine challenge in sensitised animals is proposed as a model for evaluating the antipsychotic or antimanic potential of compounds.

Subjects. Male NMRI mice (Charles River) weighing approx. 35 g are used. The animals were housed 6 mice pr cage in a 12-hr light/dark cycle under controlled conditions for regular in-door temperature (21±2°C) and humidity (55±5%) with food and tap water available ad libitum. 12 mice are used pr experimental group.

Experimental procedure. All mice are pre-treated once daily for five days with either d-amphetamine sulphate (2.5 mg/kg s.c.) or saline (10 ml/kg). For the 17 days between the last day of pre-treatment and the test day, the animals are kept in their homecage receiving the startard care as described above. The experiment is performed under normal light conditions in an undisturbed room. The mice are treated with test substance or vehicle and placed individually in the test cages for 30 min. The mice are then challenged with D-amphetamine sulphate (1.25 mg/kg s.c.) or saline (5 ml/kg) and replaced in the test-cage and data acquisition is begun. 5 x 8 infrared light sources and photocells interspaced by 4 cm monitor the locomotor activity. The light beams cross the cage 1.8 cm above the bottom of the cage. The recording of a motility count requires interruption of adjacent light beams, thereby avoiding counts induced by stationary movements of the mice.

Administration of compounds. Amphetamine-pretreated mice and vehicle-pretreated mice were s.c. treated with N-(2-amino-4-(4-fluorobenzylamino)-phenyl) carbamic acid ethyl ester (0-10 mg/kg), 2-Cyclopentyl-N-(2,6-dimethyl-4-morpholin-4-yl-phenyl)-acetamide (0-5 mg/kg) or N-(2,6-Dimethyl-4-morpholin-4-yl-phenyl)-3,3-dimethyl-butyramide (0-5 mg/kg) or vehicle (10% 2-hydroxy-propyl-beta-cyclodextrin, isotonic, pH 5-7, 5 ml/kg) 30 min prior to the data acquisition.

Data analyses. The total counts obtained in the 30 min test were averaged pr animal group and used for calculation of drug effects in the following manner: The average motility induced by an amphetamine challenge in amphetamine-pretreated animals is used as the sensitised response. The average motility induced by a vehicle challenge to vehicle-pretreated animals is used as a baseline motility response. The baseline value is subtracted from the sensitized amphetamine response value and set as 100% i.e. the sensitised response. This calculation is repeated for each dose group and the value for each dose-group is subsequently expressed relative to the 100% value. That is, the response in amphetamine-sensitized groups receiving test compound is thus determined as the sensitised response minus the baseline motility, expressed in per cent of the similar result recorded in the sensitized amphetamine response group. The percent responses are converted to percent inhibition and exposed to log-probit analysis thus producing an ED50 for inhibiting the sensitised response. Similarly an ED50 for inhibiting baseline motility is calculated by expressed the motility response in vehicle-pretreated, vehicle-challenged, drug-treated animals relative to the baseline motility response. A therapeutic index value can subsequently be calculated by dividing the first ED50 by the second.

Results. As can be seen in Table 4, N-(2-amino-4-(4-fluorobenzylamino)-phenyl) carbamic acid ethyl ester, N-(2,6-Dimethyl-4-morpholin-4-yl-phenyl)-3,3-dimethylbutyramide and 2-Cyclopentyl-N-(2,6-dimethyl-4-morpholin-4-yl-phenyl)-acetamide as well as that antimanic compound lithium, and the antipsychotic olanzapine, all inhibit the hyperactivity induced by an amphetamine challenge in sensitised mice. The potency with which these compounds exert this effect is stronger than the potency with which these compounds inhibit baseline motility. That is, the compounds posses a calming effect, i.e. antipsychotic/antimanic effect, that is separable from its sedative effects (i.e. therapeutic index > 1). This separation is characteristic for neuroleptics (Kapur and Mamo 2003, Biol. Psych. 27(7), 1081-1090) and thus support an antipsychotic/antimanic potential for N-(2-amino-4-(4-fluorobenzylamino)-phenyl) carbamic acid ethyl ester, N-(2,6-Dimethyl-4-morpholin-4-yl-phenyl)-3,3-dimethylbutyramide, 2-Cyclopentyl-N-(2,6-dimethyl-4-morpholin-4-yl-phenyl)-acetamide. Table 4. Effects of compounds on a sensitised behavioural response to amphetamine in mice.

| Compound | Inhibition of amphetamine sensitised response. ED50 (± S.D.) (mg/kg) | Inhibition of baseline motility. ED50 (± S.D.) (mg/kg) | Therapeutic index |
|---|---|---|---|
| N-(2-amino-4-(4-fluorobenzylamino)-phenyl) carbamic acid ethyl ester | 4,4 (1,4) | 7,6 (1,3) | 2 |
| N-(2,6-Dimethyl-4-morpholin-4-yl-phenyl)-3,3-dimethyl-butyramide | 1,6 (1,2) | >2,5 | >1 |
| 2-Cyclopentyl-N-(2,6-dimethyl-4-morpholin-4-yl-phenyl)-acetamide | 1,2 (1,3) | 2,2 (1,3) | 2 |
| N-(4,6-Dimethyl-2-morpholin-4-yl-pyrimidin-5-yl)-2-(4-fluoro-henyl)-acetamide | 0,4 | 2,2 | 5 |
| Hexanoic acid (2,6-difluoro-4-morpholin-4-yl-phenyl)-amide | 2,4 | >5 | >2 |
| 2-Cyclopentyl-N- | 0,9 | 2,5 | 3 |
| (4,6-dimethyl-2-morpholin-4-yl-pyrimidin-5-yl)-acetamide | | | |
| N-(2-Bromo-4-morpholin-4-yl-6-trifluoromethyl-phenyl)-propionamide | 3,1 | 6,0 | 2 |
| N-(2,4-Dimethyl-6-morpholin-4-yl-pyridin-3-yl)-3,3-dimethyl-butyramide | 1,4 | 3,1 | 2 |
| [2-Amino-4-(2,4,6-trimethyl-benzylamino)-phenyl]-carbamic acid ethyl ester | 1,7 | 4,3 | 3 |
| 2-Cyclopentyl-N-(2-methoxy-6-methyl-4-morpholin-4-yl-phenyl)-acetamide | 1,7 | 3,0 | 2 |
| Lithium-cloride | 34 (7,2) | >>40 | >>1 |
| Olanzapine | 0,11 (1,4) | >0,31 | >3 |

### Example 5. Conditioned avoidance, rat.

In the conditioned avoidance response (CAR) model, rats are trained to respond to a stimulus within a fixed time by moving from one place to another in order to avoid a footshock. Antipsychotics selectively suppress the avoidance response within a certain dose-range without suppressing escape behavior elicited by the appearance of the footshock. The CAR model is considered to be a predictive and reliable animal model that is sensitive to compounds with an antipsychotic potential. Thus, all clinically effective antipsychotics have been shown to inhibit CAR (Wadenberg and Hicks, Neuroscience and Biobehav Rev 23, 851-862, 1999).

Subject. Male Wistar rats (Taconic, Denmark) weighing 150 g at the beginning of the study are used. The rats are housed in pairs and maintained on a 12 h light/dark cycle (lights on 06:00). The animals are fed once daily (approx. 6 pellets/rat) in order to keep the rats at 80% of their free-feeding weight. Water is available ad libitum. Temperature (21 ± 1°C) and relative humidity (55 ± 5%) are automatically controlled.

Experimental procedure. Conditioned avoidance testing is conducted using four automated shuttle-boxes (ENV-010M, MED-Associates) each placed in a sound-attenuated chamber. Each box is divided into two compartments by a partition with an opening. The position of the animal and crossings from one compartment to the other are detected by two photocells placed on either side of the dividing wall. Upon presentation of the conditioned stimuli (CS), tone and light, the animals have 10s to cross to the other compartment of the shuttle-box in order to turn the CS off (end the trial) and avoid the appearance of the unconditioned stimulus (UCS). If the rat remains in the same compartment for more than 10s, the UCS is presented as 0.5 mA scrambled foot-shocks until escape is performed or 10s in maximal duration. The following behavioural variables are evaluated: avoidance (response to CS within 10s); escape (reponse to CS + UCS); escape failures (failure to respond); intertrial crosses and locomotor activity. The rats are habituated to the shuttle-box 3 min before each test session. During training each test session consists of 30 trials with intertrial intervals varying randomly between 20s and 30s. Training is carried until the rats display an avoidance of 80% or more, on 3 consecutive days. A test is preceded by a pre-test the day before giving rise to a baseline value for each animal, thus the animals serve as their own control. Seven to eight rats are used at each dose level. A parallel control group receiving the vehicle of the test compound is also included.

Administration of compound. N-(2-amino-4-(4-fluorobenzylamino)-phenyl) carbamic acid ethyl ester (5 and 10 mg/kg), N-(2,6-Dimethyl-4-morpholin-4-yl-phenyl)-3,3-dimethyl-butyramide (2,5 and 5 mg/kg) and 2-Cyclopentyl-N-(2,6-dimethyl-4-morpholin-4-yl-phenyl)-acetamide (2,5 and 5 mg/kg) were administered s.c. 30 min before the test, in a volume of 5 ml/kg. All compounds were dissolved in a vehicle of 10% 2-hydroxy-propyl-beta-cyclodextrin (isotonic with glucose, pH 5-7).

Statistical analyses. The effects of compounds on avoidance and escape failure behaviours were statistically evaluated by means of a two-way repeated measures ANOVA followed by post hoc comparisons (Student-Newman-Keuls Method) when appropriate. P-levels < 0.05 were considered statistically significant.

Results. As can be seen in Table 5, N-(2-amino-4-(4-fluorobenzylamino)-phenyl) carbamic acid ethyl ester, 2-Cyclopentyl-N-(2,6-dimethyl-4-morpholin-4-yl-phenyl)-acetamide, and N-(2,6-Dimethyl-4-morpholin-4-yl-phenyl)-3,3-dimethyl-butyramide all significantly reduced the number of avoidances indicative of an antipsychotic-like activity of 2-Cyclopentyl-N-(2,6-dimethyl-4-morpholin-4-yl-phenyl)-acetamide and N-(2,6-Dimethyl-4-morpholin-4-yl-phenyl)-3,3-dimethyl-butyramide (5 mg/kg) and N-(2-amino-4-(4-fluorobenzylamino)-phenyl) carbamic acid ethyl ester (10 mg/kg). None of the tested doses caused any incidences of escape failures, corresponding to a lack of effect on motor performance (data not shown). In conclusion, these data support an antipsychotic potential of N-(2-amino-4-(4-fluorobenzylamino)-phenyl) carbamic acid ethyl ester, N-(2,6-Dimethyl-4-morpholin-4-yl-phenyl)-3,3-dimethylbutyramide, 2-Cyclopentyl-N-(2,6-dimethyl-4-morpholin-4-yl-phenyl)-acetamide.

**Table 5. Effects of compounds on the conditioned avoidance response in rats.**

| Treatment | % inhibition of avoidance (Std.dev.) Relative to baseline. |
|---|---|
| Vehicle (10%Hpbeta) | -2 (4,2) |
| N-(2-amino-4-(4-fluorobenzylamino)-phenyl) carbamic acid ethyl ester, 5 mg/kg | 1 (6,7) |
| N-(2-amino-4-(4-fluorobenzylamino)-phenyl) carbamic acid ethyl ester, 10 mg/kg | 59 (37) *** P < 0.001 |
| 2-Cyclopentyl-N-(2,6-dimethyl-4-morpholin-4-yl-phenyl)-acetamide, 2,5 mg/kg | 5 (3,5) |
| 2-Cyclopentyl-N-(2,6-dimethyl-4-morpholin-4-yl-phenyl)-acetamide, 5 mg/kg | 65 (36) *** P < 0.001 |
| N-(2,6-Dimethyl-4-morpholin-4-yl-phenyl)-3,3-dimethyl-butyramide, 2,5 mg/kg | 1 (14) |
| N-(2,6-Dimethyl-4-morpholin-4-yl-phenyl)-3,3-dimethyl-butyramide, 5 mg/kg | 71 (26) *** P < 0.001 |

### Example 6. Forced swim test, mouse.

The schizophrenic spectrum of symptoms involves a cluster of negative symptoms including anhedonia, social withdrawal and emotional flattening. Such symptoms are inadequately treated by currently available antipsychotics (Duncan et al. 2004, Schizoph. Res., 71(2-3), 239-248; Meltzer et al. 1986, J. Clin. Psychopharmacol., 6(6), 329-338). The forced swim is test is a widely and frequently used model.for preclinical evaluation of antipressant activity (Porsolt et al. 1977, Arch. Int. Pharmacodyn. 229, 327-336). In order to test whether N-(2-amino-4-(4-fluorobenzylamino)-phenyl) carbamic acid ethyl ester, 2-Cyclopentyl-N-(2,6-dimethyl-4-morpholin-4-yl-phenyl)-acetamide or N-(2,6-Dimethyl-4-morpholin-4-yl-phenyl)-3,3-dimethyl-butyramide could have an antidepressant-like or mood elevating effect, these compounds were tested in the mouse forced swim test.

Subjects. Male NMRI mice (Charles River) weighing 23-25 g were used. The mice were kept 8 mice pr cage in a 12-hr light/dark cycle under controlled conditions for regular in-door temperature (21±2°C) and humidity (55±5%) with food and tap water available ad libitum. 8 mice weree used pr experimental group.

Experimental procedure. The mice were placed in 2000 ml beaker containing 1200 ml of tempered water (25°C) and left to swim for 6 min. The performance of the mice was videorecorded, digitalized and analysed by means of a digital analysis system (Bioobserve). The time spent immobile for the last 3 min. of the test session was quantified for each mouse.

Treatment. 30 min. before the test, mice were treated s.c. with N-(2,6-Dimethyl-4-morpholin-4-yl-phenyl)-3,3-dimethyl-butyramide, 2-Cyclopentyl-N-(2,6-dimethyl-4-morpholin-4-yl-phenyl)-acetamide or vehicle (10-%-2-OH-propyl-cyclodextrin, 10 ml/kg). In addition as positive control, imipramine-HCl (40 mg/kg) and a saline control (10 ml/kg) was included.

Analyses. The time spent immobile was statistically compared across the experimental groups against the relevant control group by means of one-way analysis of variance. A post-hoc test (Student-Newman-Keuls) was employed when appropriate. P-levels < 0.05 were considered significant.

Results. As can be seen from Table 6, 2-Cyclopentyl-N-(2,6-dimethyl-4-morpholin-4-yl-phenyl)-acetamide and N-(2,6-Dimethyl-4-morpholin-4-yl-phenyl)-3,3-dimethylbutyramide both significantly reduced the time spent immobile during the 3-6 min swim in mice. Their efficacy was inferior to, yet comparable to, the effect of an antidepressant dose of imipramine-HCl. In contrast, the antipsychotic olanzapine had only a weak effect in this test which is in line with the observation that this compound has an inadequate effect on negative symptoms in humans. These data support an antidepressant potential of 2-Cyclopentyl-N-(2,6-dimethyl-4-morpholin-4-yl-phenyl)-acetamide and N-(2,6-Dimethyl-4-morpholin-4-yl-phenyl)-3,3-dimethyl-butyramide which may translate into a potential to treat negative symptoms in schizophrenic patients.

**Table 6. Effects of compounds on immobility in the mouse forced swim test.**

| Dose: mg/kg | 2-Cyclopentyl-N-(2,6-dimethyl-4-morpholin-4-yl-phenyl)-acetamide Immobility in % (± S.D.) | N-(2,6-Dimethyl-4-morpholin-4-yl-phenyl)-3,3-dimethyl-butyramide Immobility in % (± S.D.) | Olanzapine Immobility in % (± S.D.) | Imipramine-HCl Immobility in % (± S.D.) |
|---|---|---|---|---|
| Vehicle | 100 (6,6) | 100 (6,6) | 100 (6,61) | 100 (7,1) |
| 0,31 | - | - | 96 (14) | - |
| 1,3 | 97,5 (6,3) | 102 (4,6) | 95 (11) | - |
| 2,5 | 97 (6,7) | 96,7 (7,5) | - | - |
| 5,0 | 81,0 (18) * | 82,3 (20) * | - | - |
| 40 | - | - | - | 73,8 (22) * |

### Example 6. Relative efflux through the KCNQ2 channel.

This exemplifies a KCNQ2 screening protocol for evaluating compounds of the present invention. The assay measures the relative efflux through the KCNQ2 channel, and was carried out according to a method described by Tang et al. (Tang, W. et. al., J. Biomol. Screen. 2001, 6, 325-331) for hERG potassium channels with the modifications described below.

An adequate number of CHO cells stably expressing voltage-gated KCNQ2 channels were plated at a density sufficient to yield a mono-confluent layer on the day of the experiment. Cells were seeded on the day before the experiment and loaded with 1 µCi/ml [⁸⁶Rb] over night. On the day of the experiment cells were washed with a HBSS-containing buffer. Cells were pre-incubated with drug for 30 minutes and the ⁸⁶Rb⁺ efflux was stimulated by a submaximal concentration of 15 mM KCl in the continued presence of drug for additional 30 minutes. After a suitable incubation period, the supernatant was removed and counted in a liquid scintillation counter (Tricarb). Cells were lysed with 2 mM NaOH and the amount of ⁸⁶Rb⁺ was counted. The relative efflux was calculated ((CPMₛᵤₚₑᵣ/(CPMₛᵤₚₑᵣ+ CPM_{cell}))Cmpd/ (CPMₛᵤₚₑᵣ/(CPMₛᵤₚₑᵣ+ CPm_{cell}))_{15mM KCl}) * 100-100.

The compounds of the invention have an EC₅₀ of less than 20000nM, in most cases less than 2000 nM and in many cases less than 200 nM. Accordingly, the compounds of the invention are considered to be useful in the treatment of diseases associated with the KCNQ family potassium channels.

All non-patent references, patents, and patent applications cited and discussed in this specification are incorporated herein by reference in their entirety and to the same extent as if each was individually incorporated by reference.

## Claims

1. Use of a compound able to selectively increase the ion flow through KCNQ potassium channels in the manufacture of a medicament for treating or reducing the symptoms of schizophrenia.

2. The use according to claim 1, for reducing positive symptoms of schizophrenia.

3. The use according to claim 1, for reducing negative symptoms of schizophrenia.

4. The use according to claim 1, for reducing cognitive symptoms of schizophrenia.

5. The use according to claim 1, for reducing the symptoms of one or more schizophrenia subtypes selected from the group consisting of: the catatonic subtype; the paranoid subtype; the disorganized subtype; and the residual subtype.

6. The use according to any one of claims 1-5, wherein said compound able to increase the ion flow through KCNQ potassium channels is effective in a model predictive for an anti-psychotic potential of said compound.

7. The use according to claim 6, wherein said model is selected from the group consisting of: the acute stimulant-induced hyperactivity test; the sensitised amphetamine-induced hyperactivity test; the conditioned avoidance test; the spontaneous firing of mesolimbic DA cells test; and the mouse forced-swim test.

8. The use according to any one of claims 1-7, wherein said compound does not to any reasonable extent manifest any side-effects associated with compounds known to treat schizophrenia.

9. The use according to claim 8, wherein said side-effects are side-effects mediated directly through dopamine D2 receptor modulation.

10. The use according to any one of claims 1-9, wherein said compound is administered in an amount of more than 1 mg/day.

11. The use according to any one of claims 1-10, wherein said compound has a fast onset of action.

12. The use according to any one of claims 1-11, wherein said compound is a compound according to formula 1, 2, 3, 4, 5, 6, 7, 8 or 9,
where formula 1 is: 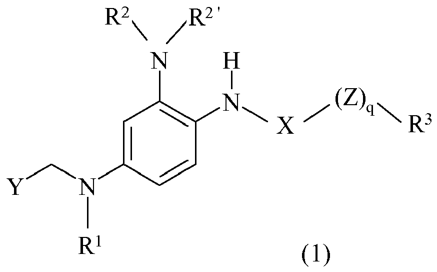 wherein
**R¹** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, acyl, hydroxy-C₁₋₆-alk(en/yn)yl and hydroxy-C₃₋₈-cycloalk(en)yl;
**R²** and **R^{2'}** are independently selected from the group consisting of hydrogen, C₁-₆-alk(en/yn)yl, C₃₋₈-cycloalk(en)yl, aryl, C₃₋₈-cycloalk(en)yl-C₁₋₆-alk(en/yn)yl, aryl-C₁₋₆-alk(en/yn)yl, acyl, hydroxy-C₁₋₆-alk(en/yn)yl and hydroxy-C₃₋₈-cycloalk(en)yl;
**R³** is selected from the group consisting of hydrogen, C₁₋₆-alk(en/yn)yl, C₃₋₈-cycloalk(en)yl, aryl, C₃₋₈-cycloalk(en)yl-C₁₋₆-alk(en/yn)yl, aryl-C₁₋₆-alk(en/yn)yl, hydroxy-C₁₋₆-alk(en/yn)yl, aryl-C₃₋₈-cycloalk(en)yl, NR¹⁰R^{10'}-C₁₋₆-alk(en/yn)yl, NR¹⁰R^{10'}-C₃₋₈-cycloalk(en)yl and hydroxy-C₃₋₈-cycloalk(en)yl; wherein
**R¹⁰** and **R^{10'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, hydroxy-C₁₋₆-alk(en/yn)yl, hydroxy-C₃₋₈-cycloalk(en)yl, hydroxy-C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, cyano-C₁₋₆-alk(en/yn)yl, cyano-C₃₋₈-cycloalk(en)yl and cyano-C₃₋₈-cycloalk(en)yl-C₁₋₆-alk(en/yn)yl, or
**R¹⁰** and **R^{10'}** together with the nitrogen atom to which they are attached form a 4-8 membered saturated or unsaturated ring which optionally contains 1, 2 or 3 further heteroatoms;
**X** is CO or SO₂;
**Z** is O or NR⁴, wherein
**R⁴** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, hydroxy-C₁₋₆-alk(en/yn)yl and hydroxy-C₃₋₈-cycloalk(en)yl; or
**R³** and **R⁴** together with the nitrogen atom to which they are attached form a 4-8 membered saturated or unsaturated ring which optionally contains 1, 2 or 3 further heteroatoms, the ring formed by **R³** and **R⁴** and the nitrogen atom is optionally substituted with one or more substituents independently selected from C₁₋₆-alk(en/yn)yl, aryl and aryl-C₁₋₆-alk(en/yn)yl;
**q** is 0 or 1;
and
**Y** represents a heteroaryl of formula II or III 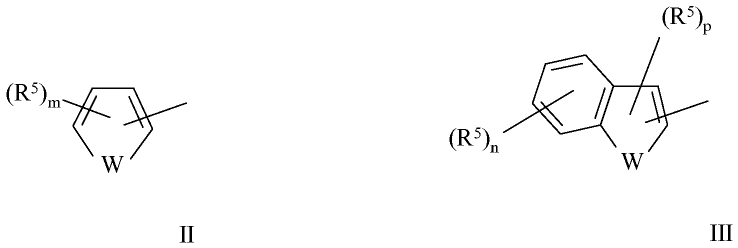 wherein
**W** is O or S;
**m** is 0,1, 2 or 3;
**n** is 0, 1, 2, 3 or 4;
**p** is 0 or 1; and
each **R⁵** is independently selected from the group consisting of C₁₋₆-alk(en/yn)yl, C₃₋₈-cycloalk(en)yl, aryl, C₃-₈-cycloalk(en)yl-C₁₋₆-alk(en/yn)yl, aryl-C₁₋₆-alk(en/yn)yl, acyl, halogen, halo-C₁₋₆-alk(en/yn)yl, C₁₋₆-alk(en/yn)yloxy, -CO-NR⁶R^{6'}, cyano, nitro, -NR⁷R^{7'}, -S-R⁸, -SO₂R⁸, SO₂OR⁸;
wherein
**R⁶** and **R^{6'}** are independently selected from the group consisting of hydrogen, C₁-₆-alk(en/yn)yl, C₃₋₈-cycloalk(en)yl, C₃₋₈-cycloalk(en)yl-C₁₋₆-alk(en/yn)yl and aryl;
**R⁷**and **R^{7'}** are independently selected from the group consisting of hydrogen, C₁-₆-alk(en/yn)yl, C₃₋₈-cycloalk(en)yl, C₃₋₈-cycloalk(en)yl-C₁₋₆-alk(en/yn)yl, aryl and acyl; and
**R⁸** is selected from the group consisting of C₁₋₆-alk(en/yn)yl, C₃₋₈-cycloalk(en)yl, C₃₋₈-cycloalk(en)yl-C₁₋₆-alk(en/yn)yl, aryl and -NR⁹R^{9'}; wherein
**R⁹** and **R^{9'}** are independently selected from the group consisting of hydrogen, C₁-₆-alk(en/yn)yl, C₃₋₈-cycloalk(en)yl and C₃₋₈-cycloalk(en)yl-C₁₋₆-alk(en/yn)yl;
or pharmaceutically acceptable salts thereof; and
where formula 2 is: 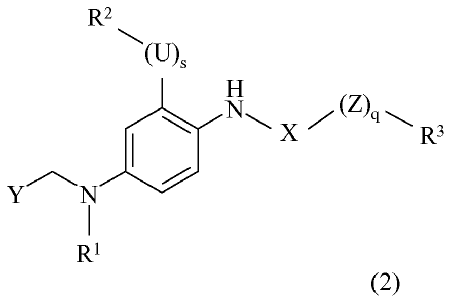 wherein
s is 0 or 1;
**U** is O, S, SO₂, SO₂N**R¹¹**, CO-O or CON**R¹¹**; wherein
**R¹¹** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl; or
**R²** and **R¹¹** together with the nitrogen atom form a 5-8 membered saturated or unsaturated ring which optionally contains 1, 2 or 3 further heteroatoms; **q** is 0 or 1;
**X** is CO or SO₂; with the proviso that **q** is 0 when **X** is SO₂;
**Z** is O or S;
**R¹** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, acyl, hydroxy-C₁₋₆-alk(en/yn)yl, hydroxy-C₃₋₈-cycloalk(en)yl, hydroxy-C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, cyano-C₁₋₆-alk(en/yn)yl, cyano-C₃₋₈-cycloalk(en)yl and cyano-C₃₋₈-cycloalk(en)yl-C₁₋₆-alk(en/yn)yl;
**R²** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar, Ar-C₁₋₆-alk(en/yn)yl, Ar-C₃₋₈-cycloalk(en)yl, Ar-C₃₋₈-cycloalk(en)yl-C₁₋₆-alk(en/yn)yl, acyl, hydroxy-C₁₋₆-alk(en/yn)yl, hydroxy-C₃₋₈-cycloalk(en)yl, hydroxy-C₃₋₈-cycloalk(en)yl-C₁₋₆-alk(en/yn)yl, halogen, halo-C₁₋₆-alk(en/yn)yl, halo-C₃₋₈-cycloalk(en)yl, halo-C₃₋₈-cycloalk(en)yl-C₁₋₆-alk(en/yn)yl, cyano, cyano-C₁₋₆-alk(en/yn)yl, cyano-C₃₋₈-cycloalk(en)yl, cyano-C₃₋₈-cycloalk(en)yl-C₁₋₆-alk(en/yn)yl, N**R¹⁰R^{10'}**-C₁₋₆-alk(en/yn)yl, N**R¹⁰R^{10'}**-C₃₋₈-cycloalk(en)yl and N**R¹⁰R^{10'}**-C₃-₈-cycloalk(en)yl-C₁₋₆-alk(en/yn)yl; wherein
**R¹⁰** and **R^{10'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, hydroxy-C₁₋₆-alk(en/yn)yl, hydroxy-C₃₋₈-cycloalk(en)yl, hydroxy-C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁-₆-alk(en/yn)yl, cyano-C₁₋₆-alk(en/yn)yl, cyano-C₃₋₈-cycloalk(en)yl and cyano-C₃₋₈-cycloalk(en)yl-C₁₋₆-alk(en/yn)yl, or
**R¹⁰** and **R^{10'}** together with the nitrogen atom form a 5-8 membered saturated or unsaturated ring which optionally contains 1, 2 or 3 further heteroatoms; provided that when **R²** is halogen or cyano then s is 0; and
provided that **U** is O or S when **s** is 1 and **R²** is a hydrogen atom or acyl;
**R³** is selected from the group consisting of C₁₋₆-alk(en/yn)yl, C₃₋₈-cycloalk(en)yl, heterocycloalk(en)yl, C₃₋₈-cycloalk(en)yl-C₁₋₆-alk(en/yn)yl, C₁₋₆-alk(en/yn)yl-C₃-₈-cycloalk(en)yl, C₁₋₆-alk(en/yn)yl-heterocycloalk(en)yl, heterocycloalk(en)yl-C₁-₆-alk(en/yn)yl, Ar, Ar-C₁₋₆-alk(en/yn)yl, Ar-C₃₋₈-cycloalk(en)yl, Ar-heterocycloalk(en)yl, Ar-C₃₋₈-cycloalk(en)yl-C₁₋₆-alk(en/yn)yl, Ar-C₁₋₆-alk(en/yn)yl-C₃₋₈-cycloalk(en)yl, Ar-C₁₋₆-alk(en/yn)yl-heterocycloalk(en)yl, C₁-₆-alk(en/yn)yloxy-C₁₋₆-alk(en/yn)yl, C₃₋₈-cycloalk(en)yloxy-C₁₋₆-alk(en/yn)yl, C₁-₆-alk(en/yn)yloxy-C₃₋₈-cycloalk(en)yl, C₁₋₆-alk(en/yn)yloxy-heterocycloalk(en)yl, Ar-oxy-C₁₋₆-alk(en/yn)yl, Ar-C₁₋₆-alk(en/yn)yloxy-C₁₋₆-alk(en/yn)yl, C₁₋₆-alk(en/yn)yloxy-carbonyl-C₁₋₆-alk(en/yn)yl, C₃₋₈-cycloalk(en)yloxy-carbonyl-C₁-₆-alk(en/yn)yl, C₃₋₈-cycloalk(en)yl-C₁₋₆-alk(en/yn)yloxy-carbonyl-C₁₋₆-alk(en/yn)yl, hydroxy-C₁₋₆-alk(en/yn)yl, hydroxy-C₃₋₈-cycloalk(en)yl, hydroxy-heterocycloalk(en)yl, hydroxy-C₃₋₈-cycloalk(en)yl-C₁₋₆-alk(en/yn)yl, hydroxy-C₁-₆-alk(en/yn)yl-C₃₋₈-cycloalk(en)yl, hydroxy-C₁₋₆-alk(en/yn)yl-heterocycloalk(en)yl, halo-_{C1-6}-alk(en/yn)yl, halo-C₃-₈-cycloalk(en)yl, halo-heterocycloalk(en)yl, halo-C₃₋₈-cycloalk(en)yl-C₁₋₆-alk(en/yn)yl, halo-C₁₋₆-alk(en/yn)yl-C₃₋₈-cycloalk(en)yl, halo-C₁₋₆-alk(en/yn)yl-heterocycloalk(en)yl, halo-C₁₋₆-alk(en/yn)yl-Ar, halo-C₃₋₈-cycloalk(en)yl-Ar, halo-C₃₋₈-cycloalk(en)yl-C₁₋₆-alk(en/yn)yl-Ar, halo-C₁₋₆-alk(en/yn)yl-C₃₋₈-cycloalk(en)yl-Ar, cyano-C₁₋₆-alk(en/yn)yl, cyano-C₃₋₈-cycloalk(en)yl, cyano-heterocycloalk(en)yl, cyano-C₃₋₈-cycloalk(en)yl-C₁₋₆-alk(en/yn)yl, cyano-C₁₋₆-alk(en/yn)yl-C₃₋₈-cycloalk(en)yl, cyano-C₁₋₆-alk(en/yn)yl-heterocycloalk(en)yl, acyl-C₁₋₆-alk(en/yn)yl, acyl-C₃₋₈-cycloalk(en)yl, acyl-heterocycloalk(en)yl, acyl-C₃₋₈-cycloalk(en)yl-C₁₋₆-alk(en/yn)yl, acyl-C₁₋₆-alk(en/yn)yl-C₃₋₈-cycloalk(en)yl, acyl-C₁₋₆-alk(en/yn)yl-heterocycloalk(en)yl, N**R¹²R^{12'},** optionally substituted N**R¹²R^{12'}**-C₁₋₆-alk(en/yn)yl, optionally substituted N**R¹²R^{12'}**-C₃₋₈-cycloalk(en)yl, optionally substituted N**R¹²R^{12'}**-C₃₋₈-cycloalk(en)yl-C₁₋₆-alk(en/yn)yl; wherein
**R¹²** and **R^{12'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar, Ar-C₁₋₆-alk(en/yn)yl, Ar-C₃₋₈-cycloalk(en)yl, Ar-C₃₋₈-cycloalk(en)yl-C₁₋₆-alk(en/yn)yl, Ar-heterocycloalk(en)yl, Ar-oxy-C₁₋₆-alk(en/yn)yl, Ar-oxy-C₃₋₈-cycloalk(en)yl, Ar-oxy-C₃₋₈-cycloalk(en)yl-C₁₋₆-alk(en/yn)yl, Ar-oxy-heterocycloalk(en)yl, hydroxy-C₁₋₆-alk(en/yn)yl, hydroxy-C₃₋₈-cycloalk(en)yl, hydroxy-C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, cyano-C₁₋₆-alk(en/yn)yl, cyano-C₃₋₈-cycloalk(en)yl and cyano-C₃₋₈-cycloalk(en)yl-C₁₋₆-alk(en/yn)yl, or
**R¹²** and **R^{12'}** together with the nitrogen atom form a 5-8 membered saturated or unsaturated ring which optionally contains 1, 2 or 3 further heteroatoms;
with the proviso that when **R³** is N**R¹²R^{12'}** then q is 0;
and
**Y** represents a group of formula **XXIV, XXV, XXVI, XXVII, XXVIII, XXXXI** or **XXXXII** 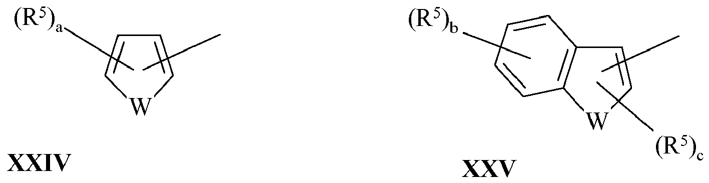 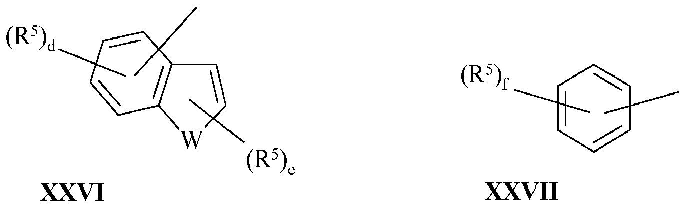 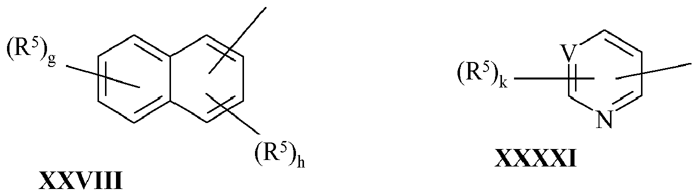 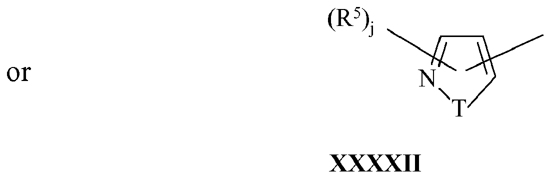 wherein
the line represents a bond attaching the group represented by Y to the carbon atom;
**W** is O or S;
**V** is N, C or CH;
**T** is N, NH or O;
**a** is 0, 1, 2 or 3;
**b** is 0, 1, 2, 3 or 4;
**c** is 0 or 1;
**d** is 0, 1, 2 or 3;
**e** is 0, 1 or 2;
**f** is 0, 1, 2, 3, 4 or 5;
**g** is 0, 1, 2, 3 or 4;
**h** is 0, 1, 2 or 3;
**j** is 0, 1 or 2;
**k** is 0, 1, 2 or 3; and
each **R⁵** is independently selected from the group consisting of a C₁₋₆-alk(en/yn)yl, C₃₋₈-cycloalk(en)yl, C₃₋₈-cycloalk(en)yl-C₁₋₆-alk(en/yn)yl, Ar, ArC₁₋₆-alk(en/yn)yl, Ar-C₃₋₈-cycloalk(en)yl, Ar-C₃₋₈-cycloalk(en)yl-C₁₋₆-alk(en/yn)yl, Ar-oxy, Ar-oxy-C₁₋₆-alk(en/yn)yl, Ar-oxy-C₃₋₈-cycloalk(en)yl, C₁₋₆-alk(en/yn)yl-heterocycloalk(en)yl, Ar-oxy-C₃₋₈-cycloalk(en)yl-C₁₋₆-alk(en/yn)yl, acyl, C₁₋₆-alk(en/yn)yloxy, C₃₋₈-cycloalk(en)yloxy, C₃₋₈-cycloalk(en)yl-C₁₋₆-alk(en/yn)yloxy, C₁₋₆-alk(en/yn)yloxy-carbonyl, halogen, halo-C₁₋₆-alk(en/yn)yl, halo-C₃₋₈-cycloalk(en)yl, halo-C₃₋₈-cycloalk(en)yl-C₁₋₆-alk(en/yn)yl, -CO-NR⁶R^{6'} cyano, cyano-C₁₋₆-alk(en/yn)yl, cyano-C₃₋₈-cycloalk(en)yl, cyano-C₃₋₈-cycloalk(en)yl-C₁₋₆-alk(en/yn)yl, N**R⁷R^{7'}**, S-**R⁸** and SO₂**R⁸**, or
two adjacent **R⁵** together with the aromatic group form a 5-8 membered ring which optionally contains one or two heteroatoms;
**R⁶** and **R^{6'}** are independently selected from the group consisting of hydrogen, C₁-₆-alk(en/yn)yl, C₃₋₈-cycloalk(en)yl, C₃₋₈-cycloalk(en)yl-C₁₋₆-alk(en/yn)yl and Ar;
**R⁷** and **R^{7'}** are independently selected from the group consisting of hydrogen, C₁-₆-alk(en/yn)yl, C₃₋₈-cycloalk(en)yl, C₃₋₈-cycloalk(en)yl-C₁₋₆-alk(en/yn)yl, Ar, heterocycloalk(en)yl-C₁₋₆-alk(en/yn)yl, heterocycloalk(en)yl-C₃₋₈-cycloalk(en)yl, heterocycloalk(en)yl-C₃₋₈-cycloalk(en)yl-C₁₋₆-alk(en/yn)yl, heterocycloalk(en)yl-Ar and acyl; or
**R⁷** and **R^{7'}** together with the nitrogen atom form a 5-8 membered saturated or unsaturated ring which optionally contains 1, 2 or 3 further heteroatoms; and
**R⁸** is selected from the group consisting of hydrogen, C₁₋₆-alk(en/yn)yl, C₃₋₈-cycloalk(en)yl, C₃-₈-cycloalk(en)yl-C₁₋₆-alk(en/yn)yl, Ar and -N**R⁹R^{9'}**; wherein **R⁹** and **R^{9'}** are independently selected from the group consisting of hydrogen, C₁-₆-alk(en/yn)yl, C₃₋₈-cycloalk(en)yl and C₃₋₈-cycloalk(en)yl-C₁₋₆-alk(en/yn)yl;
or pharmaceutically acceptable salts thereof; and
where formula 3 is: 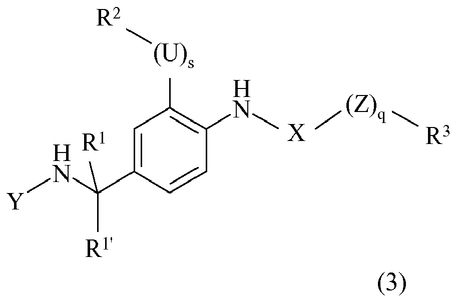 wherein
**U** is O, S or N**R^{2'}**_{;}
**s** is 0 or 1;
**X** is CO or SO₂;
**Z** is O, S or N**R⁴**, wherein **R⁴** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, hydroxy-C₁₋₆-alk(en/yn)yl and hydroxy-C₃₋₈-cycloalk(en)yl;
**q** is 0 or 1;
**R¹** and **R^{1'}** are independently selected from the group consisting of hydrogen, C₁-₆-alk(en/yn)yl, C₃₋₈-cycloalk(en)yl, C₃-₈-cycloalk(en)yl-C₁₋₆-alk(en/yn)yl, acyl, hydroxy-C₁₋₆-alk(en/yn)yl, hydroxy-C₃₋₈-cycloalk(en)yl, halo-C₁₋₆-alk(en/yn)yl and halo-C₃₋₈-cycloalk(en)yl;
**R²** is selected from the group consisting of hydrogen, halogen, C₁₋₆-alk(en/yn)yl, C₃₋₈-cycloalk(en)yl, C₃₋₈-cycloalk(en)yl-C₁₋₆-alk(en/yn)yl, Ar, Ar-C₁₋₆-alk(en/yn)yl, Ar-C₃₋₈-cycloalk(en)yl, acyl, hydroxy-C₁₋₆-alk(en/yn)yl, hydroxy-C₃₋₈-cycloalk(en)yl, halo-C₁₋₆₋alk(en/yn)yl, halo-C₃₋₈-cycloalk(en)yl and cyano; provided that when **R²** is halogen or cyano, then s is 0;
when **s** is 1 and **U** is N**R^{2'}** then **R^{2'}** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar, Ar-C₁₋₆-alk(en/yn)yl, Ar-C₃₋₈-cycloalk(en)yl, acyl, hydroxy-C₁₋₆-alk(en/yn)yl, hydroxy-C₃₋₈-cycloalk(en)yl, halo-C₁₋₆-alk(en/yn)yl and halo-C₃₋₈-cycloalk(en)yl; or **R²** and **R^{2'}** together form a 5-8 membered saturated or unsaturated ring which optionally contains one further heteroatom;
**R³** is selected from the group consisting of C₁₋₆-alk(en/yn)yl, C₃₋₈-cycloalk(en)yl, C₃₋₈-cycloalk(en)yl-C₁₋₆-alk(en/yn)yl, Ar, Ar-C₁₋₆-alk(en/yn)yl, Ar-C₃₋₈-cycloalk(en)yl, hydroxy-C₁₋₆-alk(en/yn)yl, hydroxy-C₃₋₈-cycloalk(en)yl, halo-C₁₋₆-alk(en/yn)yl and halo-C₃₋₈-cycloalk(en)yl;
and
**Y** represents a group of formulae **VI, VII, VIII, IX** or **XXX:** 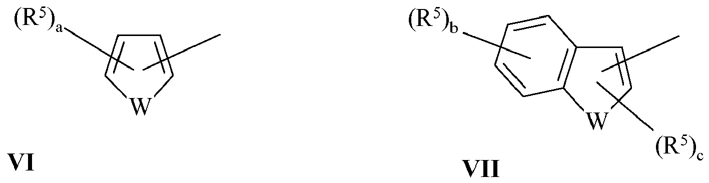 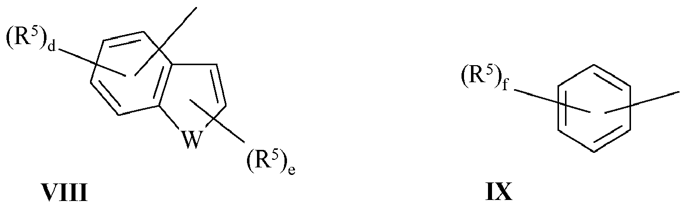 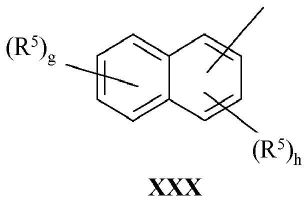 wherein
the line represents a bond attaching the group represented by Y to the nitrogen atom;
**W** is O or S;
**a** is 0, 1, 2 or 3;
**b** is 0, 1, 2, 3 or 4;
**c** is 0 or 1;
**d** is 0, 1, 2 or 3;
**e** is 0, 1 or 2;
**f** is 0, 1, 2, 3, 4 or 5;
**g** is 0, 1, 2, 3 or 4;
**h** is 0, 1, 2 or 3; and
each **R⁵** is independently selected from the group consisting of a C₁₋₆-alk(en/yn)yl, C₃₋₈-cycloalk(en)yl, Ar, C₃₋₈-cycloalk(en)yl-C₁₋₆-alk(en/yn)yl, ArC₁₋₆-alk(en/yn)yl, acyl, C₁₋₆-alk(an/en/yn)yloxy, halogen, halo-C₁₋₆-alk(en/yn)yl, -CO-N**R⁶R^{6'}**, cyano, nitro, -N**R⁷R^{7'}**, -S-**R⁸,** -SO₂**R⁸** and SO₂O**R⁸**, or two substituents together form a 5-8 membered saturated or unsaturated ring which optionally contains one or two heteroatoms;
**R⁶** and **R^{6'}** are independently selected from the group consisting of hydrogen, C₁-₆-alk(en/yn)yl, C₃₋₈-cycloalk(en)yl, C₃₋₈-cycloalk(en)yl-C₁₋₆-alk(en/yn)yl and Ar;
**R⁷** and **R^{7'}** are independently selected from the group consisting of hydrogen, C₁-₆-alk(en/yn)yl, C₃₋₈-cycloalk(en)yl, C₃₋₈-cycloalk(en)yl-C₁₋₆-alk(en/yn)yl, Ar and acyl; and
**R⁸** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar and -NR⁹R^{9'}; wherein **R⁹** and **R^{9'}** are independently selected from the group consisting of hydrogen, C₁₋₆-alk(en/yn)yl, C₃₋₈-cycloalk(en)yl and C₃₋₈-cycloalk(en)yl-C₁₋₆-alk(en/yn)yl; with the provisos that when **R⁵** is SO₂O**R⁸** then **R⁸** is not -N**R⁹R^{9'}** and when **R⁵** is SO₂**R⁸**, then **R⁸** is not a hydrogen atom;
or pharmaceutically acceptable salts thereof; and
where formula 4 is: 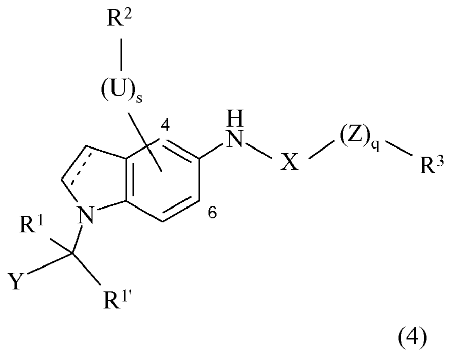 wherein
the dotted line represents an optional bond;
**R¹** and **R^{1'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, hydroxy-C₁₋₆-alk(en/yn)yl, hydroxy-C₃₋₈-cycloalk(en)yl, hydroxy-C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, cyano-C₁₋₆-alk(en/yn)yl, cyano-C₃₋₈-cycloalk(en)yl and cyano-C₃₋₈-cycloalk(en)yl-C₁₋₆-alk(en/yn)yl; or
**R¹** and **R^{1'}** together with the carbon atom to which they are attached form a 3-8 membered saturated or unsaturated ring which optionally contains 1 or 2 heteroatoms;
**s** is 0 or 1;
**U** is O, N**R¹¹**, S, SO₂, SO₂N**R¹¹**, CO-O or CO-N**R¹¹**; wherein **R¹¹** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl; or **R²** and **R¹¹** together with the nitrogen atom to which they are attached form a 4-8 membered saturated or unsaturated ring which optionally contains 1, 2 or 3 further heteroatoms;
**R²** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar, Ar-C₁₋₆-alk(en/yn)yl, Ar-C₃₋₈-cycloalk(en)yl, Ar-C₃₋₈-cycloalk(en)yl-C₁₋₆-alk(en/yn)yl, acyl, hydroxy-C₁₋₆-alk(en/yn)yl, hydroxy-C₃₋₈-cycloalk(en)yl, hydroxy-C₃₋₈-cycloalk(en)yl-C₁₋₆-alk(en/yn)yl, halogen, halo-C₁₋₆-alk(en/yn)yl, halo-C₃₋₈-cycloalk(en)yl, halo-C₃₋₈-cycloalk(en)yl-C₁₋₆-alk(en/yn)yl, cyano, cyano-C₁₋₆-alk(en/yn)yl, cyano-C₃₋₈-cycloalk(en)yl, cyano-C₃₋₈-cycloalk(en)yl-C₁₋₆-alk(en/yn)yl, -NO₂, N**R¹⁰R^{10'}**-C₁₋₆-alk(en/yn)yl, N**R¹⁰R^{10'}**-C₃₋₈-cycloalk(en)yl and N**R¹⁰R^{10'}**-C₃₋₈-cycloalk(en)yl-C₁₋₆-alk(en/yn)yl; wherein
**R¹⁰** and **R^{10'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, hydroxy-C₁₋₆-alk(en/yn)yl, hydroxy-C₃₋₈-cycloalk(en)yl, hydroxy-C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, cyano-C₁₋₆-alk(en/yn)yl, cyano-C₃₋₈-cycloalk(en)yl and cyano-C₃₋₈-cycloalk(en)yl-C₁₋₆-alk(en/yn)yl, or
**R¹⁰** and **R^{10'}** together with the nitrogen atom to which they are attached form a 4-8 membered saturated or unsaturated ring which optionally contains 1, 2 or 3 further heteroatoms;
with the proviso that when **R²** is NO₂, halogen or cyano then **s** is 0; and
with the proviso that when **R²** is a hydrogen atom or acyl and s is 1 then **U** is N**R¹¹,** O or S;
wherein the group **-(U)ₛ-R²** is linked to position 4 or 6 of the indole or indoline;
**q** is 0 or 1;
**Z** is O or S;
**X** is CO or SO₂; with the proviso that **q** is 0 when **X** is SO₂;
**R³** is selected from the group consisting of C₁₋₆-alk(en/yn)yl, C₃₋₈-cycloalk(en)yl, heterocycloalk(en)yl, C₃₋₈-cycloalk(en)yl-C₁₋₆-alk(en/yn)yl, C₁₋₆-alk(en/yn)yl-C₃₋₈-cycloalk(en)yl, C₁₋₆-alk(en/yn)yl-heterocycloalk(en)yl, Ar, Ar-C₁₋₆-alk(en/yn)yl, Ar-C₃₋₈-cycloalk(en)yl, Ar-heterocycloalk(en)yl, Ar-C₃₋₈-cycloalk(en)yl-C₁₋₆-alk(en/yn)yl, Ar-C₁₋₆-alk(en/yn)yl-C₃₋₈-cycloalk(en)yl, Ar-C₁₋₆-alk(en/yn)yl-heterocycloalk(en)yl, C₁₋₆-alk(en/yn)yloxy-C₁₋₆-alk(en/yn)yl, C₃₋₈-cycloalk(en)yloxy-C₁₋₆-alk(en/yn)yl, C₁₋₆-alk(en/yn)yloxy-C₃₋₈-cycloalk(en)yl, C₁₋₆-alk(en/yn)yloxy-heterocycloalk(en)yl, Ar-oxy-C₁₋₆-alk(en/yn)yl, Ar-C₁₋₆-alk(en/yn)yloxy-C₁₋₆-alk(en/yn)yl, C₁₋₆-alk(en/yn)yloxy-carbonyl-C₁₋₆-alk(en/yn)yl, C₃₋₈-cycloalk(en)yloxy-carbonyl-C₁₋₆-alk(en/yn)yl, C₃₋₈-cycloalk(en)yl-C₁₋₆₋alk(en/yn)yloxy-carbonyl-C₁₋₆-alk(en/yn)yl, hydroxy-C₁₋₆-alk(en/yn)yl, hydroxy-C₃₋₈-cycloalk(en)yl, hydroxy-heterocycloalk(en)yl, hydroxy-C₃₋₈-cycloalk(en)yl-C₁₋₆-alk(en/yn)yl, hydroxy-C₁₋₆-alk(en/yn)yl-C₃₋₈-cycloalk(en)yl, hydroxy-C₁₋₆-alk(en/yn)yl-heterocycloalk(en)yl, halo-C₁₋₆-alk(en/yn)yl, halo-C₃₋₈-cycloalk(en)yl, halo-heterocycloalk(en)yl, halo-C₃₋₈-cycloalk(en)yl-C₁₋₆₋alk(en/yn)yl, halo-C₁₋₆-alk(en/yn)yl-C₃₋₈-cycloalk(en)yl, halo-C₁₋₆-alk(en/yn)yl-heterocycloalk(en)yl, halo-C₁₋₆-alk(en/yn)yl-Ar, halo-C₃₋₈-cycloalk(en)yl-Ar, halo-C₃₋₈-cycloalk(en)yl-C₁₋₆-alk(en/yn)yl-Ar, halo-C₁₋₆-alk(en/yn)yl-C₃₋₈-cycloalk(en)yl-Ar, cyano-C₁₋₆-alk(en/yn)yl, cyano-C₃₋₈-cycloalk(en)yl, cyano-heterocycloalk(en)yl, cyano-C₃₋₈-cycloalk(en)yl-C₁₋₆-alk(en/yn)yl, cyano-C₁₋₆-alk(en/yn)yl-C₃₋₈-cycloalk(en)yl, cyano-C₁₋₆-alk(en/yn)yl-heterocycloalk(en)yl, acyl-C₁₋₆-alk(en/yn)yl, acyl-C₃₋₈-cycloalk(en)yl, acyl-heterocycloalk(en)yl, acyl-C₃₋₈-cycloalk(en)yl-C₁₋₆-alk(en/yn)yl, acyl-C₁₋₆-alk(en/yn)yl-C₃₋₈-cycloalk(en)yl, acyl-C₁₋₆-alk(en/yn)yl-heterocycloalk(en)yl and -N**R¹²R^{12'}**, optionally substituted N**R¹²R^{12'}**-C₁₋₆-alk(en/yn)yl, optionally substituted NR¹²R^{12'}-C₃₋₈-cycloalk(en)yl, optionally substituted N**R¹²R^{12'}**-C₃₋₈-cycloalk(en)yl-C₁₋₆-alk(en/yn)yl; wherein
**R¹²** and **R^{12'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar, Ar-C₁₋₆-alk(en/yn)yl, Ar-C₃₋₈-cycloalk(en)yl, Ar-C₃₋₈-cycloalk(en)yl-C₁₋₆-alk(en/yn)yl, hydroxy-C₁₋₆-alk(en/yn)yl, hydroxy-C₃₋₈-cycloalk(en)yl, hydroxy-C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, cyano-C₁₋₆-alk(en/yn)yl, cyano-C₃₋₈-cycloalk(en)yl and cyano-C₃₋₈-cycloalk(en)yl-C₁₋₆-alk(en/yn)yl, or
**R¹²** and **R^{12'}** together with the nitrogen atom to which they are attached form a 4-8 membered saturated or unsaturated ring which optionally contains 1, 2 or 3 further heteroatoms;
with the proviso that when **R³** is N**R¹²R^{12'}** then q is 0;
and
**Y** represents a group of formula **II, III, IV, V, , VI, XXX** and **XXXI**: 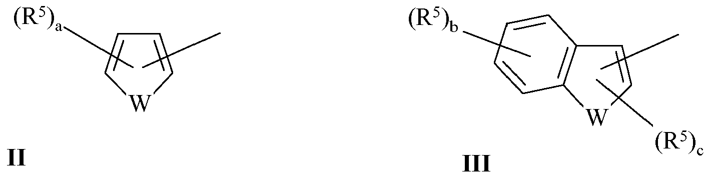 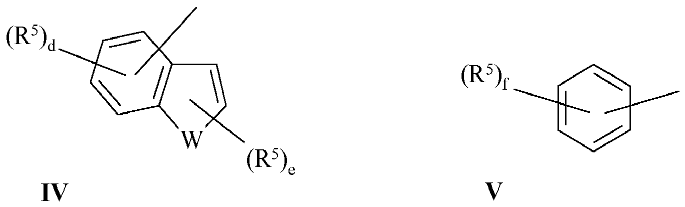 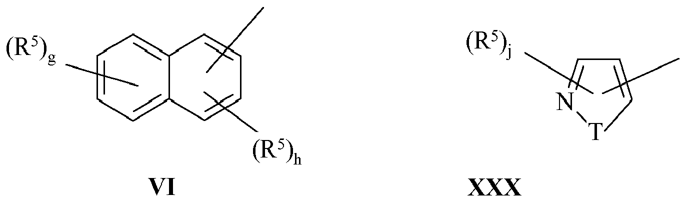 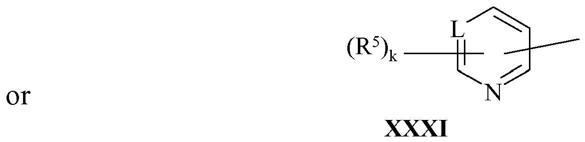 wherein
the line represents a bond attaching the group represented by Y to the carbon atom;
**W** is O or S;
**T** is N, NH or O;
**L** is N, C or CH;
**a** is 0, 1 , 2 or 3;
**b** is 0, 1, 2, 3 or 4;
**c** is 0 or 1;
**d** is 0, 1, 2 or 3;
**e** is 0, 1 or 2;
**f** is 0, 1, 2, 3, 4 or 5;
**g** is 0, 1, 2, 3 or 4;
**h** is 0, 1, 2 or 3;
**j** is 0, 1, 2 or 3; with the proviso that when **T** is a nitrogen atom then **j** is 0, 1, 2 or 3; and when **T** is NH or an oxygen atom then **j** is 0, 1 or 2;
**k** is 0,1, 2, 3 or 4; and
each **R⁵** is independently selected from the group consisting of a C₁₋₆-alk(en/yn)yl, C₃₋₈-cycloalk(en)yl, C₃₋₈-cycloalk(en)yl-C₁₋₆-alk(en/yn)yl, Ar, ArC₁₋₆-alk(en/yn)yl, Ar-thio, Ar-oxy, acyl, C₁₋₆-alk(en/yn)yloxy, C₃₋₈-cycloalk(en)yloxy, C₃₋₈-cycloalk(en)yl-C₁₋₆-alk(en/yn)yloxy, halogen, halo-C₁₋₆-alk(en/yn)yl, halo-C₃₋₈-cycloalk(en)yl, halo-C₃₋₈-cycloalk(en)yl-C₁₋₆-alk(en/yn)yl, -CO-N**R⁶R^{6'},** cyano, cyano-C₁₋₆-alk(en/yn)yl, cyano-C₃₋₈-cycloalk(en)yl, cyano-C₃₋₈-cycloalk(en)yl-C₁₋₆-alk(en/yn)yl, -N**R⁷R^{7'},** -S-**R⁸** and -SO₂**R⁸,** or
two adjacent **R⁵** together with the aromatic group to which they are attached form a 4-8 membered ring which optionally contains one or two heteroatoms;
**R⁶** and **R^{6'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl and Ar;
**R⁷** and **R^{7'}** are independently selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar and acyl;
and
**R⁸** is selected from the group consisting of hydrogen, C₁₋₆-alk(en/yn)yl, C₃₋₈-cycloalk(en)yl, C₃₋₈-cycloalk(en)yl-C₁₋₆-alk(en/yn)yl, Ar and -N**R⁹R^{9'}**; wherein **R⁹** and **R^{9'}** are independently selected from the group consisting of hydrogen, C₁₋₆-alk(en/yn)yl, C₃₋₈-cycloalk(en)yl and C₃₋₈-cycloalk(en)yl-C₁₋₆-alk(en/yn)yl; provided that when **R⁸** is -N**R⁹R^{9'}** then **R⁵** is not -S-**R⁸**;
or pharmaceutically acceptable salts thereof; and
where formula 5 is: 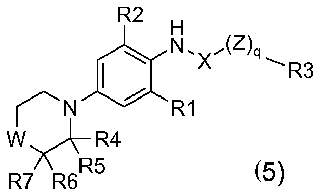 wherein
q is 0 or 1;
W is O or S;
X is CO;
Z is O;
R1 is selected from the group consisting of halogen, cyano, C₁₋₆-alk(en/yn)yl, C₃₋₈-cycloalk(en)yl, C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, C₁₋₆-alk(en/yn)yloxy, C₃₋₈-cycloalk(en)yloxy and C₃₋₈-cycloalk(en)yl-C₁₋₆-alk(en/yn)yloxy;
R2 is selected from the group consisting of halogen, cyano, C₁₋₆-alk(en/yn)yl, C₃₋₈-cycloalk(en)yl, C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, C₁₋₆-alk(en/yn)yloxy, C₃₋₈-cycloalk(en)yloxy, C₃₋₈-cycloalk(en)yl-C₁₋₆-alk(en/yn)yloxy, optionally substituted phenyl and optionally substituted pyridyl; wherein phenyl and pyridyl are optionally substituted with one or more substituents independently being halogen, C₁₋₆-alk(en/yn)yl, C₃₋₈-cycloalk(en)yl or C₃₋₈-cycloalk(en)yl-C₁₋₆-alk(en/yn)yl;
R3 is selected from the group consisting of C₁₋₁₀-alk(en/yn)yl, C₃₋₈-cycloalk(en)yl, C₃₋₈-cycloalk(en)yl-C₁₋₆-alk(en/yn)yl, ArC₁₋₆-alk(en/yn)yl, Ar-C₃₋₈-cycloalk(en)yl, Ar-C₃₋₈-cycloalk(en)yl-C₁₋₆-alk(en/yn)yl and Ar; and
each of R4, R5, R6 and R7 is independently selected from the group consisting of hydrogen and Ar;
or pharmaceutically acceptable salts thereof; and
where formula 6 is: 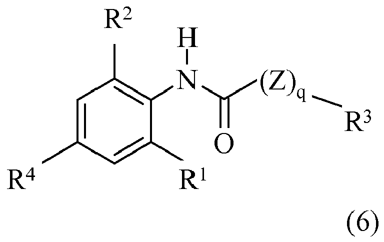 wherein
Z is O or S;
and
q is 0 or 1;
and
each of R¹ and R² is independently selected from the group consisting of halogen, cyano, amino, C₁₋₆-alk(en/yn)yl, C₃₋₈-cycloalk(en)yl, C₃₋₈-cycloalk(en)yl-C₁₋₆-alk(en/yn)yl, C₃₋₈-heterocycloalk(en)yl, Aryl, Heteroaryl, halo-C₁₋₆-alk(en/yn)yl, halo-C₃₋₈-cycloalk(en)yl, halo-C₃₋₈-cycloalk(en)yl-C₁₋₆-alk(en/yn)yl, C₁₋₆-alk(en/yn)yloxy, C₃₋₈-cycloalk(en)yloxy, C₃₋₈-cycloalk(en)yl-C₁₋₆-alk(en/yn)yloxy, C₃₋₈-heterocycloalk(en)yloxy;
and
R³ is selected from the group consisting of C₁₋₈-alk(en/yn)yl,C₃₋₈-cycloalk(en)yl, C₃₋₈-cycloalk(en)yl-C₁₋₆-alk(en/yn)yl, Aryl-C₁₋₆-alk(en/yn)yl, Aryl-C₃₋₈-cycloalk(en)yl, Aryl-C₃₋₈-cycloalk(en)yl-C₁₋₆-alk(en/yn)yl, C₃₋₈-heterocycloalk(en)yl-C₁₋₆₋alk(en/yn)yl, C₁₋₆-alk(en/yn)yl-C₃₋₈-heterocycloalk(en)yl-C₁₋₆-alk(en/yn)yl, Heteroaryl-C₁₋₆-alk(en/yn)yl, Heteroaryl-C₃₋₈-cycloalk(en)yl, Heteroaryl-C₃₋₈-cycloalk(en)yl-C₁₋₆-alk(en/yn)yl, amino-C₁₋₆-alk(en/yn)yl, amino-C₃₋₈-cycloalk(en)yl, amino-C₃₋₈-cycloalk(en)yl-C₁₋₆-alk(en/yn)yl, C₁₋₆-alk(en/yn)yloxy-C₁₋₆-alk(en/yn)yl, C₃₋₈-cycloalk(en)yloxy-C₁₋₆-alk(en/yn)yl, C₃₋₈-cycloalk(en)yl-C₁₋₆-alk(en/yn)yloxy-C₁₋₆-alk(en/yn)yl, halo-C₁₋₆-alk(en/yn)yl, halo-C₃₋₈-cycloalk(en)yl and halo-C₃₋₈-cycloalk(en)yl-C₁₋₆-alk(en/yn)yl;
and
R⁴ is selected from the group consisting of halogen, cyano, C₁₋₆-alk(en/yn)yl, C₃₋₈-cycloalk(en)yl, C₃₋₈-cycloalk(en)yl-C₁₋₆-alk(en/yn)yl, C₃₋₈-heterocycloalk(en)yl, Aryl, Heteroaryl, Aryl-C₁₋₆-alk(en/yn)yl, Aryl-C₃₋₈-cycloalk(en)yl, Aryl-C₃₋₈-cycloalk(en)yl-C₁₋₆-alk(en/yn)yl, Aryl-C₃₋₈-heterocycloalk(en)yl, halo-C₁₋₆-alk(en/yn)yl, halo-C₃₋₈-cycloalk(en)yl, halo-C₃₋₈-cycloalk(en)yl-C₁₋₆-alk(en/yn)yl, halo-C₁₋₆-alk(en/yn)yl-C₃₋₈-heterocycloalk(en)yl-C₁-₆-alk(en/yn)yl, NR⁵R⁶ and R⁷NH-C₁₋₆-alk(en/yn)yl; wherein R⁵ and R⁶ are independently selected from the group consisting of hydrogen, Aryl-C₁₋₆-alk(en/yn)yl, Aryl-C₃₋₈-cycloalk(en)yl, Aryl-C₃₋₈-cycloalk(en)yl-C₁₋₆-alk(en/yn)yl, C₁₋₆-alk(en/yn)yl, C₃₋₈-cycloalk(en)yl, C₃₋₈-cycloalk(en)yl-C₁₋₆-alk(en/yn)yl, Heteroaryl-C₁₋₆-alk(en/yn)yl, Heteroaryl-C₃₋₈-cycloalk(en)yl and Heteroaryl-C₃₋₈-cycloalk(en)yl-C₁₋₆-alk(en/yn)yl with the proviso that R⁵ and R⁶ are not hydrogen at the same time; and R⁷ is selected from the group consisting of C₁₋₆-alk(en/yn)yl, C₃₋₈-cycloalk(en)yl, C₃₋₈-cycloalk(en)yl-C₁₋₆-alk(en/yn)yl, Aryl, halo-C₁₋₆-alk(en/yn)yl, halo-C₃₋₈-cycloalk(en)yl, halo-C₃₋₈-cycloalk(en)yl-C₁₋₆-alk(en/yn)yl, Aryl-C₁₋₆-alk(en/yn)yl, Aryl-C₃₋₈-cycloalk(en)yl and Heteroaryl;
or pharmaceutically acceptable salts thereof; and
where formula 7 is: 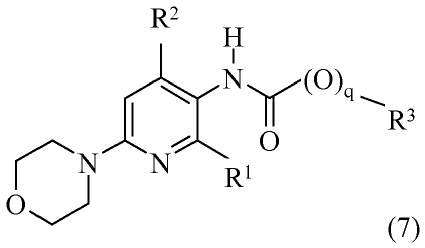 wherein:
q is 0 or 1;
each of R¹ and R² is independently selected from the group consisting of halogen, cyano, C₁₋₆-alk(en/yn)yl, C₃₋₈-cycloalk(en)yl, C₃₋₈-cycloalk(en)yl-C₁₋₆-alk(en/yn)yl, halo-C₁₋₆-alk(en/yn)yl, halo-C₃₋₈-cycloalk(en)yl, halo-C₃₋₈-cycloalk(en)yl-C₁₋₆-alk(en/yn)yl, C₁₋₆-alk(en/yn)yloxy, C₃₋₈-cycloalk(en)yloxy and C₃₋₈-cycloalk(en)yl-C₁₋₆-alk(en/yn)yloxy; and
R³ is selected from the group consisting of C₁₋₈-alk(en/yn)yl, C₃₋₈-cycloalk(en)yl, C₃₋₈-cycloalk(en)yl-C₁₋₆-alk(en/yn)yl, optionally substituted Aryl-C₁₋₆-alk(en/yn)yl, optionally substituted Aryl-C₃₋₈-cycloalk(en)yl,
optionally substituted Aryl-C₃₋₈-cycloalk(en)yl-C₁₋₆-alk(en/yn)yl, C₁₋₆-alk(en/yn)yl-C₃₋₈-heterocycloalk(en)yl-C₁₋₆-alk(en/yn)yl, C₃₋₈-heterocycloalk(en)yl-C₁₋₆-alk(en/yn)yl, C₁₋₆-alk(en/yn)yl-C₃₋₈-heterocycloalk(en)yl-C₁₋₆-alk(en/yn)yl, Heteroaryl-C₁₋₆-alk(en/yn)yl, Heteroaryl-C₃₋₈-cycloalk(en)yl, Heteroaryl-C₃₋₈-cycloalk(en)yl-C₁₋₆-alk(en/yn)yl, NR⁴R⁵-C₁₋₆-alk(en/yn)yl, NR⁴R⁵-C₃₋₈-cycloalk(en)yl, NR⁴R⁵-C₃₋₈-cycloalk(en)yl-C₁₋₆-alk(en/yn)yl, C₁₋₆-alk(en/yn)yloxy-C₁₋₆-alk(en/yn)yl, C₃₋₈-cycloalk(en)yloxy-C₁₋₆-alk(en/yn)yl, C₃₋₈-cycloalk(en)yl-C₁₋₆-alk(en/yn)yloxy-C₁₋₆-alk(en/yn)yl, halo-C₁₋₆-alk(en/yn)yl, halo-C₃₋₈-cycloalk(en)yl and halo-C₃₋₈-cycloalk(en)yl-C₁₋₆-alk(en/yn)yl; wherein
each of R⁴ and R⁵ is independently selected from the group consisting of hydrogen, C₁₋₆-alk(en/yn)yl, C₃₋₈-cycloalk(en)yl and C₃₋₈-cycloalk(en)yl-C₁₋₆-alk(en/yn)yl;
or pharmaceutically acceptable salts thereof; and
where formula 8 is: 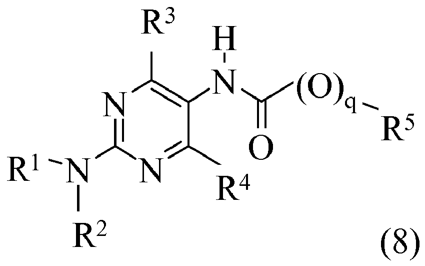 wherein: q is 0 or 1;
R¹ and R² are independently selected from the group consisting of hydrogen and optionally substituted aryl-C₁₋₆-alk(en/yn)yl, provided that R¹ and R² are not both hydrogen, or R¹ and R² together with the nitrogen to which they are attached form a 5 to 7 membered ring optionally containing a further heteroatom;
R³ and R⁴ are independently selected from hydrogen, halogen, cyano, amino, C₁-₆-alk(en/yn)yl, C₃₋₈-cycloalk(en)yl, halo-C₁₋₆-alk(en/yn)yl, halo-C₃₋₈-cycloalk(en)yl, C₁-₆-alk(en/yn)yloxy, C₃₋₈-cycloalk(en)yloxy, C₃₋₈-cycloalk(en)yl-C₁₋₆-alk(en/yn)yloxy, halo-C₁₋₆-alk(en/yn)yloxy, halo-C₃₋₈-cycloalk(en)yloxy and halo-C₃₋₈-cycloalk(en)yl-C₁₋₆-alk(en/yn)yloxy, provided that R³ and R⁴ are not both hydrogen;
R⁵ is selected from the group consisting of C₁₋₁₀-alk(en/yn)yl, C₃₋₈-cycloalk(en)yl-C₁₋₆-alk(en/yn)yl, optionally substituted aryl-C₁₋₆-alk(en/yn)yl and optionally substituted aryl;
or pharmaceutically acceptable salts thereof; and
where formula 9 is: 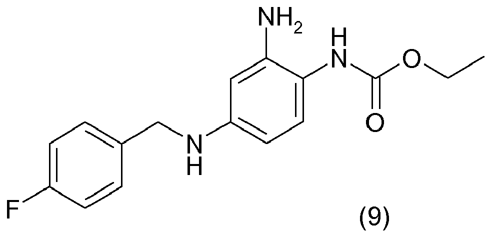 or a pharmaceutically acceptable salt thereof.

13. The use according to claim 12, wherein the compound is selected from the group consisting of:
N-(2-amino-4-(4-fluorobenzylamino)-phenyl) carbamic acid ethyl ester;
2-cyclopentyl-N-(2,6-dimethyl-4-morpholin-4-yl-phenyl)-acetamide;
N-(2,6-dimethyl-4-morpholin-4-yl-phenyl)-3,3-dimethyl-butyramide;
N-(4,6-dimethyl-2-morpholin-4-yl-pyrimidin-5-yl)-2-(4-fluoro-phenyl)-acetamide;
hexanoic acid (2,6-difluoro-4-morpholin-4-yl-phenyl)-amide;
2-cyclopentyl-N-(4,6-dimethyl-2-morpholin-4-yl-pyrimidin-5-yl)-acetamide;
N-(2-bromo-4-morpholin-4-yl-6-trifluoromethyl-phenyl)-propionamide;
N-(2,4-dimethyl-6-morpholin-4-yl-pyridin-3-yl)-3,3-dimethyl-butyramide;
[2-amino-4-(2,4,6-trimethyl-benzylamino)-phenyl]-carbamic acid ethyl ester; and
2-cyclopentyl-N-(2-methoxy-6-methyl-4-morpholin-4-yl-phenyl)-acetamide.

14. A method of screening for a compound which is a selective KCNQ potassium channel opener and which is capable of having an anti-psychotic potential, comprising the steps of:
a. screening for a KCNQ potassium channel opener;
b. contra-screening against other channels and/or receptors, and
c. testing the compound in a model predictive for an anti-psychotic potential.

15. Use of a compound obtainable by the method according to claim 14 in the manufacture of a medicament for the treatment of schizophrenia.
